# EUROPÄISCHE PATENTSCHRIFT

(11) **EP 2 547 664 B1**
(45) Veröffentlichungstag und Bekanntmachung des Hinweises auf die Patenterteilung: **04.05.2016**
(21) Anmeldenummer: 11704552.6
(22) Anmeldetag: 17.02.2011
(51) Int. Cl.: C07D 239/86, C07D 239/94, C07D 401/04, C07D 401/14, C07D 409/06, C07D 409/14, C07D 413/14, C07D 417/06, C07D 417/14, C07D 295/155, A61K 31/517, A61P 35/00

(54) **MORPHOLINYLCHINAZOLINE**
MORPHOLINYLQUINAZOLINES
MORPHOLINYLCHINAZOLINE

(30) Priorität: 16.03.2010 DE 102010011493
(43) Veröffentlichungstag der Anmeldung: 23.01.2013
(73) Patentinhaber: Merck Patent GmbH, 64293 Darmstadt (DE)
(72) Erfinder: MEDERSKI, Werner, 64673 Zwingenberg (DE); FUCHSS, Thomas, 64625 Bensheim-Auerbach (DE); ZENKE, Frank, 64291 Darmstadt (DE)
(86) Internationale Anmeldenummer: PCT/EP2011/000767
(87) Internationale Veröffentlichungsnummer: WO 2011/113512

(56) Entgegenhaltungen:
- EP-A1- 0 566 226
- WO-A1-2008/028691
- WO-A1-2010/014939
- WO-A2-02/20500
- US-A1- 2004 116 422
- US-A1- 2009 069 320
- US-B1- 6 265 411
- DATABASE REGISTRY [Online] CHEMICAL ABSTRACTS SERVICE, COLUMBUS, OHIO, US; 25. September 2009 (2009-09-25), XP002632526,
- DEGRAW ET AL.: "Reaction of indolylmagnesium bromide and chloromethylpyridines. Synthesis of skatylpyridines and piperidines", J. HETEROCYCLIC CHEM., Bd. 3, Nr. 1, 1966, Seiten 67-69, XP002632527, DOI: 10.1002/jhet.5570030114

## Beschreibung

Die Erfindung betrifft Verbindungen der Formeln (IA) und (II) 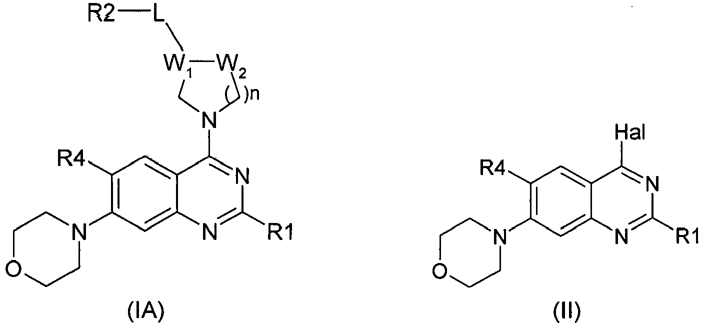 worin R1, R2, R3, R4, Y, W₁, W₂, L, A, Alk, Cyc, Ar, Het¹, Het², Hal und n die in Anspruch 1 angegebene Bedeutung aufweisen, und/oder ihre physiologisch unbedenklichen Salze, Tautomere und Stereoisomere, einschließlich deren Mischungen in allen Verhältnissen. Die Verbindungen der Formel (IA) können zur Hemmung von Serin-Threonin-Proteinkinasen sowie zur Sensibilisierung von Krebszellen gegenüber Antikrebsmitteln und/oder ionisierender Strahlung verwendet werden. Gegenstand der Erfindung ist auch die Verwendung der Verbindungen der Formel (IA) in der Prophylaxe, Therapie oder Verlaufskontrolle von Krebs, Tumoren, Metastasen oder Störungen der Angiogenese, in Kombination mit Radiotherapie und/oder einem Antikrebsmittel. Die Erfindung betrifft ferner ein Verfahren zum Herstellen der Verbindungen der Formel (IA) durch Umsetzen von Verbindungen der Formeln (II) und (III) und gegebenenfalls Umwandeln einer Base oder Säure der Verbindungen der Formel (IA) in eines ihrer Salze.

Die DNA-abhängige Proteinkinase (DNA-PK) ist eine Serin/Threonin-Proteinkinase, die in Verbindung mit DNA aktiviert wird. Biochemische und genetische Daten zeigen, dass DNA-PK (a) aus einer katalytischen Untereinheit, die man DNA-PKcs nennt, und (b) zwei regulatorischen Komponenten (Ku70 und Ku80) besteht. Funktional ist DNA-PK ein entscheidender Bestandteil einerseits der Reparatur von DNA-Doppelstrangbrüchen (DSBs) und anderseits der somatischen oder V(D)J Rekombination. Darüber hinaus werden DNA-PK und ihre Komponenten mit einer Vielzahl weiterer physiologischer Prozesse in Verbindung gebracht, darunter die Modulation der Chromatinstruktur sowie die telomere Wartung (Smith & Jackson (1999) Genes and Dev 13: 916; Goytisolo et al. (2001) Mol. Cell. Biol. 21: 3642; Williams et al. (2009) Cancer Res. 69: 2100).
Das menschliche Erbgut in Form der DNA ist fortwährend dem Angriff reaktiver Sauerstoff-Spezies (ROS) ausgesetzt, die vornehmlich als Nebenprodukte des oxidativen Metabolismus anfallen. ROS sind imstande, DNA-Schädigungen in Form von Einzelstrangbrüchen hervorzurufen. Doppelstrangbrüche können entstehen, wenn vorangegangene Einzelstrangbrüche in enger Nachbarschaft erfolgen. Darüber hinaus können Einzel- und Doppelstrangbrüche verursacht werden, wenn die DNA-Replikationsgabel auf beschädigte Basenmuster trifft. Des Weiteren sind körperfremde Einflüsse, wie ionisierende Strahlung (z.B. gamma- oder Schwerionen-Strahlung), und bestimmte Anti-Krebsmedikamente (z.B. Bleomycin) in der Lage, DNA-Doppelstrangbrüche hervorzurufen. DSB können ferner als Zwischenprodukte der somatischen Rekombination auftreten, einem Prozess, der bedeutend ist für die Ausbildung eines funktionalen Immunsystems aller Wirbeltiere. Werden DNA-Doppelstrangbrüche nicht oder fehlerhaft behoben, können Mutationen und/oder Chromosomenaberrationen auftreten, die in der Folge zum Zelltod führen können. Um den schwerwiegenden Gefährdungen, die von DNA-Doppelstrangbrüchen ausgehen, entgegenzuwirken, haben eukaryotische Zellen mehrere Mechanismen entwickelt, diese zu beheben. Höhere Eukaryoten bedienen sich dabei überwiegend der sogenannten nicht-homologen Endverknüpfung (*non-homologous end-joining,* NHEJ), bei der die DNA-abhängige Proteinkinase die Schlüsselrolle einnimmt. Biochemische Untersuchungen haben gezeigt, dass DNA-PK am wirksamsten durch das Auftreten von DNA-DSBs aktiviert wird. Zelllinien, deren DNA-PK Komponenten mutiert und nicht funktional sind, erweisen sich als strahlungsempfindlich (Smith und Jackson, 1999).

DNA-PK gehört wegen Ihrer katalytischen Domäne, die in der etwa 500 Aminosäuren zählenden C-terminalen katalytischen Untereinheit (DNA-PKcs) liegt, zur Familie der *Phosphatidylinositol-3-kinase-related kinases (PIKK),* wobei DNA-PK keine Lipid-Kinase darstellt (Hartley et al. (1995) Cell 82: 849; Smith & Jackson (1999) Genes and Dev 13: 916; Lempiäinen & Halazonetis (2009) EMBO J. 28: 3067).

Die Proteinkinase ATM (Ataxia-Telangiectasia-mutierte Kinase) gehört ebenfalls zur PIKK-Familie. Auch sie besitzt eine zentrale Bedeutung bei der Erkennung von DNA-Schäden. Patienten, die an Ataxia-Telangiectasia leiden, zeigen u.a. eine erhöhte Empfindlichkeit gegenüber ionisierender Strahlung. (Lavin & Shiloh (1997) Annu. Rev. Immunol. 15: 177; Rotman & Shiloh (1998) Hum. Mol. Genet. 7: 1555).

Es ist durch Izzard et al. (1999) Cancer Res. 59: 2581 beschrieben, dass der PI3-Kinase Inhibitor LY294002 in in-vitro Versuchen die Funktion von DNA-PK inhibiert. Der IC₅₀-Wert (Konzentration bei der 50 % der Enzymaktivität gehemmt ist) liegt bei wenig wirksamen 1,25 µM (5,0 mM ATP). Obwohl der Nachweis, dass der Inhibitor LY294002 Säugerzellen strahlungsempfindlicher werden lässt, d.h. die Zytotoxizität ionisierender Strahlung verstärkt wird, prinzipiell eine Anwendung bei der Bestrahlungstherapie von z.B. soliden Krebstumoren impliziert, konnte für LY294002 zellulär lediglich eine schwache Empfindlichkeitssteigerung gegenüber ionisierender Bestrahlung nachgewiesen werden (Rosenzweig et al. (1999) Clin. Cancer Res. 3: 1149). KuDOS Pharmaceuticals Ltd. haben die Leitstruktur LY294002 optimiert und verschiedene DNA-PK-Inhibitoren vorgestellt. Die Einführung einer Dibenzothiophenylgruppe führte zum Inhibitor NU-7441, einer ATPkompetitiven Verbindung mit einem IC₅₀-Wert von 20.0 nM (Hardcastle et al. (2005) J. Med. Chem. 48: 7829). KU-0060648 vereint inhibitorische Eigenschaften gegenüber DNA-PK mit einem verbesserten Löslichkeitsprofil im wässrigen Medium, jedoch werden die Kinasen der PI3K-Isoenzym-Familie durch KU-0060648 ebenfalls potent gehemmt. Das seit Langem bestehende Bedürfnis nach einem potenten und selektiven DNA-PK-Inhibitor ist folglich bisher nicht befriedigt worden.

WO 2008/028691 A1 offenbart gegen Krebs einsetzbare N-(1-Hetarylpiperidin-4-yl)-(Het)arylamide, wie z.B. 2,3-Dichlor-N-[1-(7-morpholin-4-yl-chinazolin-4-yl)-piperidin-4-yl]-benzamid, die den EP₂-Rezeptor modulieren. Database registry, RNs 1189950-64-8, 118902-41-7, offenbart (2-Methoxyphenyl)-(4-[2-methyl-6-(4-morpholinyl)-4-chinazolinyl]-1-piperazinyl]-methanon und (4-Fluorphenyl)-(4-[2-methyl-6-(4-morpholinyl)-4-chinazolinyl]-1-piperazinyl]-methanon. EP 0566226 A1 beschreibt Chinazolin-Verbindungen, wie z.B. 4-Chlor-6-morpholinochinazolin, das über Morpholinylanthranilsäure und die Chlorierung von 6-Morpholinochinazolin-4-on hergestellt wird. Ebenso beschreibt US 6,265,411 B1 Chinazolin-Verbindungen, wie z.B. 4-Chlor-6-morpholinochinazolin, die ausgehend von Anthranilsäurederivaten und über 3H-Chinazolin-4-one hergestellt werden können. Bicyclische Pyrimidine als PIKK-Inhibitoren sind aus WO 2010/014939 A1 bekannt. DNA-PK-Inhibitoren sind darüber hinaus in WO 2002/020500 A2 beschrieben. US 2009/0069320 A1 und US 2004/0116422 A1 offenbaren die Umsetzung von Anthranilsäurederivaten mit Formamidinacetat in einem Alkohol zu (R5)Halogen-3H-chinazolin-4-onen.

Der Erfindung liegt die Aufgabe zugrunde, die im Stand der Technik aufgezeigten Nachteile zu überwinden und wirksame Inhibitoren der DNA-PK zu entwickeln, die selektiv gegenüber den verwandten Kinasen der PIKK Familie sowie von niedermolekularer Größe sind und insbesondere eine effektive Anwendung in der Krebstherapie als Radio- und Chemosensibilisierer ermöglichen - mit dem Ziel, die therapeutische Wirksamkeit bei gleichzeitiger Verringerung der Nebenwirkungen zu verbessern.

Die Aufgabe der Erfindung wird gemäß den unabhängigen Ansprüchen gelöst. Die Unteransprüche beinhalten bevorzugte Ausführungsformen. Erfindungsgemäß werden Verbindungen der Formel (IA) bereitgestellt 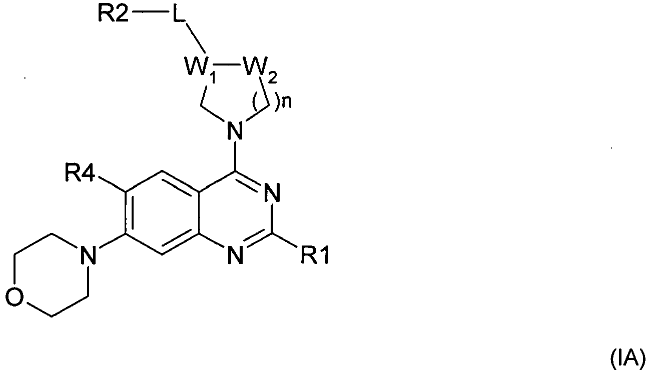 worin
- R1: H, Hal, CN, A, OY, NYY oder -NH-C(NYY)=NY,
- R2: H, Cyc, Ar, Het¹ oder Het²,
- R3: Y, OH oder OA,
- R4: H oder Hal,
- Y: H, A oder Alk-OA,
- W₁: CR3 oder N,
- W₂: CH₂
- L: Einfachbindung, CYR3-, -CO-, -CO-NY-, -NY-CO-, -NY-CO-NY-, -NY-SO₂-, -C(=NR3)-, -C(=N-CN)-, -Alk-, -Alk-NY-, Alk-CO-, -Alk-CO-NY-, -AlkO-, -Alk-OAlk-, -Alk-C(Y)(OY)-, -C(Y)(CN)-, -C(Y)(Het¹)-, -C(R3)(Het¹)-, -C(Y)(Het¹)-NY-, -C(Y)(Het²)-, -C(Y)(OY)-, -(C(Y)(OCOOY)-, -C(Y)(NYY)-, -C(Y)(NY-COY)-, -C(Y)(NY-CO-NYY)-, -C(Y)(OAlk-CN)-, -C(Y)(OAlk-Het²)-, -C(Y)(OAlk-NYY)-, -C(Y)(OAlk-CO-NYY)-, -C(Y)(OY)-Alk-, -C(Y)(OCO-NYY)-, -C(Y)(OCO-NY-Alk-COOY)- oder -C(Y)(OY)-Het¹-Alk-OCO-NY-,
- A: unverzweigtes oder verzweigtes Alkyl mit 1-10 C-Atomen, wobei unabhängig voneinander 1-7 H-Atome durch Hal ersetzt sein können,
- Alk: Alkylen mit 1-6 C-Atomen, wobei unabhängig voneinander 1-4 H-Atome durch Hal und/oder CN ersetzt sein können,
- Cyc: zyklisches Alkyl mit 3-7 C-Atomen, wobei unabhängig voneinander 1-4 H-Atome durch A, Hal und/oder OY ersetzt sein können,
- Ar: unsubstituiertes oder ein-, zwei- oder dreifach durch Y, OY, Hal, CN, COOY, -Alk-OY, NYY, -NY-COY, SiY₃, Cyc und/oder Phenyl substituiertes Phenyl, Naphthyl oder Biphenyl,
- Het¹: ein- oder zweikerniges Heteroaryl mit 2-9 C-Atomen und 1-4 N-, O- und/oder S-Atomen, das unsubstituiert oder ein-, zwei- oder dreifach durch Y, OY, Hal, CN, COOY, -Alk-OY, NYY, -NY-COY, -Alk-Het², -Alk-OCO-Het², -Alk-OCO-NY-Het², -NY-CO-Het², -NY-CO-Alk-Het², SiY₃, Cyc und/oder Phenyl substituiert sein kann,
- Het²: einen einkernigen gesättigten Heterozyklus mit 2-7 C-Atomen und 1-4 N-, O- und/oder S-Atomen, der unsubstituiert oder einfach durch R3 und/oder COY substituiert sein kann,
- Hal: F, Cl, Br oder I, und
- n: 1, 2, 3 oder 4
bedeuten,
und/oder ihre physiologisch unbedenklichen Salze, Tautomere und/oder Stereoisomere, einschließlich deren Mischungen in allen Verhältnissen.

Überraschenderweise hat sich gezeigt, dass die erfindungsgemäßen Verbindungen mit inhibierenden Eigenschaften auf Serin-Threonin-Proteinkinasen versehen sind. Die Verbindungen der Formel (IA) sind durch ihre Kernstruktur des Chinazolins, dem ein Morpholinylrest sowie ein stickstoffhaltiger Heterozyklus anhaften, derart ausgestaltet, dass eine potente und selektive Hemmung von DNA-PK erfolgt. Die erfindungsgemäßen Verbindungen eröffnen damit völlig neue Möglichkeiten hinsichtlich der anticancerogenen Wirkung von Antikrebsmitteln. Bemerkenswerterweise spielen die Verbindungen der Formel (IA) eine therapeutische Rolle als Radio- und Chemosensibilisierer in der Behandlung von Krebs.

Es ist bisher lediglich aus WO 1992/07844 bekannt, dass 2,4-Diaminochinazolin-Derivate Verstärker von chemotherapeutischen Mitteln in der Krebsbehandlung sind. Die Derivate adressieren die Mehrfachresistenz von Tumorzellen infolge von Überexpression des mdr1-Gens, dessen Genprodukt einer Efflux-P-Glycoproteinpumpe die intrazelluläre Wirkstoffkonzentration gering hält. Weder sind physiko-chemische und pharmakologische Daten offenbart, noch ist ein vermarktetes Medikament bekannt. Im Gegensatz dazu enthüllt die vorliegende Erfindung, dass gerade Verbindungen der Formel (IA) zur spezifischen Hemmung von Serin-Threonin-Proteinkinasen wie DNA-PK befähigt sind. Die erfindungsgemäßen Verbindungen und ihre Salze besitzen folglich wertvolle pharmakologische Eigenschaften bei gleichzeitig guter Verträglichkeit. In einer Ausgestaltung der vorliegenden Erfindung ist Pyridylmethoxy in der Definition des Het² -Radikals ausgeschlossen, wenn das R1-Radikal den Rest NYY bedeutet.

Im Rahmen der Erfindung sind die Verbindungen der Formel (IA) so definiert, dass man darunter auch pharmazeutisch verwendbare Derivate, Salze, Hydrate, Solvate, Vorstufen der Verbindungen, Tautomere und optisch aktive Formen (wie z.B. Stereoisomere, Diastereomere, Enantiomere, Racemate) versteht. Unter Solvaten der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate. Die Erfindung umfasst selbstverständlich auch die Solvate der Salze der erfindungsgemäßen Verbindungen. Unter pharmazeutisch verwendbaren Derivaten versteht man z.B. die Salze der erfindungsgemäßen Verbindungen als auch sogenannte Vorstufen der Verbindungen. Unter Vorstufen versteht man z.B. mit Alkyl- oder Acylgruppen, Zuckern oder Oligopeptiden abgewandelte Verbindungen der Formel (IA), die im Organismus rasch zu den wirksamen erfindungsgemäßen Verbindungen gespalten werden. Hierzu gehören auch bioabbaubare Polymerderivate der erfindungsgemäßen Verbindungen, wie dies z.B. in Int. J. Pharm. 115, 61-67 (1995) beschrieben ist. Jegliche Verbindung, die in-vivo in ein bioaktives Mittel, d.h. Verbindungen der Formel (IA) umgewandelt werden kann, ist eine Vorstufe im Sinne dieser Erfindung. Jegliche biologisch aktive Verbindung, die aus der in-vivo Verstoffwechslung einer erfindungsgemäßen Verbindung resultiert, ist ein Metabolit im Sinne der vorliegenden Erfindung. Die Verbindungen der Formel (IA) können ein oder mehrere chirale Zentren besitzen und daher in verschiedenen stereoisomeren Formen vorkommen. Die Formel (IA) schließt alle diese Formen ein.

Gegenstand der Erfindung ist auch die Verwendung von Mischungen der Verbindungen der Formel (IA), z.B. Gemische zweier Diastereomerer, z.B. im Verhältnis 1:1, 1:2, 1:3, 1:4, 1:5, 1:10, 1:100 oder 1:1000. Besonders bevorzugt handelt es sich dabei um Mischungen stereoisomerer Verbindungen.

Vor- und nachstehend haben die Radikale R1, R2, R3, Y, W₁, W₂, L, A, Alk, Cyc, Ar, Het¹, Het² , Hal und n die bei der Formel (IA) angegebenen Bedeutungen, sofern nicht ausdrücklich etwas anderes angegeben ist. Bei mehrfachem Auftreten einzelner Reste innerhalb einer Verbindung oder eines Radikals nehmen die Reste unabhängig voneinander die angegebenen Bedeutungen an, sofern nicht ausdrücklich etwas anderes angegeben ist. Beispielsweise sind die Reste YY im Radikal R1, in dem sie mehrfach vorkommen, identisch oder verschieden, aber vorzugsweise in jedem Fall unabhängig voneinander unter den vorstehend und/oder nachstehend angegebenen Bedeutungen ausgewählt (z.B. Methyl und/oder Ethyl), sofern nicht ausdrücklich etwas anderes angegeben ist. Den vorliegend verwendeten Bezeichnungen zur Definition der Verbindungen liegen im Allgemeinen die Regeln der IUPAC-Organisation für chemische Verbindungen und insbesondere organische Verbindungen zugrunde. Die Bezeichnungen zur Erläuterung der o.g. Verbindungen der Erfindung haben immer die nachstehenden Bedeutungen, sofern die Beschreibung oder die Ansprüche nicht etwas anderes anzeigen.

Der Begriff "unsubstituiert" bedeutet, dass ein Radikal, eine Gruppe oder eine Rest keine Substituenten trägt. Der Begriff "substituiert" bedeutet, dass ein Radikal, eine Gruppe oder ein Rest einen oder mehrere Substituenten trägt.

"Alkyl" oder "A" bezeichnet im Sinne der Erfindung ein nicht-zyklisches, gesättigtes oder ungesättigtes Kohlenwasserstoffradikal, das unverzweigt (linear) oder verzweigt ist und vorzugsweise 1, 2, 3, 4, 5, 6, 7, 8, 9 oder 10 C-Atome besitzt, d.h. C₁₋₁₀-Alkanyl. Beispiele für Alkylradikale sind Methyl, Ethyl, Propyl, Isopropyl, 1,1-, 1,2- oder 2,2-Dimethylpropyl, 1-Ethylpropyl, 1-Ethyl-1-methylpropyl, 1-Ethyl-2-methylpropyl, 1,1,2- oder 1,2,2-Trimethyl-propyl, Butyl, Isobutyl, sek.-Butyl, tert.-Butyl, 1-, 2- oder 3-Methylbutyl, 1,1-, 1,2-, 1,3-, 2,2-, 2,3- oder 3,3-Dimethylbutyl, 1- oder 2-Ethylbutyl, Pentyl, Isopentyl, Neopentyl, tert.-Pentyl, 1-, 2-, 3- oder 4-Methylpentyl, Hexyl.

In einer bevorzugten Ausführungsform der Erfindung ist "A" unverzweigtes oder verzweigtes Alkyl mit 1-10 C-Atomen, wobei unabhängig voneinander 1-7 H-Atome durch Hal ersetzt sein können. Besonders bevorzugt als "A" ist unverzweigtes oder verzweigtes Alkyl mit 1-6 C-Atomen, wobei unabhängig voneinander 1-5 H-Atome durch Hal ersetzt sein können. Ganz besonders bevorzugt ist C₁₋₄-Alkyl. Ein C₁₋₄-Alkyl ist beispielsweise Methyl, Ethyl, Propyl, Isopropyl, Butyl, Isobutyl, sek.-Butyl, tert.-Butyl, Fluormethyl, Difluormethyl, Trifluormethyl, Pentafluorethyl, 1,1,1-Trifluorethyl oder Brommethyl, höchst bevorzugt Methyl, Ethyl oder Trifluoromethyl. Es versteht sich, dass die jeweiligen Bedeutungen von "A" unabhängig voneinander in den Radikalen R1, R3, Y und Cyc sind.

"Cycloalkyl" oder "Cyc" bezeichnet im Sinne der Erfindung gesättigte und teilweise ungesättigte nicht-aromatische zyklische Kohlenwasserstoffgruppen mit 1 bis 3 Ringen, die 3 bis 20, vorzugsweise 3 bis 12, besonders bevorzugt 3 bis 9 C-Atome umfassen. Die Bindung an das Grundgerüst der Formel (IA) kann über jedes Ringmitglied der Cycloalkylgruppe erfolgen. Beispiele für geeignetes Cycloalkyl sind Cyclopropyl, Cyclobutyl, Cyclopentyl, Cyclohexyl, Cycloheptyl, Cyclooctyl, Cyclodecyl, Cyclopentenyl, Cyclohexenyl und Cyclooctadienyl.

In einer bevorzugten Ausführungsform der Erfindung ist "Cyc" zyklisches Alkyl mit 3-7 C-Atomen, wobei unabhängig voneinander 1-4 H-Atome durch A, Hal und/oder OY ersetzt sein können. Besonders bevorzugt ist zyklisches Alkyl mit 3-6 C-Atomen. Es versteht sich, dass die jeweiligen Bedeutungen von "Cyc" unabhängig voneinander in den Radikalen R2, Ar und Het¹ sind.

Grundgerüst der Formel (IA) ist hierbei jede generische oder nicht-generische Struktur, an die irgendein Radikal im Sinne der Erfindung, wie z.B. Cyc, Ar, Het¹ oder Het², gebunden werden kann, um zu einer erfindungsgemäßen Verbindung der Formel (IA) zu gelangen.

Der Begriff "Alk" bezeichnet im Sinne der Erfindung unverzweigtes oder verzweigtes Alkylen, Alkenyl oder Alkynyl mit 1, 2, 3, 4, 5 oder 6 C-Atomen, d.h. C₁₋₆-Alkylene, C₂₋₆-Alkenyle und C₂₋₆-Alkynyle. Alkenyle haben mindestens eine C-C Doppelbindung and Alkynyle mindestens eine C-C Dreifachbindung. Alkynyle können außerdem mindestens eine C-C Doppelbindung aufweisen. Beispiele für geeignete Alkylene sind Methylen, Ethylen, Propylen, Butylen, Pentylen, Hexylen, Isopropylen, Isobutylen, sek.-Butylen, 1-, 2- oder 3-Methylbutylen, 1,1-, 1,2- oder 2,2-Dimethylpropylen, 1-Ethylpropylen, 1-, 2-, 3- oder 4-Methylpentylen, 1,1-, 1,2-, 1,3-, 2,2-, 2,3- oder 3,3-Dimethylbutylen, 1- oder 2- Ethylbutylen, 1-Ethyl-1-methylpropylen, 1-Ethyl-2-methylpropylen, 1,1,2- oder 1,2,2-Trimethylpropylen. Beispiele für geeignete Alkenyle sind Allyl, Vinyl, Propenyl (-CH₂CH=CH₂; -CH=CH-CH₃; -C(=CH₂)-CH₃), 1-, 2- oder 3-Butenyl, Isobutenyl, 2-Methyl-1- oder 2-Butenyl, 3-Methyl-1-butenyl, 1,3-Butadienyl, 2-Methyl-1,3-butadienyl, 2,3-Dimethyl-1,3-butadienyl, 1-, 2-, 3- oder 4-Pentenyl and Hexenyl. Beispiele für geeignete Alkynyle sind Ethynyl, Propynyl (-CH₂-C≡CH; -C≡C-CH₃), 1-, 2- oder 3-Butynyl, Pentynyl, Hexynyl oder Pent-3-en-1-in-yl, insbesondere Propynyl.

In einer bevorzugten Ausführungsform der Erfindung ist "Alk" Alkylen mit 1-6 C-Atomen, d.h. Methylen, Ethylen, Propylen, Butylen, Pentylen oder Hexylen, wobei unabhängig voneinander 1-4 H-Atome durch Hal und/oder CN ersetzt sein können. Es ist besonders bevorzugt, dass "Alk" Alkylen mit 1-3 C-Atomen, wobei 1-2 H-Atome durch Hal ersetzt sein können, bedeutet. Besondere Beispiele hierfür sind Methylen, Ethylen und Propylen. Es versteht sich, dass die jeweiligen Bedeutungen von "Alk" in den Radikalen Y, L, Ar und Het¹ unabhängig voneinander sind.

Der Begriff "Aryl", "Carboaryl" oder "Ar" bezeichnet im Sinne der Erfindung ein mono- oder polyzyklisches aromatisches Kohlenwasserstoffsystem mit 3 bis 14, vorzugsweise 4 bis 10, besonders bevorzugt 5 bis 8 C-Atomen, die gegebenenfalls substituiert sein können. Der Begriff "Aryl" schließt solche Systeme ein, in denen der aromatische Zyklus Teil eines bi- oder polyzyklischen gesättigten, teilweise ungesättigten und/oder aromatischen Systems ist, z.B. wenn der aromatische Zyklus an "Aryl", "Cycloalkyl", "Heteroaryl" oder "Heterocyclyl" über ein beliebiges Ringmitglied des Arylradikals fusioniert ist. Die Bindung an das Grundgerüst der Formel (IA) kann über jedes Ringmitglied der Arylgruppe erfolgen. Beispiele für geeignetes "Aryl" sind Phenyl, Biphenyl, Naphthyl, 1-Naphthyl, 2-Naphthyl, Anthracenyl, In-danyl, In-denyl, 1,2,3,4-Tetrahydronaphthyl, insbesondere Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Trifluormethylphenyl, o-, m- oder p-Fluorphenyl, o-, m- oder p-Bromphenyl, o-, m- oder p-Chlorphenyl, o-, m- oder p-Hydroxyphenyl, o-, m- oder p-Methoxyphenyl, o-, m- oder p-Methylsulfonylphenyl, o-, m- oder p-Nitrophenyl, o-, m- oder p-Aminophenyl, o-, m- oder p-Methylaminophenyl, o-, m- oder p-Dimethylaminophenyl, o-, m- oder p-Aminosulfonylphenyl, o-, m- oder p-Methylaminosulfonylphenyl, o-, m- oder p-Aminocarbonylphenyl, o-, m- oder p-Carboxyphenyl, o-, m- oder p-Methoxycarbonylphenyl, o-, m- oder p-Ethoxycarbonylphenyl, o-, m- oder p-Acetylphenyl, o-, m- oder p-Formylphenyl, o-, m- oder p-Cyanphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3,4-, 2,3,5-, 2,3,6-, 2,4,6- oder 3,4,5-Trichlorphenyl, p-Iodphenyl, 4-Fluor-3-chlorphenyl, 2-Fluor-4-bromphenyl, 2,5-Difluor-4-bromphenyl oder 2,5-Dimethyl-4-chlorphenyl.

In einer bevorzugten Ausführungsform der Erfindung ist "Ar" unsubstituiertes oder ein-, zwei- oder dreifach durch Y, OY, Hal, CN, COOY, -Alk-OY, NYY, -NY-COY, SiY₃, Cyc und/oder Phenyl substituiertes Phenyl, Naphthyl oder Biphenyl. Es ist besonders bevorzugt, dass "Ar" unsubstituiertes oder ein- oder zweifach durch R3 oder Hal substituiertes Phenyl bedeutet, ganz besonders bevorzugt unsubstituiertes oder ein- oder zweifach durch A, OA oder Hal substituiertes Phenyl, höchst bevorzugt unsubstituiertes oder ein- oder zweifach durch Methyl, Methoxy oder Trifluoromethyl substituiertes Phenyl.

Der Begriff "Heteroaryl" bezeichnet im Sinne der Erfindung ein 2 bis 15, vorzugsweise 2 bis 9, besonders bevorzugt 5-, 6- oder 7-gliedriges mono- oder polyzyklisches aromatisches Kohlenwasserstoffradikal, das mindestens 1, wenn passend auch 2, 3, 4 oder 5 Heteroatome umfasst, insbesondere Stickstoff, Sauerstoff und/oder Schwefel, wobei die Heteroatome identisch oder verschieden sind. Die Anzahl der Stickstoffatome ist vorzugsweise 0, 1, 2, 3 oder 4, und die der Sauerstoff- und Schwefelatome ist unabhängig voneinander 0 oder 1. Der Begriff "Heteroaryl" schließt solche Systeme ein, in denen der aromatische Zyklus Teil eines bi- oder polyzyklischen gesättigten, teilweise ungesättigten und/oder aromatischen Systems ist, z.B. wenn der aromatische Zyklus an "Aryl", "Cycloalkyl", "Heteroaryl" oder "Heterocyclyl" über ein beliebiges Ringmitglied des Heteroarylradikals fusioniert ist. Die Bindung an das Grundgerüst der Formel (IA) kann über jedes Ringmitglied der Heteroarylgruppe erfolgen, sofern es chemisch sinnvoll erscheint, wobei eine Bindung über die C-Atome bevorzugt ist.

"Heteroaryl" bedeutet, ungeachtet weiterer Substitutionen, z.B. 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, 1,2,3-Triazol-1-, -4- oder -5-yl, 1,2,4-Triazol-1-, -3- oder 5-yl, 1- oder 5-Tetrazolyl, 1,2,3-Oxadiazol-4-oder-5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 2-, 3-, 4-, 5-, 6- oder 7-Indazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]-oxazinyl, 1,3-Benzodioxol-5-yl, 1,4-Benzodioxan-6-yl, 2,1,3-Benzothiadiazol-4- oder -5-yl, 2,1,3-Benzoxadiazol-5-yl, Imidazolyl, Triazinyl, Phthalazinyl, Indolizinyl, Pteridinyl, Carbazolyl, Phenazinyl, Phenoxazinyl, Phenothiazinyl oder Acridinyl.

Die heterozyklischen Reste können auch teilweise oder vollständig hydriert sein. Unsubstituiertes Heteroaryl kann also z.B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4-oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder -8-isochinolyl, 2-, 3-, 5-, 6-, 7- oder 8-3,4-Dihydro-2H-benzo[1,4]oxazinyl, 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylen-dioxy)phenyl, oder auch 3,4-Dihydro-2H-1,5-benzodioxepin-6- oder -7-yl, 2,3-Dihydro-benzofuranyl oder 2,3-Dihydro-2-oxo-furanyl.

Es ist bevorzugt, dass "Heteroaryl" im Rahmen von "Het¹" einen ein- oder zweikernigen aromatischen Heterozyklus mit 2-9 C-Atomen und 1-4 N-, O- und/oder S-Atomen, der unsubstituiert oder ein-, zwei- oder dreifach durch Y, OY, Hal, CN, COOY, -Alk-OY, NYY, -NY-COY, -Alk-Het², -Alk-OCO-Het², -Alk-OCO-NY-Het², -NY-CO-Het², -NY-CO-Alk-Het²,-NY-CO-Het¹, SiY₃, Cyc und/oder Phenyl substituiert sein kann, bedeutet. Es ist besonders bevorzugt, dass "Het¹" ein- oder zweikerniges Heteroaryl mit 2-8 C-Atomen und 1-3 N-, O- und/oder S-Atomen, das unsubstituiert oder ein- oder zweifach durch Y oder Hal substituiert sein kann, bedeutet. Ganz besonders bevorzugt ist "Het¹" unsubstituiertes oder ein- oder zweifach durch Y oder Hal substituiertes Heteroaryl, ausgewählt aus der Gruppe:

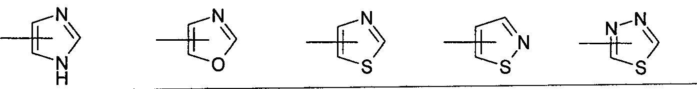

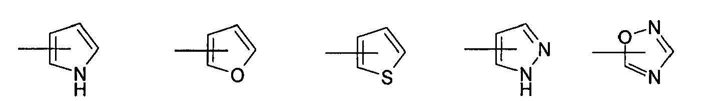

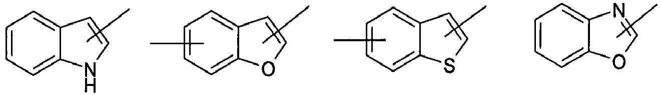

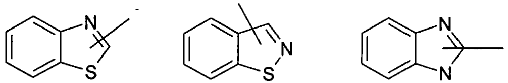

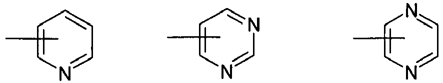

Es versteht sich, dass die in den vorstehend und nachstehend bevorzugten Strukturen eingezeichnete Bindung lediglich die Verknüpfung mit dem Grundgerüst der Formel (IA) symbolisiert, ohne auf ein oder mehrere bestimmte Ringatome beschränkt zu sein. Insbesondere soll im Fall eines Bizyklus die Verknüpfung nicht auf den die gezeichnete Bindung enthaltenen Ring beschränkt sein, obwohl dieser Ring als bevorzugt anzusehen ist. Höchst bevorzugt bedeutet "Het¹" unsubstituiertes oder ein- oder zweifach durch A oder Hal substituiertes Heteroaryl, ausgewählt aus der Gruppe:

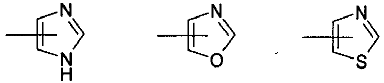

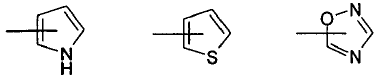

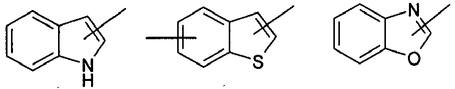

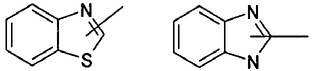

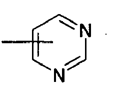

Es versteht sich, dass die jeweiligen Bedeutungen von "Het¹" in den Radikalen R2 und L unabhängig voneinander sind.

Der Begriff "Heterozyklus" bezeichnet im Sinne der Erfindung ein mono- oder polyzyklisches System mit 3 bis 20 Ringatomen, vorzugsweise 3 bis 14 Ringatomen, besonders bevorzugt 3 bis 10 Ringatomen, umfassend C-Atome und 1, 2, 3, 4 oder 5 Heteroatome, insbesondere Stickstoff, Sauerstoff und/oder Schwefel, wobei die Heteroatome identisch oder verschieden sind. Das zyklische System kann gesättigt oder ein- oder mehrfach ungesättigt sein. Der Begriff "Heteroaryl" schließt solche Systeme ein, in denen der aromatische Zyklus Teil eines bi- oder polyzyklischen gesättigten, teilweise ungesättigten und/oder aromatischen Systems ist, z.B. wenn der aromatische Zyklus an "Aryl", "Cycloalkyl", "Heteroaryl" oder "Heterocyclyl" über ein beliebiges Ringmitglied des Heterozyklus fusioniert ist. Die Bindung an das Grundgerüst der Formel (IA) kann über jedes Ringmitglied des Heterozyklus erfolgen. Beispiele für geeignete Heterozyklen sind Pyrrolidinyl, Thiapyrrolidinyl, Piperidinyl, Piperazinyl, Oxapiperazinyl, Oxapiperidinyl, Oxadiazolyl, Tetrahydrofuryl, Imidazolidinyl, Thiazolidinyl, Tetrahydropyranyl, Morpholinyl, Tetrahydrothiophenyl, Dihydropyranyl.

In einer Ausgestaltung der Erfindung ist "Het²" ein einkerniger gesättigter Heterozyklus mit 2-7 C-Atomen und 1-4 N-, O- und/oder S-Atomen, der unsubstituiert oder einfach durch R3 und/oder COY substituiert sein kann. Es ist bevorzugt, dass "Het²" einen einkernigen gesättigten Heterozyklus mit 2-5 C-Atomen und 1-2 N- und/oder O-Atomen, der unsubstituiert oder einfach durch A substituiert sein kann, bedeutet, besonders bevorzugt einen unsubstituierten oder einfach durch A substituierten Heterozyklus, ausgewählt aus der Gruppe: 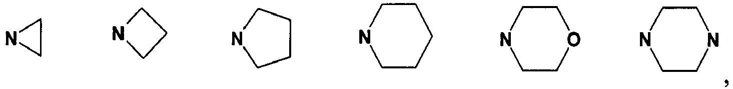 ganz besonders bevorzugt Pyrrolidinyl, Piperidinyl, Morpholinyl oder einfach durch A substituiertes Piperazinyl, höchst bevorzugt Pyrrolidinyl, Piperidinyl oder Morpholinyl. Es versteht sich, dass die jeweiligen Bedeutungen von "Het²" in den Radikalen R2, L und Het¹ unabhängig voneinander sind.

Der Begriff "Halogen", "Halogenatom", "Halogensubstituent" oder "Hal" bezeichnet im Sinne der Erfindung ein oder mehrere Atome von Fluor (F), Brom (Br), Chlor (Cl) oder Iod (I). Die Bezeichnungen "Dihalogen", "Trihalogen" und "Perhalogen" beziehen sich auf zwei, drei oder vier Substituenten, wobei jeder Substituent unabhängig voneinander aus der Gruppe von F, Cl, Br oder I ausgewählt werden kann. "Halogen" meint vorzugsweise F, Cl oder Br. F und Cl sind besonders bevorzugt, insbesondere wenn die Halogene an einer Alkyl-(Haloalkyl) oder Alkoxygruppe substituiert sind (z.B. CF₃ and CF₃O).

Der Index n ist kann die Werte 1, 2, 3 oder 4 annehmen, vorzugsweise 1, 2 oder 3, besonders bevorzugt 2.

Das Radikal R1 bedeutet vorzugsweise H, Hal, CN, A, OA oder NH₂, besonders bevorzugt H, Hal oder A.

Das Radikal R3 bedeutet vorzugsweise A, Alk-OA, OH oder OA, besonders bevorzugt OH oder OA.

Das Radikal Y bedeutet vorzugsweise H oder A.

In den erfindungsgemäßen Verbindungen der Formel (IA) bedeutet W₂ eine CH₂-Gruppe. Es ist ebenso bevorzugt, dass W₁ eine CH- oder N-Gruppe und W₂ eine CH₂-Gruppe bedeuten, während in einer anderen bevorzugten Ausführungsform W₁ eine CR3-Gruppe und W₂ eine CH₂-Gruppe bedeuten. Pyrrolidin, Piperidin, Azepan, Imidazolidin,

Hexahydropyrimidin und [1,3]Diazepan sind folglich besondere Ausgestaltungen der Teilformel 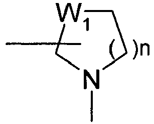 Besonders bevorzugt sind W₁ und W₂ CH bzw. CH₂. Insofern sind die ringförmigen sekundären Amine Pyrrolidin, Piperidin und Azepan ganz besonders bevorzugte Ausgestaltungen der vorgenannten Teilformel. Höchst bevorzugt ist Piperidin, welches optional durch -L-R2 substituiert sein.

In noch einer bevorzugten Ausführungsform der Erfindung ist der Linker L eine Einfachbindung, -CYR3-, -CO-, -CO-NY-, -NY-CO-, -NY-SO₂-, -Alk- oder -Alk-OAlk-.

Es ist eine weitere bevorzugte Ausführungsform der Erfindung, worin der Linker unsubstituiert bleibt oder mit einem Aromaten substituiert ist, so dass R2 insbesondere H, Ar, Het¹ oder Het² bedeutet, besonders bevorzugt H, Ar oder Het¹.

Dementsprechend sind Gegenstand der Erfindung diejenigen Verbindungen der Formel (IA), in denen mindestens eines der genannten Radikale eine der vorstehend angegebenen Bedeutungen hat. Nicht näher bezeichneten Radikale im Kontext einer Ausführungsform der Formel (IA), Teilformel davon oder irgendeines Restes daran sollen die bei der Formel (IA) angegebene Bedeutung haben, wie sie hierin offenbart ist, um die Aufgabe der Erfindung zu lösen. Das heißt, dass die genannten Radikale sämtliche ihnen zugeordneten Bedeutungen annehmen können, wie sie vor- oder nachstehend beschrieben sind, einschließlich jeglicher bevorzugter Ausführungsformen, ohne auf diese beschränkt zu sein und unabhängig von ihrem Auftreten in einem anderen bestimmten Kontext. Es versteht sich insbesondere, dass jede Ausführungsform eines bestimmten Radikals mit jeder Ausführungsform eines oder mehrerer anderer Radikale kombiniert werden kann.

Gemäß der vorliegenden Erfindung werden entsprechend Morpholinylchinazolin-Derivate der Formel (IA) bereitgestellt 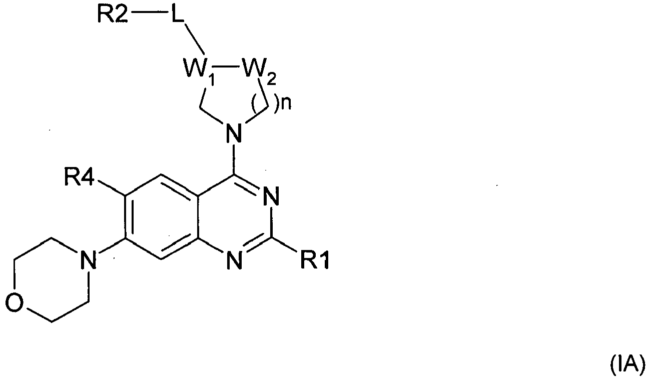 worin
W₁ CR3 oder N, und
W₂ CH₂ bedeutet, und
R1, R2, R3, R4, Y, L, A, Alk, Cyc, Ar, Het¹, Het², Hal und n die oben angegebene Bedeutung aufweisen,
und/oder ihre physiologisch unbedenklichen Salze, Tautomere und/oder Stereoisomere, einschließlich deren Mischungen in allen Verhältnissen.

Die CR3-Gruppe in W₁ ist vorzugsweise als CH-Gruppe ausgestaltet, so dass CH oder N für W₁ bevorzugt sind, besonders bevorzugt CH. In einer anderen Ausführungsform von W₁ kann es ausschließlich CR3 bedeuten.

In einer besonders bevorzugten Ausgestaltung der vorliegenden Erfindung werden Morpholinylchinazolin-Derivate der Teilformel (IB) bereitgestellt 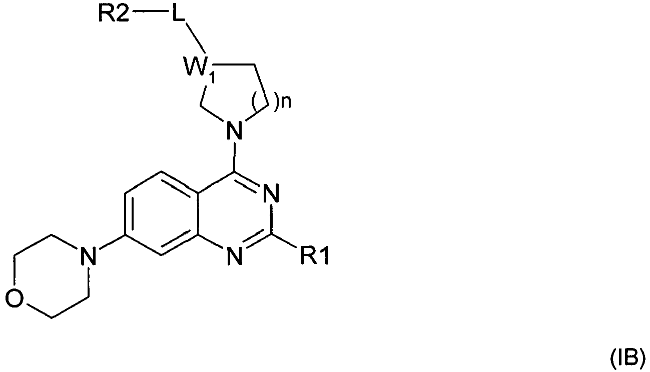 worin
W₁ CH oder N,
   - R1: H, Hal, CN, A, OA oder NH₂,
   - R2: H, Ar, Het¹ oder Het²,
   - R3: A, Alk-OH, OH oder OA,
   - Y: H, A oder Alk-OA, L Einfachbindung, -CYR3-, -CO-, -CO-NY-, -NY-CO-, -NY-SO₂-, -Alk- oder -Alk-OAlk-,
   - A: unverzweigtes oder verzweigtes Alkyl mit 1-4 C-Atomen, wobei unabhängig voneinander 1-5 H-Atome durch Hal ersetzt sein können,
   - Alk: Alkylen mit 1-3 C-Atomen, wobei 1-2 H-Atome durch Hal ersetzt sein können,
   - Ar: unsubstituiertes oder ein- oder zweifach durch R3 oder Hal substituiertes Phenyl,
   - Het¹: unsubstituiertes oder ein- oder zweifach durch Y oder Hal substituiertes Heteroaryl ausgewählt aus der Gruppe:

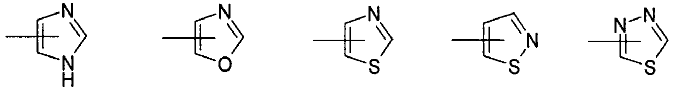

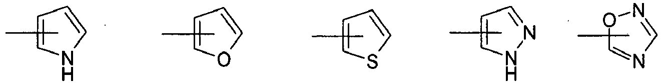

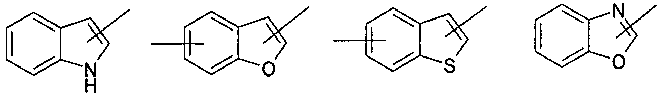

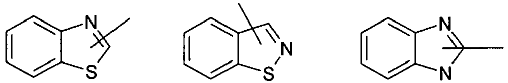

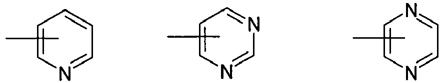

- Het²: einen unsubstituierten oder einfach durch A substituierten Heterozyklus aus der Gruppe:

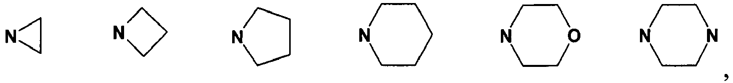

- Hal: F, Cl oder Br, und
- n: 1, 2 oder 3
bedeuten,
und/oder ihre physiologisch unbedenklichen Salze, Tautomere und/oder Stereoisomere, einschließlich deren Mischungen in allen Verhältnissen.

In einer ganz besonders bevorzugten Ausgestaltung der vorliegenden Erfindung werden Morpholinylchinazolin-Derivate der Teilformel (IC) bereitgestellt 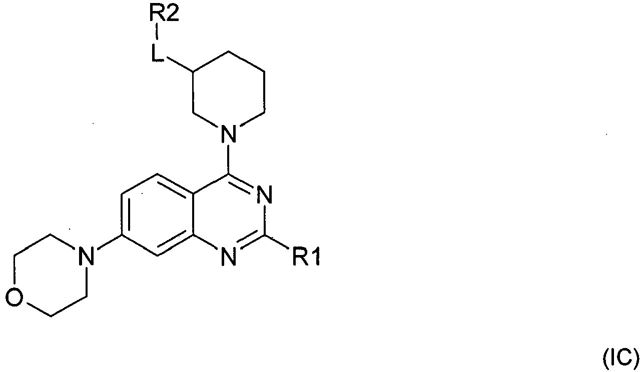 worin
- R1: H, Hal oder A,
- R2: H, Ar oder Het¹,
- Y: H oder A,
- L: Einfachbindung, -C(Y)(OH)-, -CO-, -CO-NH-, -NH-CO-, -NH-SO₂ oder -Alk-,
- A: unverzweigtes oder verzweigtes Alkyl mit 1-3 C-Atomen, wobei unabhängig voneinander 1-3 H-Atome durch Hal ersetzt sein können,
- Alk: Alkylen mit 1-2 C-Atomen, wobei 1-2 H-Atome durch Hal ersetzt sein können,
- Ar: unsubstituiertes oder ein- oder zweifach durch A, OA oder Hal substituiertes Phenyl,
- Het¹: unsubstituiertes oder ein- oder zweifach durch A oder Hal substituiertes Heteroaryl aus der Gruppe:

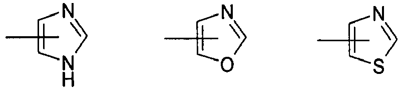

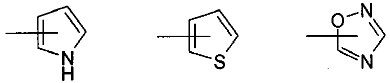

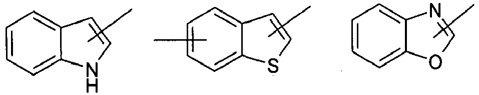

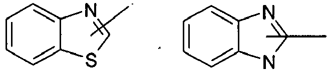

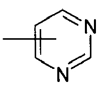

- Het²: Pyrrolidinyl, Piperidinyl oder Morpholinyl, und
- Hal: F oder Cl
bedeuten,
und/oder ihre physiologisch unbedenklichen Salze, Tautomere und/oder Stereoisomere, einschließlich deren Mischungen in allen Verhältnissen.

Höchst bevorzugt sind solche Verbindungen der Formeln (IA), (IB) und (IC), die in Tabelle 1 zusammengestellt sind.

**Tab. 1: Bevorzugte Verbindungen der Formeln (IA), (IB) und (IC)**

| | |
|---|---|
| 1 | 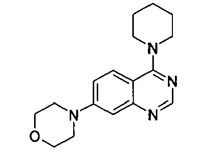 |
| 2 | 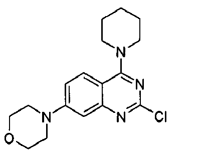 |
| 4 | 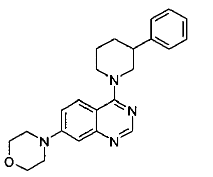 |
| 6 | 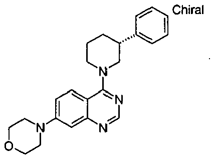 |
| 13 | 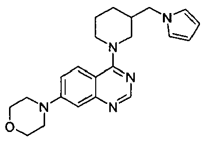 |
| 14 | 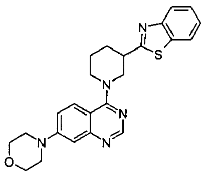 |
| 22 | 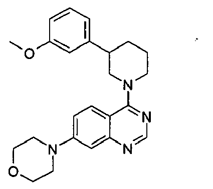 |
| 23 | 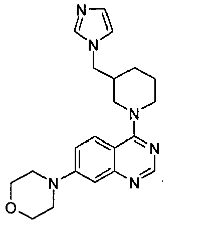 |
| 25 | 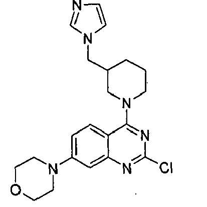 |
| 27 | 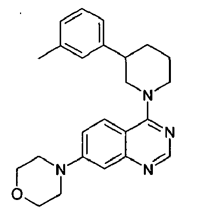 |
| 29 | 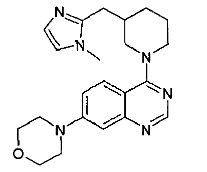 |
| 30 | 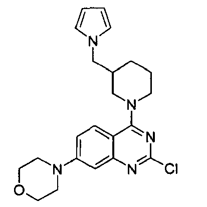 |
| 31 | 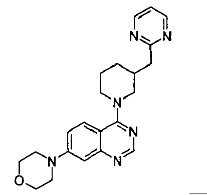 |
| 32 | 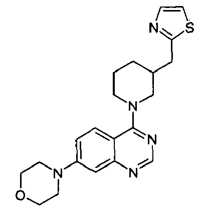 |
| 33 | 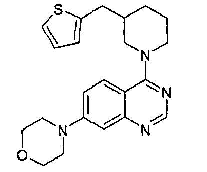 |
| 36 | 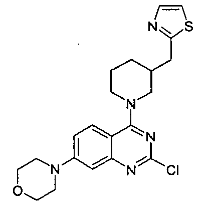 |
| 38 | 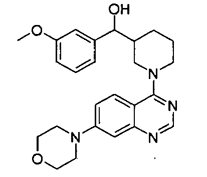 |
| 40 | 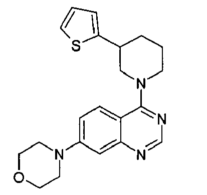 |
| 41 | 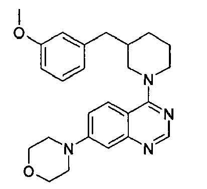 |
| 44 | 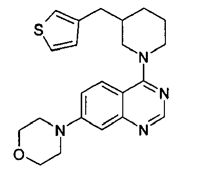 |
| 45 | 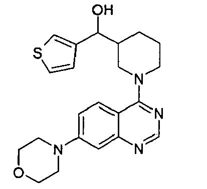 |
| 46 | 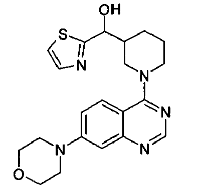 |
| 47 | 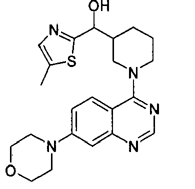 |
| 48 | 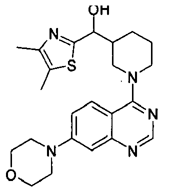 |
| 49 | 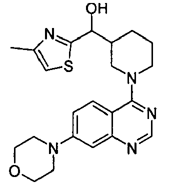 |
| 50 | 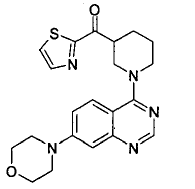 |
| 51 | 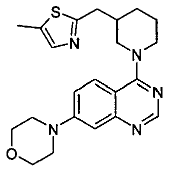 |
| 52 | 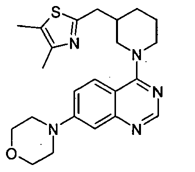 |
| 53 | 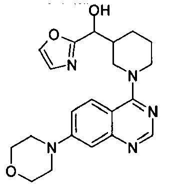 |
| 56 | 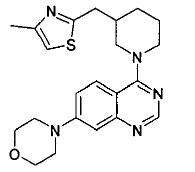 |
| 59 | 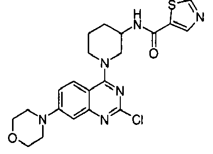 |
| 60 | 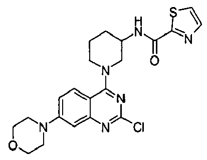 |
| 65 | 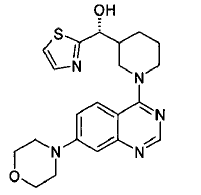 |
| 66 | 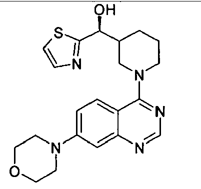 |
| 67 | 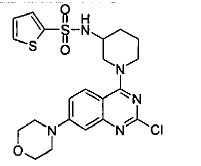 |
| 70 | 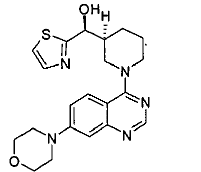 |

Die Verbindungen der Formel (IA) und auch die Ausgangsstoffe zu ihrer Herstellung werden nach an sich bekannten Methoden hergestellt, wie sie in der Literatur (z.B. in Standardwerken wie Houben-Weyl, Methoden der organischen Chemie, Georg-Thieme-Verlag, Stuttgart) beschrieben und/oder Fachmann bekannt sind, sowie unter Reaktionsbedingungen, die für die genannten Umsetzungen bekannt und geeignet sind. Dabei kann man auch von an sich bekannten, hier nicht näher erwähnten Varianten Gebrauch machen.

Die Reaktionszeit liegt je nach angewendeten Bedingungen zwischen einigen Minuten und 14 Tagen, die Reaktionstemperatur zwischen -15°C und 150°C, normalerweise zwischen 10°C und 100°C, besonders bevorzugt zwischen 20°C und 70°C.

Die Umsetzung erfolgt in einem inerten Lösungsmittel und in der Regel in Gegenwart eines säurebindenden Mittels, vorzugsweise einer organischen Base wie DIPEA, Triethylamin, Dimethylanilin, Pyridin, Chinolin, Piperidin oder Diethanolamin. Auch der Zusatz eines Alkali- oder Erdalkalimetallhydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein. Als Basen eignen sich Metalloxide, wie z.B. Aluminiumoxid, Alkalimetallhydroxide (darunter Kaliumhydroxid, Natriumhydroxid und Lithiumhydroxid), Erdalkalimetallhydroxide (z.B. Bariumhydroxid und Calciumhydroxid) und Alkalimetallalkoholate (z.B. Kaliumethanolat und Natriumpropanolat).

Als inerte Lösungsmittel eignen sich u.a. Kohlenwasserstoffe wie Hexan, Petrolether, Benzol, Toluol oder Xylol; chlorierte Kohlenwasserstoffe wie Trichlorethylen, 1,2-Dichlorethan, Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel. Besonders bevorzugt sind Glykolether, wie Ethylenglycolmonomethylether, THF, Dichlormethan und/oder DMF.

Die Verbindungen der Formel (IA) können vorzugsweise erhalten werden, indem man eine Verbindung der Formel (II) mit einer Verbindung der Formel (III) umsetzt. Gegenstand der vorliegenden Erfindung ist damit auch ein Verfahren zum Herstellen von Verbindungen der

Formel (IA) und/oder ihrer physiologisch unbedenklichen Salze, Tautomere und/oder Stereoisomere mit den folgenden Schritten:
(a) Umsetzen einer Verbindung der Formel (II) 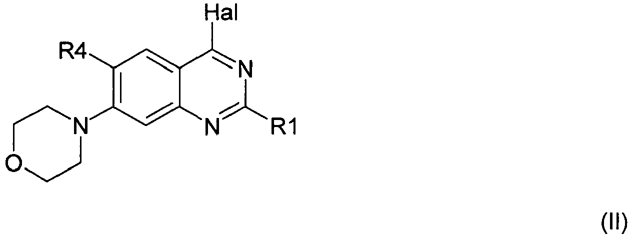 worin R1, R4, Y, A, Alk und Hal die oben angegebene Bedeutung aufweisen, mit einer Verbindung der Formel (III) 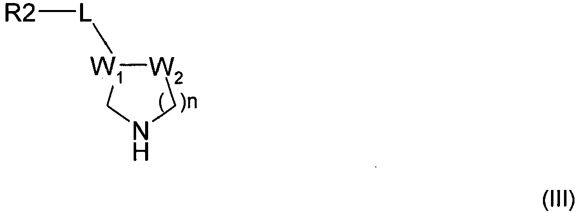 worin R2, R3, Y, W₁, W₂, L, A, Alk, Cyc, Ar, Het¹, Het², Hal und n die oben angegebene Bedeutung aufweisen,
   unter Erhalt der Verbindungen der Formel (IA) 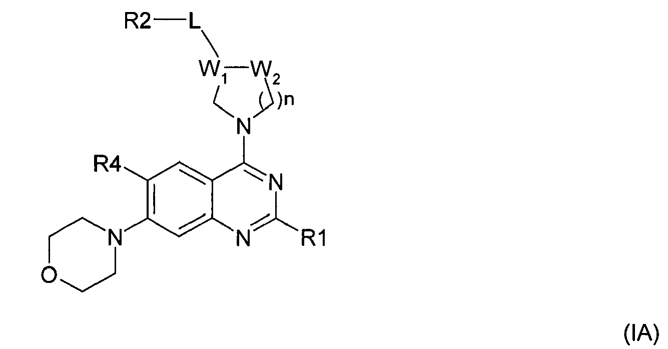 worin R1, R2, R3, R4, Y, W₁, W₂, L, A, Alk, Cyc, Ar, Het¹, Het², Hal und n die oben angegebene Bedeutung aufweisen,
   und gegebenenfalls
(b) Umwandeln einer Base oder Säure der Verbindungen der Formel (IA) in eines ihrer Salze.

Die Erfindung betrifft auch Zwischenverbindungen der Formel (II) 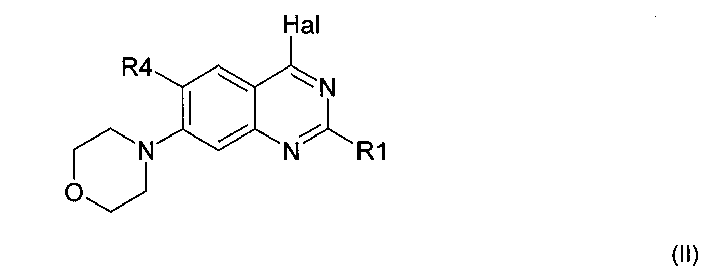 worin
R1 H, CN, A, OY, NYY oder -NH-C(NYY)=NY bedeutet, und
R4, Y, A, Alk und Hal die oben angegebene Bedeutung aufweisen,
und/oder ihre physiologisch unbedenklichen Salze, Tautomere und/oder Stereoisomere, einschließlich deren Mischungen in allen Verhältnissen.

In einer besonders bevorzugten Ausführungsform der vorliegenden Erfindung werden Zwischenverbindungen mit der Teilformel (IIB) bereitgestellt 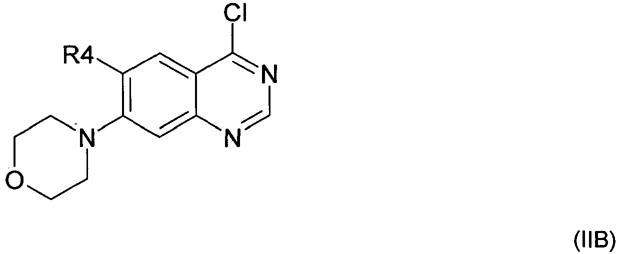 worin R4 und Hal die oben angegebene Bedeutung aufweisen, wobei R4 insbesondere H ist, und/oder ihre physiologisch unbedenklichen Salze, Tautomere und/oder Stereoisomere, einschließlich deren Mischungen in allen Verhältnissen.
Gegenstand der Erfindung ist auch ein Verfahren zum Herstellen von Zwischenverbindungen der Formel (II), wobei eine Verbindung der Formel (IIC) 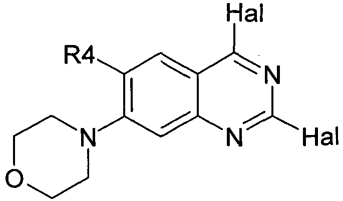 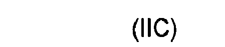
worin R4 und Hal die oben angegebene Bedeutung aufweisen,
mit einer Verbindung X-R1, worin
X ein Element der 1. Hauptgruppe, und
R1 H, CN, A, OY, NYY oder -NH-C(NYY)=NY bedeutet,
unter der Maßgabe, dass H₂ für X-R1 ausgeschlossen ist, und
Y, A, Alk und Hal die oben angegebene Bedeutung aufweisen,
unter Erhalt der Zwischenverbindungen der Formel (II) 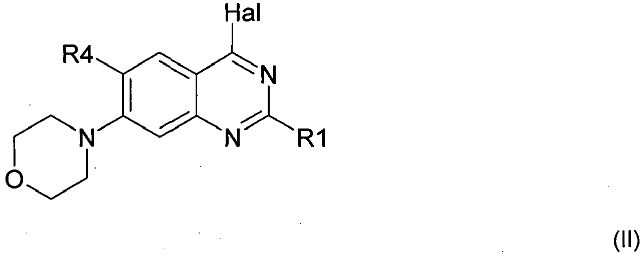 worin R1, R4, Y, A, Alk und Hal die unter Formel (IIC) angegebene Bedeutung aufweisen, umgesetzt wird.

Offenbart ist auch ein Verfahren zum Herstellen von Zwischenverbindungen der Teilformel (IIB), wobei eine Verbindung der Formel (IID) 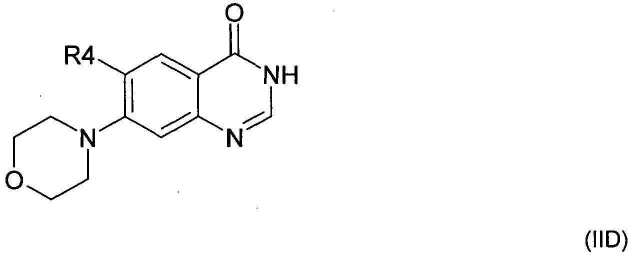 worin R4 und Hal die oben angegebene Bedeutung aufweisen,
mit einem Halogenierungsmittel in einem Amin, vorzugsweise einem organischen Amin, unter Erhalt der Zwischenverbindungen der Teilformel (IIB) 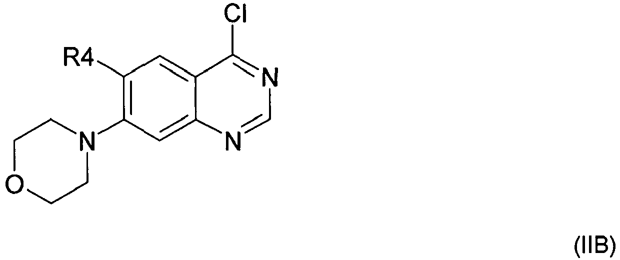 worin R4 und Hal die oben angegebene Bedeutung aufweisen,
umgesetzt wird.

Offenbart wird weiterhin ein Verfahren zum Herstellen von Verbindungen der Formel (II) mit den folgenden Schritten:
(a) Umsetzen einer Verbindung der Formel (IIE) 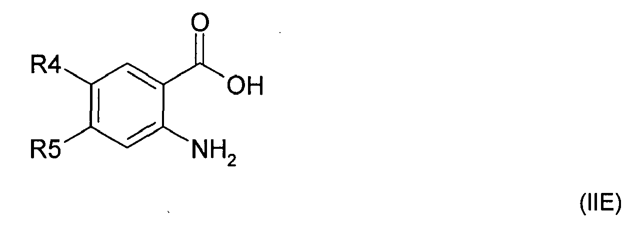 worin
   R5 Morpholinyl oder Hal bedeutet, und
   R4 und Hal die oben angegebene Bedeutung aufweisen,
   mit Formamidinacetat in einem Alkohol unter Erhalt der Verbindungen der Formel (IID-1) 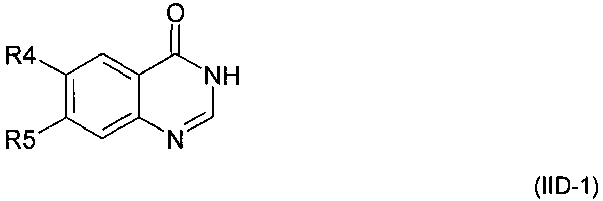 worin
   R5 Morpholinyl oder Hal bedeutet, und
   R4 und Hal die oben angegebene Bedeutung aufweisen,
   und wenn R5 Hal ist
(b) Umsetzen der Verbindungen der Formel (IID-1) mit Morpholin unter Erhalt der Verbindungen der Formel (IID) 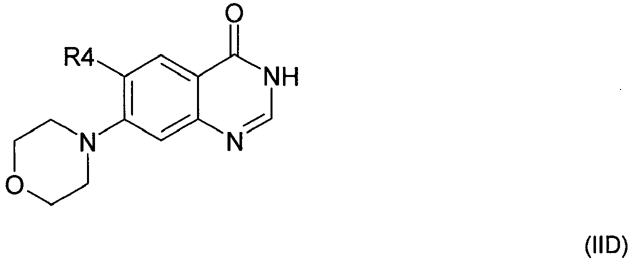
   worin R4 und Hal die oben angegebene Bedeutung aufweisen,
   und Schritten (b') und ggf. (b") wie in Anspruch 8.

Offenbart wird weiterhin ein Verfahren zum Herstellen von Verbindungen unter Formel (IIE) mit den folgenden Schritten:
(a) Reduzieren der Nitrogruppe einer Verbindung der Formel (IIF) 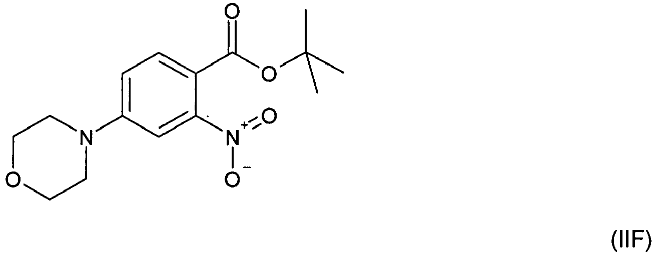 unter Erhalt einer Verbindung der Formel (IIG) 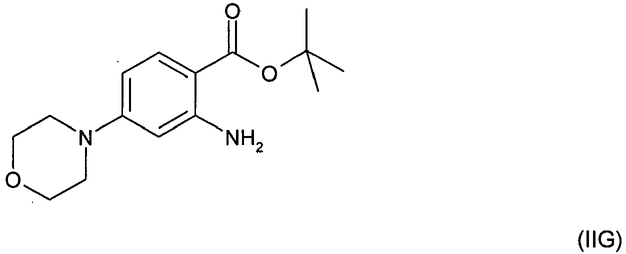 und
(b) Verseifen der Verbindung der Formel (IIG) unter Erhalt der Verbindung der Formel (IIE-1) 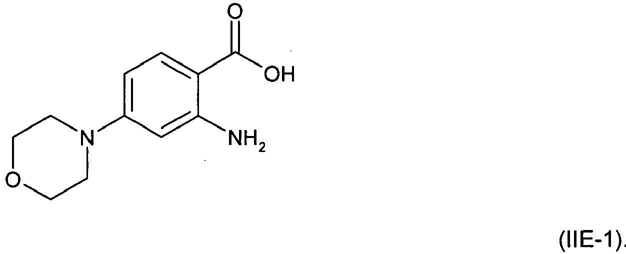 Offenbart wird ferner die Verbindung der Formel (IIG) 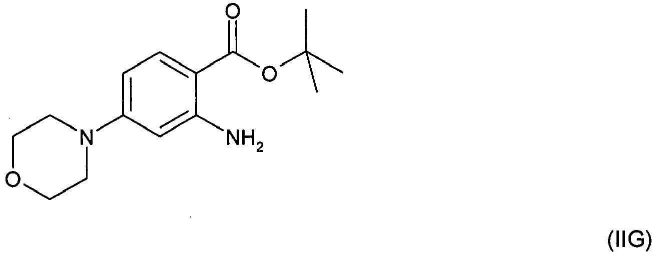 und/oder ihre physiologisch unbedenklichen Salze, Tautomere und/oder Stereoisomere.

Offenbart werden weiterhin Zwischenverbindungen der Formel (III) 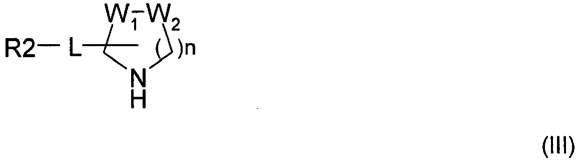 worin R2, R3, Y, W₁, W₂, L, A, Alk, Cyc, Ar, Het¹, Het², Hal und n die oben angegebene Bedeutung aufweisen,
und/oder ihre physiologisch unbedenklichen Salze, Tautomere und/oder Stereoisomere, einschließlich deren Mischungen in allen Verhältnissen.

Zusätzlich offenbart werden Zwischenverbindungen mit der Teilformel (IIIA) 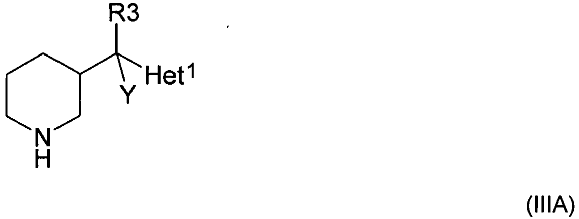 worin
Het¹ unsubstituiertes Heteroaryl aus der Gruppe: 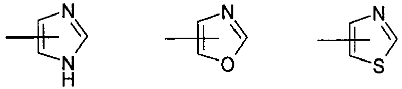 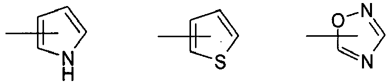 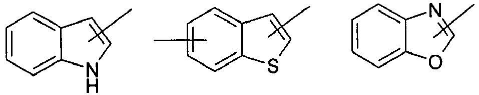 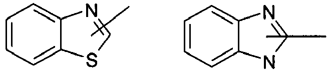 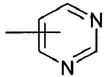 bedeutet, und
   R3, Y, A, Alk und Hal die oben angegebene Bedeutung aufweisen,
   und/oder ihre physiologisch unbedenklichen Salze, Tautomere und/oder Stereoisomere, einschließlich deren Mischungen in allen Verhältnissen.

Offenbart werden auch Zwischenverbindungen mit der Teilformel (IIIB) 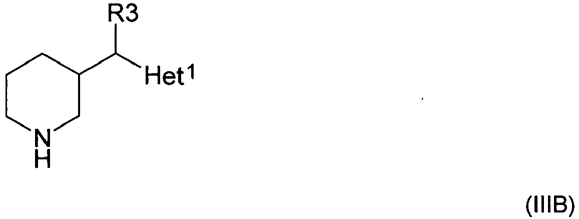 worin
Het¹ unsubstituiertes Heteroaryl aus der Gruppe: 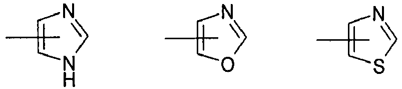 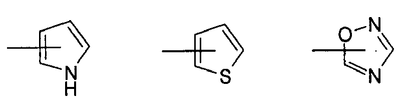 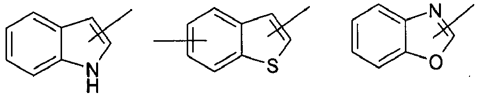 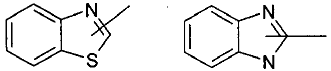 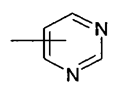 und
   R3 H, OH oder OA bedeuten, und
   A und Hal die oben angegebene Bedeutung aufweisen,
   und/oder ihre physiologisch unbedenklichen Salze, Tautomere und/oder Stereoisomere, einschließlich deren Mischungen in allen Verhältnissen.

Beispielhaft werden Zwischenverbindungen mit den Teilformeln (IIIC), (IIID) und (IIIE) offenbart 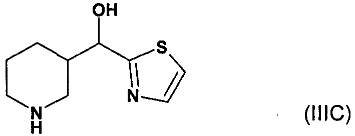 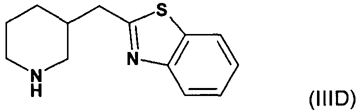 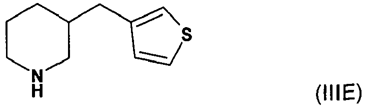 und/oder ihre physiologisch unbedenklichen Salze, Tautomere und/oder Stereoisomere, einschließlich deren Mischungen in allen Verhältnissen.

Offenbart wird darüber hinaus ein Verfahren zum Herstellen von Zwischenverbindungen der Teilformel (IIIB) mit den folgenden Schritten:
(a) Umsetzen einer Verbindung R4-Het¹, worin
   R4 H oder Hal bedeutet, und
   Het¹ und Hal die unter Formel (IIIB) angegebene Bedeutung aufweisen,
   mit einer Verbindung der Formel (IIIF) 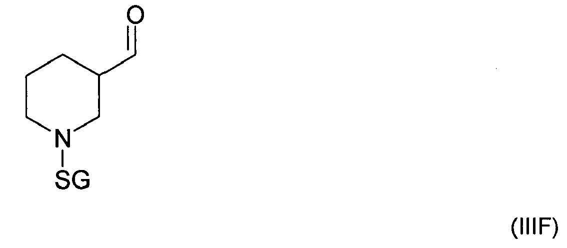 worin SG Schutzgruppe bedeutet,
   unter Erhalt einer Verbindung (IIIG) 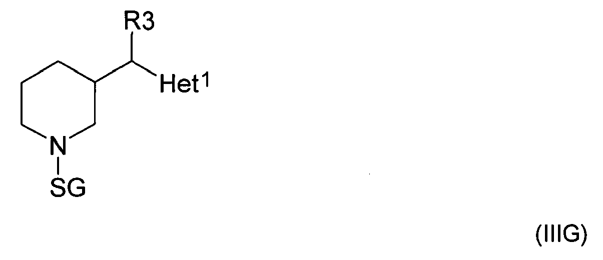 worin
   SG Schutzgruppe bedeutet, und
   Het¹, R3, A und Hal die oben angegebene Bedeutung aufweisen,
   und
(b) Abspalten der Schutzgruppe und optional der R3-Gruppe unter sauren Reaktionsbedingungen unter Erhalt der Zwischenverbindungen der Formel (IIIB) 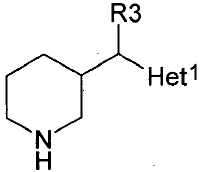 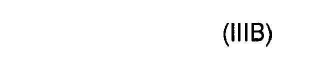 worin Het¹, R3, A und Hal die oben angegebene Bedeutung aufweisen.

Die Ausgangsverbindungen sind in der Regel bekannt. Sind sie neu, so können sie nach an sich bekannten Methoden hergestellt werden. Die Verbindungen der Formeln (IIC) und (IIIF) können nach bekannten Methoden bereitgestellt werden. Fall gewünscht, können die Ausgangsstoffe in-situ gebildet werden, so dass man sie aus dem Reaktionsgemisch nicht isoliert, sondern sofort weiter zu den erfindungsgemäßen Verbindungen umsetzt. Es ist ebenso möglich, die Reaktion schrittweise durchzuführen.

Die genannten erfindungsgemäßen Verbindungen lassen sich in ihrer endgültigen Nichtsalzform verwenden. Andererseits umfasst die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formeln (IA), (II) und (III) sowie ihrer Teilformeln werden größtenteils konventionell hergestellt. Sofern die Verbindungen eine Carbonsäuregruppe enthalten, lässt sich eines ihrer geeigneten Salze dadurch bilden, dass man die Verbindung mit einer geeigneten Base zum entsprechenden Basenadditionssalz umsetzt. Solche Basen sind zum Beispiel Alkalimetallhydroxide (z.B. Kaliumhydroxid, Natriumhydroxid und Lithiumhydroxid), Erdalkalimetallhydroxide (z.B. Bariumhydroxid und Calciumhydroxid), Alkalimetallalkoholate (z.B. Kaliumethanolat und Natriumpropanolat) sowie verschiedene organische Basen wie Piperidin, Diethanolamin und N-Methylglutamin. Eine Base der Formeln (IA), (II) und (III) sowie deren Teilformeln kann mit einer Säure in das zugehörige Säureadditionssalz übergeführt werden, beispielsweise durch Umsetzung äquivalenter Mengen der Base und der Säure in einem inerten Lösungsmittel, wie z.B. Ethanol, und Eindampfen im Anschluss. Für diese Umsetzung kommen insbesondere Säuren in Frage, die physiologisch unbedenkliche Salze liefern, wie z.B. Halogenwasserstoffe (z.B. Chlorwasserstoff, Bromwasserstoff oder Jodwasserstoff), andere Mineralsäuren und ihre entsprechenden Salzen (z.B. Sulfat, Nitrat oder Phosphat und dergleichen), Alkyl- und Monoarylsulfonaten (z.B. Ethansulfonat, Toluolsulfonat und Benzolsulfonat) sowie andere organische Säuren und ihre entsprechenden Salze (z.B. Acetat, Trifluoracetat, Tartrat, Maleat, Succinat, Citrat, Benzoat, Salicylat, Ascorbat und dergleichen. Salze mit physiologisch bedenklichen Säuren, z.B. Pikrate, können zur Isolierung und/oder Aufreinigung der Verbindungen der Formel (IA) verwendet werden.

Im Hinblick auf das oben Gesagte sieht man, dass unter dem Ausdruck "pharmazeutisch unbedenkliches Salz" im vorliegenden Zusammenhang ein Wirkstoff zu verstehen ist, der eine Verbindung der Formel (IA) in der Form eines ihrer Salze enthält, insbesondere dann, wenn diese Salzform dem Wirkstoff im Vergleich zu der freien Form des Wirkstoffs verbesserte pharmakokinetische Eigenschaften verleiht. Die pharmazeutisch unbedenkliche Salzform des Wirkstoffs kann diesem Wirkstoff auch erst eine gewünschte pharmakokinetische Eigenschaft verleihen und kann sogar die Pharmakodynamik dieses Wirkstoffs in Bezug auf seine therapeutische Wirksamkeit im Körper positiv beeinflussen.

Erfindungsgemäße Verbindungen können aufgrund ihrer Molekülstruktur chiral sein und dementsprechend in verschiedenen enantiomeren Formen auftreten. Sie können daher in racemischer oder in optisch aktiver Form vorliegen. Da sich die pharmazeutische Wirksamkeit der Racemate bzw. der Stereoisomeren der Verbindungen der Formel (IA) unterscheiden kann, mag es wünschenswert sein, die Enantiomere zu verwenden. In diesen Fällen kann das Endprodukt oder bereits das Zwischenprodukt in enantiomere Verbindungen, durch dem Fachmann bekannte chemische oder physikalische Maßnahmen, aufgetrennt oder bereits als solche bei der Synthese eingesetzt werden.

Es wurde überraschend gefunden, dass die erfindungsgemäßen Verbindungen eine spezifische Hemmung von en Serin-Threonin-Proteinkinasen bewirken. Ein weiterer Gegenstand der Erfindung betrifft deshalb die Verwendung von Verbindungen der Formel (IA) bzw. Teilformeln davon und/oder ihrer physiologisch unbedenklichen Salze, Tautomere und/oder Stereoisomere, einschließlich deren Mischungen in allen Verhältnissen, zur Hemmung von Serin-Threonin-Proteinkinasen, vorzugsweise PIKK, besonders bevorzugt DNA-PK. Der Begriff "Hemmung" bezieht sich auf jede Verringerung der Aktivität, die auf der Wirkung der spezifischen erfindungsgemäßen Verbindungen basiert, indem letztere in der Lage sind, mit dem Zielmolekül derart zu interagieren, dass eine Erkennung, Bindung und Blockierung ermöglicht wird. Die Verbindungen zeichnen sich durch hohe Affinität zu wenigstens einer Serin-Threonin-Proteinkinasen aus, wodurch eine verlässliche Bindung und vorzugsweise vollständige Blockade der Kinaseaktivität sichergestellt wird. Die Verbindungen sind besonders bevorzugt mono-spezifisch, um eine ausschließliche und unmittelbare Erkennung der ausgewählten Kinase zu garantieren. Der Begriff der "Erkennung" bezieht sich hierbei auf jede Art der Wechselwirkung zwischen der Verbindung und den genannten Zielmolekülen, insbesondere kovalente oder nicht-kovalente Bindungen, wie z.B. eine kovalente Bindung, hydrophobe/ hydrophile Wechselwirkungen, van der Waals'sche Kräfte, lonenbeziehung, Wasserstoffbrücken, Ligand-Rezeptor-Wechselwirkungen, Basenpaarungen von Nukleotiden oder Wechselwirkungen zwischen Epitop und Antikörper-Bindungsstelle.

Die erfindungsgemäßen Verbindungen zeigen eine vorteilhafte biologische Aktivität, die in den hierin beschriebenen Tests nachweisbar ist, wie z.B. auf Enzymen basierenden Assays. Die Messung der Kinaseaktivität ist eine dem Fachmann wohlbekannte Technik. Generische Testsysteme zur Bestimmung der Kinaseaktivität mit Substraten, z.B. Histon (Alessi et al. (1996) FEBS Lett. 399(3): 333) oder dem basischen Myelinprotein sind in der Literatur beschrieben (Campos-González & Glenney (1992) JBC 267: 14535). Zur Identifikation von Kinase-Inhibitoren stehen verschiedene Assay-Systeme zur Verfügung. Beim Scintillation Proximity Assay (Sorg et al. (2002) J Biomolecular Screening 7: 11) und dem FlashPlate Assay werden die radioaktive Phosphorylierung eines Proteins oder Peptids als Substrat mit ATP gemessen. Bei Vorliegen einer inhibitorischen Verbindung ist kein oder ein vermindertes radioaktives Signal nachweisbar. Ferner sind die Homogeneous Timeresolved Fluorescence Resonance Energy Transfer- (HTR-FRET-) und Fluoreszenzpolarisations- (FP-) Technologien als Assay-Verfahren nützlich (Sills et al. (2002) J Biomolecular Screening 191). Andere nicht-radioaktive ELISA-Verfahren verwenden spezifische Phospho-Antikörper (Phospho-AK). Der Phospho-AK bindet nur das phosphorylierte Substrat. Diese Bindung ist mit einem zweiten Peroxidase-konjugierten Anti-Schaf-Antikörper durch Chemilumineszens nachweisbar.

Die vorgenannte Verwendung der Verbindungen kann in in-vitro oder in-vivo Modellen geschehen. Die Suszeptibilität einer bestimmten Zelle gegenüber der Behandlung mit den erfindungsgemäßen Verbindungen kann durch Testen in-vitro bestimmt werden. Typischerweise wird eine Kultur der Zelle mit einer erfindungsgemäßen Verbindung bei verschiedenen Konzentrationen für eine Zeitdauer inkubiert, die ausreicht, um den aktiven Mitteln zu ermöglichen, Zelltod zu induzieren oder Zellproliferation, Zellvitalität oder Migration zu inhibieren, gewöhnlich zwischen ungefähr einer Stunde und einer Woche. Zum Testen in-vitro können kultivierte Zellen aus einer Biopsieprobe verwendet werden. Die Menge der nach der Behandlung zurückbleibenden Zellen wird dann bestimmt. Die Verwendung in-vitro erfolgt insbesondere an Proben von Säugerspezies, die an Krebs, Tumoren, Metastasen, Störungen der Angiogenese, retroviralen Erkrankungen, Immunerkrankungen und/oder pathogenen Alterungsprozessen leiden. Der Wirt oder Patient kann jeglicher Säugerspezies angehören, z. B. einer Primatenspezies, insbesondere Menschen, aber auch Nagetieren (einschließlich Mäusen, Ratten und Hamstern), Kaninchen, Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Das Testen mehrerer spezifischer Verbindungen ermöglicht die Auswahl des Wirkstoffs, der am besten für die Behandlung des Patienten geeignet erscheint. Die in-vivo Dosis der gewählten Verbindung wird vorteilhaft auf die Suszeptibilität der Kinase und/oder Schwere der Erkrankung des Patienten mit Blick auf die in-vitro Daten abgestimmt, wodurch die therapeutische Wirksamkeit merklich erhöht ist. Die Dosis variiert abhängig von der verwendeten spezifischen Verbindung, der spezifischen Erkrankung, dem Patientenstatus usw. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die nachfolgende Lehre der Erfindung und deren Ausführungsformen betreffend die Verwendung von Verbindungen der Formel (IA) für die Herstellung eines Arzneimittels zur Prophylaxe, Therapie und/oder Verlaufskontrolle ist gültig und ohne Einschränkungen auf die Verwendung der Verbindungen zur Hemmung der Kinaseaktivität anwendbar, sofern es sinnvoll erscheint.

Die Behandlung wird im Allgemeinen fortgesetzt, bis eine erhebliche Reduktion vorliegt, z.B. mindestens ca. 50 % Verminderung der Zelllast und kann fortgesetzt werden, bis im Wesentlichen keine unerwünschten Zellen mehr im Körper nachgewiesen werden. In derartigen Tests zeigen und bewirken die erfindungsgemäßen Verbindungen einen inhibierenden Effekt, der gewöhnlich durch IC₅₀-Werte in einem geeigneten Bereich, bevorzugt im mikromolaren Bereich und bevorzugter im nanomolaren Bereich dokumentiert wird. Die Kinase wird insbesondere zu 50 % gehemmt, wenn die Konzentration der Verbindungen kleiner als 1 µM ist, vorzugsweise kleiner als 0,5 µM, besonders bevorzugt kleiner als 0,1 µM. Diese Konzentration wird als IC₅₀-Wert bezeichnet.

Gegenstand der Erfindung ist auch ein Arzneimittel umfassend mindestens eine Verbindung der Formel (IA) bzw. Teilformeln davon und/oder ihre physiologisch unbedenklichen Salze, Tautomere und/oder Stereoisomere, einschließlich deren Mischungen in allen Verhältnissen. Noch ein Gegenstand der Erfindung ist eine pharmazeutische Zusammensetzung umfassend als Wirkstoff eine wirksame Menge von mindestens einer Verbindung der Formel (IA) bzw. Teilformeln davon und/oder ihrer physiologisch unbedenklichen Salze, Tautomere und/oder Stereoisomere, einschließlich deren Mischungen in allen Verhältnissen, zusammen mit pharmazeutisch verträglichen Hilfsstoffen.

Ein "Arzneimittel", "Medikament" sowie eine "pharmazeutische Zusammensetzung" oder "pharmazeutische Formulierung" ist hierbei jedes Mittel, welches in der Prophylaxe, Therapie, Verlaufskontrolle oder Nachbehandlung von Patienten eingesetzt werden kann, die zumindest zeitweise eine pathogene Modifikation des Gesamtzustandes bzw. des Zustandes einzelner Teile des Patientenorganismus zeigen, vorzugsweise infolge von Krebs, Tumoren, Metastasen, Störungen der Angiogenese, retroviralen Erkrankungen, Immunerkrankungen und/oder beschleunigten Alterungsprozessen, besonders bevorzugt infolge von Krebs, Tumoren, Metastasen und/oder Störungen der Angiogenese.

Um die protektive oder therapeutische Wirkung der erfindungsgemäßen Verbindungen zu erhöhen, können pharmazeutisch verträgliche Adjuvantien zugesetzt werden. Im Sinne der Erfindung ist jede Substanz, die mit den Verbindungen gemäß der Erfindung eine Wirkung ermöglicht, verstärkt oder modifiziert, ein "Adjuvans". Bekannte Adjuvantien sind z.B. Aluminiumverbindungen, wie z.B. Aluminiumhydroxid oder Aluminiumphosphat, Saponine, wie z.B. QS 21, Muramyldipeptid oder Muramyltripeptid, Proteine, wie z.B. Gammainterferon oder TNF, MF 59, Phosphatdibylcholin, Squalen oder Polyole. Ebenfalls kann die Co-Applikation von Ei-Albumin in komplettem Freund'schen Adjuvans eine gesteigerte zellvermittelte Immunität hervorrufen und somit die Wirkung gebildeter neutralisierender Antikörper unterstützen. Des Weiteren kann DNA, die eine immunstimulatorische Eigenschaft hat, oder die ein Protein mit Adjuvanseffekt kodiert, wie z.B. ein Cytokin, parallel oder in einem Konstrukt appliziert werden.

Das Einbringen des pharmazeutischen Mittels in eine Zelle respektive einen Organismus kann erfindungsgemäß auf jede Art und Weise geschehen, die es ermöglicht, dass die Kinasen mit den in der Zusammensetzung enthaltenen Verbindungen in Kontakt gebracht werden, infolgedessen eine Antwort induziert wird. Das pharmazeutische Mittel der vorliegenden Erfindung kann oral, transdermal, transmucosal, transurethal, vaginal, rektal, pulmonal, enteral und/oder parenteral angewendet werden. Die gewählte Art der Verabreichung richtet sich nach der Indikation, der zu verabreichenden Dosis, Individuumsspezifischen Parametern etc. Insbesondere ermöglichen die verschiedenen Arten der Verabreichung eine ortsspezifische Therapie, die Nebenwirkungen minimiert und die Wirkstoffdosis verringert. Ganz besonders bevorzugte Injektionen sind die intradermale, subkutane, intramuskuläre oder intravenöse Injektion. Die Applikation kann z.B. mit Hilfe so genannter Impfpistolen oder mittels Spritzen geschehen. Es ist auch möglich, die Substanz als Aerosol bereitzustellen, welches von dem Organismus, bevorzugt einem humanen Patienten, inhaliert wird.

Die Darreichungsformen des pharmazeutischen Mittels werden mit den üblichen festen oder flüssigen Trägerstoffen und/oder Verdünnungsmitteln und den üblicherweise eingesetzten Hilfsstoffen entsprechend der gewünschten Applikationsart in einer geeigneten Dosierung und in an sich bekannter Weise hergestellt. Grundsätzlich können also pharmazeutisch annehmbare und dem Fachkundigen bekannte Exzipienten einen Teil des erfindungsgemäßen pharmazeutischen Mittels bilden, wobei die Menge des Exzipientenmaterials, die mit dem Wirkstoff kombiniert wird, um eine Einzeldosierung herzustellen, in Abhängigkeit vom zu behandelnden Individuum und der Art der Verabreichung variiert. Diese pharmazeutisch verträglichen Zusätze umfassen Salze, Puffer, Füllstoffe, Stabilisatoren, Komplexbildner, Antioxidantien, Lösungsmittel, Bindemittel, Gleitmittel, Tablettenüberzüge, Geschmacksstoffe, Farbstoffe, Konservierungsmittel, Stellmittel und dergleichen. Beispiele für solche Exzipienten sind Wasser, pflanzliche Öle, Benzylalkohole, Alkylenglycol, Polyethylenglycol, Glycerintriacetat, Gelatine, Kohlenhydrate, wie z.B. Laktose oder Stärke, Magnesiumstearat, Talk und Vaseline.

Die pharmazeutische Formulierung kann als Tablette, Filmtablette, Dragee, Lutschtablette, Kapsel, Pille, Pulver, Granulat, Sirup, Saft, Tropfen, Lösung, Dispersion, Suspension, Suppositor, Emulsion, Implantat, Creme, Gel, Salbe, Paste, Lotion, Serum, Öl, Spray, Aerosol, Kleber, Pflaster oder Verband vorliegen. Als orale Darreichungsform werden vorzugsweise Tabletten, Filmtabletten, Dragees, Lutschtabletten, Kapseln, Pillen, Pulver, Granulate, Sirupe, Säfte, Tropfen, Lösungen, Dispersionen oder Suspensionen - auch als Depotform - zubereitet. Des Weiteren sind parenterale Arzneiformen, wie z.B. Suppositorien, Suspensionen, Emulsionen, Implantate oder Lösungen, in Betracht zu ziehen, vorzugsweise ölige oder wässrige Lösungen. Zur topischen Applikation wird der Arzneimittelwirkstoff mit mindestens einem pharmazeutisch annehmbaren Trägerstoff, wie z.B. mikrokristalliner Cellulose, und ggf. weiteren Hilfsstoffen, wie z.B. Feuchtigkeitsspendern, zu auf die Haut auftragbaren festen Formulierungen, wie z.B. Cremes, Gelen, Salben, Pasten, Pulver oder Emulsionen bzw. zu auf die Haut auftragbaren flüssigen Formulierungen, wie z.B. Lösungen, Suspensionen, Lotionen, Seren, Ölen, Sprays oder Aerosole in üblicher Weise formuliert. Vorzugsweise liegt das pharmazeutische Mittel als Injektionslösung vor. Für die Herstellung der Injektionslösung können wässrige Medien, wie z.B. destilliertes Wasser oder physiologische Salzlösungen verwendet werden, wobei letztere saure und basische Additionssalze einschließen. Das pharmazeutische Mittel kann auch als feste Zusammensetzung, beispielsweise im lyophilisierten Zustand vorliegen, und dann durch Zugabe eines auflösenden Mittels, wie z.B. destilliertes Wasser, vor der Verwendung bereitet werden. Die Grundlagen der Herstellung von Lyophilisaten sind dem Fachmann vertraut.

Die Konzentration der aktiven Verbindung in der Formulierung kann 0,1 bis 100 Gewichtsprozente betragen. Entscheidend ist, dass die pharmazeutische Zusammensetzung als Wirkstoff eine effektive Menge der Verbindung zusammen mit den pharmazeutisch verträglichen Hilfsstoffen umfasst. Die Begriffe "effektive Menge" oder "wirksame Dosis" werden hierin austauschbar verwendet und bezeichnen eine Menge des pharmazeutischen Wirkstoffs, die eine prophylaktisch oder therapeutisch relevante Wirkung auf eine Krankheit oder pathologische Veränderung in Zelle, Gewebe, Organ oder Säuger hat. Eine "prophylaktische Wirkung" verhindert das Ausbrechen einer Krankheit oder sogar die Infektion mit einem Pathogen nach dem Eindringen einzelner Vertreter derart, dass deren nachfolgende Ausbreitung stark verringert wird oder sie sogar vollständig inaktiviert werden. Eine "prophylaktische Wirkung" umfasst auch die Erhöhung der normalen physiologischen Funktion. Eine Prophylaxe ist insbesondere ratsam, wenn ein Individuum Veranlagungen für den Ausbruch der vorgenannten Krankheiten aufweist, wie z. B. eine familiäre Vorbelastung, einen Gendefekt oder eine kürzlich überstandene Krankheit. Eine "therapeutisch relevante Wirkung" befreit teilweise oder vollständig von einem, mehreren oder allen Krankheitssymptomen oder führt zur teilweisen oder vollständigen Rückkehr eines, mehrerer oder aller physiologischer oder biochemischer Parameter, die mit der Krankheit oder pathologischen Veränderung in Zusammenhang stehen oder ursächlich dafür sind, in den Normalzustand. Auch wird die Verlaufskontrolle als Art der therapeutischen Behandlung verstanden, wenn die Verbindungen in bestimmten Intervallen verabreicht werden, z.B. um die Symptome einer Krankheit vollständig zu beseitigen. Die jeweilige Dosis bzw. der Dosisbereich für die Gabe der erfindungsgemäßen Verbindungen ist groß genug, um den gewünschten prophylaktischen oder therapeutischen Effekt der Induktion einer biologischen oder medizinischen Antwort zu erreichen. Im Allgemeinen wird die Dosis mit dem Alter, der Konstitution und dem Geschlecht des Patienten variieren sowie die Schwere der Erkrankung berücksichtigen. Es versteht sich, dass die spezifische Dosis, Häufigkeit und Dauer der Verabreichung darüber hinaus von einer Vielzahl an Faktoren abhängen, wie z.B. der Zielsteuerungs- und Bindungsfähigkeit der Verbindungen, Ernährungsgewohnheiten des zu behandelnden Individuums, Art der Verabreichung, Ausscheidungsrate und Kombination mit anderen Medikamenten. Die individuelle Dosis kann sowohl in Bezug auf die primäre Erkrankung als auch in Bezug auf das Eintreten eventueller Komplikationen eingestellt werden. Die exakte Dosis ist durch einen Fachmann mit bekannten Mitteln und Methoden feststellbar. Diese Lehre der Erfindung ist gültig und ohne Einschränkungen auf die pharmazeutische Zusammensetzung umfassend die Verbindungen der Formel (IA) anwendbar, sofern es sinnvoll erscheint.

In einer Ausführungsform der Erfindung werden die Verbindungen in einer Dosis von 0,01 mg bis 1 g pro Dosierungseinheit verabreicht, vorzugsweise zwischen 1 bis 700 mg, besonders bevorzugt 5 bis 100 mg. Die tägliche Dosierung liegt insbesondere zwischen 0,02 und 100 mg/kg Körpergewicht.

Zur Unterstützung der medizinischen Wirkung kann die pharmazeutische Zusammensetzung in einer Ausgestaltung der Erfindung auch einen oder mehrere weitere Wirkstoffe umfassen, wobei eine gleichzeitige oder aufeinander folgende Verabreichung denkbar ist. Die therapeutische Wirkung der erfindungsgemäßen pharmazeutischen Zusammensetzung kann beispielsweise darin bestehen, dass durch die Hemmung der DNA-PK als erwünschter Nebeneffekt bestimmte Antikrebsmittel besser wirken oder durch die Verminderung der Dosis die Anzahl der Nebenwirkungen dieser Medikamente reduziert wird.

In einer bevorzugten Ausführungsform der Erfindung wird die erfindungsgemäße pharmazeutische Zusammensetzung mit einem Antikrebsmittel kombiniert. Wie hier verwendet, betrifft der Begriff "Antikrebsmittel" jedes Mittel, das einem Patienten mit Krebs, Tumoren, Metastasen und/oder Störungen der Angiogenese zum Zweck der Behandlung des Krebses verabreicht wird. Das Antikrebsmittel ist besonders bevorzugt ausgewählt aus der Gruppe umfassend Cytokine, Chemokine, pro-apoptotische Mittel, Interferone, radioaktive Verbindungen, Östrogenrezeptor-Modulatoren, Androgenrezeptor-Modulatoren, Retinoidrezeptor-Modulatoren, Zytotoxika, Zytostatika, Prenyl-Proteintransferase-Hemmer und Angiogenese-Hemmer oder Kombinationen davon. Es ist bevorzugt, dass das Antikrebsmittel den Nukleinsäure- und/oder Proteinstoffwechsel, die Zellteilung, DNA-Replikation, Purin-, Pyrimidin- und/oder Aminosäure-Biosynthese, Genexpression, mRNA-Prozessierung, Proteinsynthese, Apoptose oder Kombinationen davon verändert, insbesondere abschwächt.

Die Erfindung kann auch als Kit praktiziert werden, der die erfindungsgemäßen Verbindungen enthält. Das Kit besteht aus getrennten Packungen von (a) einer wirksamen Menge einer Verbindung der Formel (IA) und/oder ihrer physiologisch unbedenklichen Salze, Tautomere und/oder Stereoisomere, einschließlich deren Mischungen in allen Verhältnissen, und (b) einer wirksamen Menge eines weiteren Wirkstoffs. Das Kit enthält geeignete Behälter, wie z.B. Schachteln oder Kartons, individuelle Flaschen, Beutel oder Ampullen. Das Kit kann z.B. separate Ampullen enthalten, in denen jeweils eine wirksame Menge an einer Verbindung der Formel (IA) und/oder ihrer pharmazeutisch verwendbaren Salze, Tautomere und/oder Stereoisomere, einschließlich deren Mischungen in allen Verhältnissen, und einer wirksamen Menge eines weiteren Arzneimittelwirkstoffs gelöst oder in lyophilisierter Form vorliegt. Das Kit der Erfindung kann auch einen Artikel beinhalten, der schriftliche Anweisungen enthält oder den Anwender auf schriftliche Anweisungen hinweist, die den Umgang mit den Verbindungen der Erfindung erläutern.

Erfindungsgemäß werden die Verbindungen der Formel (IA) bzw. deren Teilformeln und/oder ihrer physiologisch unbedenklichen Salze, Tautomere und/oder Stereoisomere, einschließlich deren Mischungen in allen Verhältnissen, zur Prophylaxe, Therapie und/oder Verlaufskontrolle von Erkrankungen verwendet, die durch die Aktivität von Serin-Threonin-Proteinkinasen hervorgerufen, vermittelt und/oder verbreitet werden. Gegenstand der vorliegenden Erfindung ist deshalb auch die Verwendung von Verbindungen der Formel (IA) bzw. deren Teilformeln und/oder ihrer physiologisch unbedenklichen Salze, Tautomere und/oder Stereoisomere, einschließlich deren Mischungen in allen Verhältnissen, zur Herstellung eines Arzneimittels zur Prophylaxe, Therapie und/oder Verlaufskontrolle von Erkrankungen, die durch die Aktivität von Serin-Threonin-Proteinkinasen hervorgerufen, vermittelt und/oder verbreitet werden. Erfindungsgemäß sind Verbindungen der Formel (IA) bzw. deren Teilformeln und/oder ihre physiologisch unbedenklichen Salze, Tautomere und/oder Stereoisomere, einschließlich deren Mischungen in allen Verhältnissen, zur Verwendung in der Prophylaxe, Therapie und/oder Verlaufskontrolle von Erkrankungen geeignet, die durch Aktivität von Serin-Threonin-Proteinkinasen hervorgerufen, vermittelt und/oder verbreitet werden. Zur Identifizierung eines entsprechenden Signalübertragungswegs und zum Nachweis von Wechselwirkungen zwischen verschiedenen Signalübertragungswegen sind geeignete Modelle oder Modellsysteme entwickelt worden, z.B. Zellkulturmodelle (Khwaja et al. (1997) EMBO 16: 2783) und Modelle transgener Tiere (White et al. (2001) Oncogene 20: 7064). Zur Bestimmung bestimmter Stufen in der Signalübertragungskaskade können wechselwirkende Verbindungen genutzt werden, um das Signal zu modulieren (Stephens et al. (2000) Biochemical J 351: 95). Darüber hinaus können die erfindungsgemäßen Verbindungen auch als Reagenzien zur Testung kinaseabhängiger Signalübertragungswege in Tieren und/oder Zellkulturmodellen oder in den in dieser Anmeldung genannten klinischen Erkrankungen verwendet werden. Wie hierin besprochen, sind diese Signalwege für verschiedene Erkrankungen relevant. Dementsprechend sind die erfindungsgemäßen Verbindungen nützlich bei der Prophylaxe, Therapie und/oder Verlaufskontrolle von Erkrankungen, die von Signalwegen mit Beteiligung von Serin-Threonin-Proteinkinasen abhängig sind.

Erfindungsgemäß sind die Verbindungen der Formel (IA) bzw. deren Teilformeln und/oder ihre physiologisch unbedenklichen Salze, Tautomere und/oder Stereoisomere, einschließlich deren Mischungen in allen Verhältnissen, zur Verwendung in der Prophylaxe, Therapie und/oder Verlaufskontrolle von Krebs, Tumoren, Metastasen, Störungen der Angiogenese, retroviralen Erkrankungen und/oder Immunerkrankungen geeignet, insbesondere Krebs, Tumoren, Metastasen und/oder Störungen der Angiogenese. Erfindungsgemäß sind die Verbindungen der Formel (IA) bzw. deren Teilformeln und/oder ihre physiologisch unbedenklichen Salze, Tautomere und/oder Stereoisomere, einschließlich deren Mischungen in allen Verhältnissen, auch zur Verwendung bei der Verlangsamung von Alterungsprozessen geeignet, wobei die Verlangsamung anhand des Vergleichs der Lebenspanne des behandelten Wirts oder dessen Zellen, Zellkulturen, Geweben oder Organen mit entsprechenden Positiv- oder Negativkontrollen und/oder Statistiken erfolgt. Es versteht sich, dass auch der Wirt der pharmazeutischen Verbindungen in den Schutzumfang der vorliegenden Erfindung eingeschlossen ist.

Der Tumor ist insbesondere ausgewählt aus der Gruppe von Erkrankungen von Plattenepithel, Blase, Magen, Nieren, Kopf, Hals, Ösophagus, Gebärmutterhals, Schilddrüse, Darm, Leber, Gehirn, Prostata, Urogenitaltrakts, lymphatisches System, Kehlkopf, Lunge, Haut, Blut und Immunsystem, und/oder der Krebs ist ausgewählt aus der Gruppe von Monozytenleukämie, Lungenadenokarzinom, kleinzelliges Lungenkarzinom, Bauchspeicheldrüsenkrebs, Glioblastom, Darmkarzinom, Brustkarzinom, akute myelotische Leukämie, chronische myelotische Leukämie, akute lymphatische Leukämie, chronische lymphatische Leukämie, Hodgkin-Lymphom und non-Hodgkin-Lymphom.

Eine weitere Ausgestaltung der vorliegenden Erfindung betrifft die erfindungsgemäßen Verbindungen in Kombination mit Radiotherapie und/oder mit mindestens einem weiteren Wirkstoff, vorzugsweise in Kombination mit Radiotherapie und/oder einem Antikrebsmittel. Die erfindungsgemäßen Verbindungen erzielen bei existierenden Krebs-Chemotherapien und Bestrahlungen synergistische Effekte und/oder stellen die Wirksamkeit existierender Krebs-Chemotherapien und Bestrahlungen wieder her. Die synergistische Wirkung der Hemmung von VEGF in Kombination mit Radiotherapie ist im Stand der Technik beschrieben (WO 00/61186). Als weitere Arzneimittelwirkstoffe sind besonders solche Chemotherapeutika bevorzugt, die die Angiogenese hemmen und dadurch das Wachstum und die Verbreitung von Tumorzellen inhibieren. Beispiele hierfür sind VEGF-Rezeptorinhibitoren, beinhaltend Ribozyme und Antisense, die auf VEGF-Rezeptoren gerichtet sind, sowie Angiostatin und Endostatin. Weitere Beispiele antineoplastischer Agenzien, die in Kombination mit den erfindungsgemäßen Verbindungen verwendet werden können, beinhalten im Allgemeinen alkylierende Agenzien, Antimetaboliten, Epidophyllotoxin, ein antineoplastisches Enzym, einen Topoisomerase-Inhibitor, Procarbazin, Mitoxantron oder Platin-Koordinationskomplexe. In einer anderen Ausführungsform ist das Antikrebsmittel besonders bevorzugt ausgewählt aus der Gruppe von Östrogenrezeptor-Modulator, Androgenrezeptor-Modulator, Retinoidrezeptor-Modulator, Zytotoxikum, Zytostatikum, Prenyl-Proteintransferase-Hemmer und Angiogenese-Hemmer. Im Übrigen ist die vorherige Lehre der Erfindung und deren Ausführungsformen betreffend pharmazeutische Zusammensetzung gültig und ohne Einschränkungen auf die zweite medizinische Indikation anwendbar, sofern es sinnvoll erscheint. Eine ganz besonders bevorzugte Ausführungsform umfasst die erfindungsgemäßen Verbindungen in Kombination mit Radiotherapie und/oder einem Zytostatikum.

Noch eine weitere Ausgestaltung der Erfindung bezieht sich auf die Verwendung von mindestens einer Verbindung der Formel (IA) und/oder ihrer physiologisch unbedenklichen Salze, Tautomere und/oder Stereoisomere, einschließlich deren Mischungen in allen Verhältnissen, zur Sensibilisierung von Krebszellen gegenüber Antikrebsmittel und/oder ionisierender Strahlung unter der Maßgabe, dass die Sensibilisierung in-vivo nicht am menschlichen Körper erfolgt. Die Sensibilisierung erfolgt vorzugsweise ex-vivo oder in-vitro, indem die Verbindungen Zellen, Zellkulturen, Geweben oder Organen verabreicht werden, die Serin-Threonin-Proteinkinasen umfassen. Die ex-vivo-Verwendung wird insbesondere bei tierischen Zellen angewandt, die aus einem tierischen Organismus stammen, der von einer Krankheit betroffen ist, die ausgewählt ist aus der Gruppe von Krebs, Tumoren, Metastasen und/oder Störungen der Angiogenese. Die ex-vivo-behandelten Zellen können entweder für Folgeuntersuchungen weiter in Kultur gehalten oder in ein Tier überführt werden, wobei es sich um das Wirtstier oder ein anderes Tier handeln kann. Die erfindungsgemäße ex-vivo-Sensibilisierung ist insbesondere von Vorteil, um den spezifische Wirkung der Verbindungen zu testen, so dass unter Auswertung dieser ex-vivo-Daten die in-vivo-Dosis entsprechend voreingestellt werden kann. Im Ergebnis dessen wird der therapeutische Effekt signifikant erhöht.

Die Erfindung lehrt ferner ein Verfahren zur Prophylaxe, Therapie und/oder Verlaufskontrolle von Krebs, Tumoren, Metastasen, Störungen der Angiogenese, retroviralen Erkrankungen, Immunerkrankungen und/oder Alterungsprozessen, wobei eine wirksame Menge mindestens einer erfindungsgemäßen Verbindung und/oder ihrer physiologisch unbedenklichen Salze, Tautomere und/oder Stereoisomere, einschließlich deren Mischungen in allen Verhältnissen, einem zu behandelnden Probanden verabreicht wird. Bevorzugte Probanden im Sinne der Erfindung sind Mensch oder Tier, besonders bevorzugt der Mensch. Dem Fachmann ist hierbei bekannt, dass er die erfindungsgemäßen Verbindungen, die selbstverständlich auch als das erfindungsgemäße pharmazeutische Mittel verwendet werden können, in verschiedenen Dosierungen einem Organismus, insbesondere einem humanen Patienten, applizieren kann. Die wirksame Menge und die Art der Applikation können vom Fachmann durch Routineversuche bestimmt werden. Die vorherige Lehre der Erfindung und deren Ausführungsformen sind gültig und ohne Einschränkungen auf das Behandlungsverfahren anwendbar, sofern es sinnvoll erscheint.

Sämtliche genannte sowie weitere Bestandteile bzw. Komponenten sind dem Fachmann geläufig und können in Routineversuchen eine spezielle Ausgestaltung für die erfindungsgemäße Lehre erfahren. Sämtliche in der Beschreibung zitierten Dokumente sollen hiermit in ihrer Gesamtheit in die Offenbarung der vorliegenden Erfindung als Referenz einbezogen werden.

Im Rahmen der hier vorgestellten Erfindung wurden erstmals neuartige Morpholinylchinazolin-Verbindungen der Formel (IA) bereitgestellt. Die erfindungsgemäßen Verbindungen steuern affin und/oder selektiv Serin-Threonin-Proteinkinasen, insbesondere DNA-PK, an. Die Verbindungen aus Formel (IA) und deren Derivate zeichnen sich durch eine hohe Spezifität und Stabilität, niedrige Herstellungskosten und leichte Handhabung aus. Auf diesen Eigenschaften basieren eine reproduzierbaren Wirkungsweise, einschließlich des Fehlens von Kreuzreaktivitäten, und die verlässliche und sichere Wechselwirkung mit den entsprechenden Zielstrukturen. Die Erfindung beinhaltet auch die Verwendung der vorliegenden Morpholinylchinazolin-Derivate zur Hemmung, Regulation und/oder Modulation der Signalkaskade von Serin-Threonin-Proteinkinasen, insbesondere DNA-PK, und bietet damit neuartige Werkzeuge für Forschung und/oder Diagnostik.

Arzneimittel und pharmazeutische Zusammensetzungen, die die genannten Verbindungen enthalten, und der Einsatz dieser Verbindungen zur Behandlung Kinase-vermittelter Störungen sind außerdem ein vielversprechenden Ansatz für ein breites Spektrum von Therapien, wodurch sich bei Mensch und Tier eine direkte und unmittelbare Linderung von Symptomen erreichen lässt. Dies ist besonders für die wirksame Bekämpfung schwerer Krankheiten wie Krebs von Vorteil, entweder als Monotherapie oder in Kombination mit anderen anti-neoplastischen Therapien. Die Schlüsselbeteiligung von DNA-PK an DNA-Reparaturprozessen und der Nachweis, dass der DNA-PK-Inhibitoren Säugerzellen strahlungsempfindlicher werden lässt, ermöglicht eine therapeutische Anwendung der DNA-PK oder DNA-PK/ATM oder ATM spezifischen Inhibitoren im Rahmen der Behandlung von z.B. soliden Krebstumoren durch Bestrahlungs- und/oder einer auf DNA-DSBs abzielenden Chemotherapie. Die Verbindungen der Formel (IA), ihre Salze, Isomere, Tautomere, Enantiomere, Diastereomere, Racemate, Derivate, Prodrugs und/oder Metaboliten sind nicht nur bei den genannten klinischen Krankheitsbildern wirkungsvoll, sondern ebenso in der Diagnose und Therapie sämtlicher Erkrankungen in Zusammenhang mit der DNA-PK-Signalkaskade, vor allem im Hinblick auf die Hemmung von Zellproliferation und -migration. Darüber hinaus sind die erfindungsgemäßen Inhibitoren in der Behandlung von retroviralen Erkrankungen durch ein Zurückdrängen der retroviralen Integration nutzbar (R. Daniel (1999) Science 284: 644). Schließlich können die erfindungsgemäßen Hemmstoffe als Immunmodulatoren sowie Modulatoren der telomeren Wartung eingesetzt werden. Die niedermolekularen Inhibitoren werden einzeln und/oder in Kombination mit anderen Behandlungsmaßnahmen verwendet, wie z.B. chirurgischen Eingriffen, Immun-, Strahlen- und/oder Chemotherapie. Letztere beziehen sich auf eine zielgerichtete Therapie mit einem beliebigen NME (d.h. NCE und/oder NBE) als Mono- und/oder On-Target-/Off-Target-Kombinationstherapie.

Aufgrund ihrer überraschend starken und/oder selektiven Hemmung von solchen Enzymen, die über die Reparatur von dsDNA zelluläre Prozesse regeln, können die Verbindungen der Erfindung bei vorteilhaft niedriger Dosierung verabreicht werden, während sie im Vergleich mit den weniger potenten bzw. weniger selektiven Inhibitoren des Stands der Technik eine ähnliche oder sogar überlegene biologische Wirksamkeit erzielen. Die reduzierte Dosis geht auch mit verringerten oder keinen medizinischen Nebenwirkungen einher. Darüber hinaus wird die hoch selektive Hemmung durch die erfindungsgemäßen Verbindungen auch durch eine Verringerung unerwünschter Nebenwirkungen reflektiert, die unabhängig von der Dosierung ist.

Es versteht sich, dass diese Erfindung nicht auf die spezifischen Verbindungen, pharmazeutischen Zusammensetzungen, Verwendungen und Verfahren beschränkt ist, wie sie hierin beschrieben sind, da solche Dinge variieren können. Es versteht sich des Weiteren, dass die vorliegend verwendete Terminologie ausschließlich dem Zweck der Beschreibung besonderer Ausführungsformen dient und nicht den Schutzumfang der Erfindung einschränken soll. Wie vorliegend in der Spezifikation einschließlich der anhängigen Ansprüche verwendet, schließen Wortformen im Singular, wie z. B. "ein", "eine", "einer", "der" oder "das" die Entsprechung im Plural ein, sofern der Kontext nicht eindeutig etwas anderes vorgibt. Beispielsweise enthält der Bezug auf "eine Verbindung" ein einzelne Verbindung oder mehrere Verbindungen, die wiederum identisch oder verschieden sein können, oder der Bezug auf "ein Verfahren" schließt äquivalente Schritte und Verfahren ein, die dem Fachmann bekannt sind.

Im Folgenden wird die Erfindung anhand nicht limitierender Beispiele für konkrete Ausführungsformen näher erläutert. Die Beispiele sind insbesondere dahingehend zu interpretieren, dass sie nicht auf die konkret veranschaulichten Merkmalskombinationen beschränkt sind, sondern die beispielhaften Merkmale wiederum frei kombiniert werden können, solange die Aufgabe der Erfindung gelöst wird.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

NMR (1 H) wurde mit den folgenden Parametern durchgeführt.
Geräte: Bruker Avance DRX 500, Bruker Avance 400, Bruker DPX 300 Referenz: TMS
TD (Time Domaine = Anzahl der Datenpunkte oder digitale Auflösung): 65536 Lösungsmittel: DMSO d6
NS (Number of Scans = Häufigkeit der Abtastung): 32
SF (Spectrometer Frequence = Sendefrequenz): 500 MHz
TE (Temperatur): 303 K

HPLC-MS wurde mit den folgenden Parametern durchgeführt.
Gerät: Agilent Technologies 1200 Series
Methoden: ESI1ROD.M und POLAR.M (3,8 min., Lösungsmittel-Gradient)
Säule: ChromolithSpeedROD RP18e50-4.6
Lösungsmittel: Acetonitril + 0,05 % HCOOH / VE-Wasser + 0,04 % HCOOH Detektionswellenlänge: 220 nm
MS-Typ: API-ES

### BEISPIEL 1: Synthese von 2,4-Dichlor-7-morpholin-4-yl-chinazolin

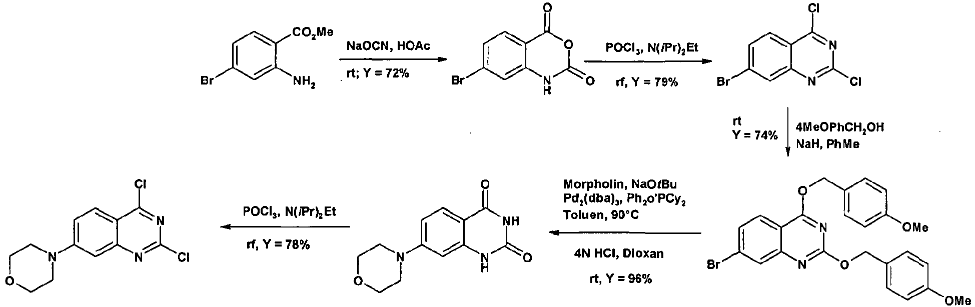

Zu 269,0 g (1,17 mol) Methyl-2-amino-4-brombenzoat in 1,2 L Essigsäure wurden unter Rühren bei Raumtemperatur 87,0 g (1,33 mol) Natriumcyanat gegeben und anschließend 22 h weiter gerührt. Das Reaktionsgemisch wurde mit 1,5 L Wasser verdünnt und der Rückstand abgesaugt. Der Feststoff wurde mit 0,42 L Natronlauge (32 %-ig) versetzt, mit 3,5 L Wasser verdünnt und auf dem Dampfbad 4 h erhitzt. Nach dem Abkühlen wurde der feste Rückstand abgesaugt. Nach üblicher Aufarbeitung erhielt man 205,2 g 7-Brom-1H-benzo[d][1,3]oxazin-2,4-dion; HPLC/MS (M+H)⁺ = 243 als Feststoff.

205,0 g (0,84 mol) 7-Brom-1H-benzo[d][1,3]oxazin-2,4-dion wurden bei Raumtemperatur in 1,26 L (12,59 mol) Phosphorylchlorid suspendiert, mit 71,34 mL (0,42 mol) N-Ethyldiisopropylamin versetzt und anschließend 36 h unter Rückfluss erhitzt. Nach üblicher Aufarbeitung erhielt man 193,6 g 7-Brom-2,4-dichlorchinazolin; HPLC/MS (M+H)⁺ = 277/279 als Feststoff.

Zu einer Suspension von 95,8 g (2,4 mol) Natriumhydrid [60 %-ig in Parafinöl] in 1,5 L Toluol wurden zwischen 15- und 20°C 199,0 mL (1,6 mol) 4-Methoxybenzylalkohol in 0,5 L Toluol getropft. Anschließend wurde 1 h bei Raumtemperatur nachgerührt. Danach wurden 193,6 g (0,67 mol) 7-Brom-2,4-dichlorchinazolin portionsweise hinzugegeben und das Reaktionsgemisch 48 h gerührt. Nach üblicher Aufarbeitung erhielt man 269,0 g 7-Brom-2,4-bis-(4-methoxy-benzyloxy)-chinazolin als Öl [Rohprodukt; wurde direkt weiter umgesetzt].

Unter Stickstoffatmosphäre wurden 2,26 g (2,47 mmol) Tris(dibenzylidenaceton)-di-palladium(0) und 4,42 g (12,35 mmol) 2-(Dicyclohexylphosphino)biphenyl in 1,5 L Toluol gelöst und nacheinander 215,2 mL (2,47 mol) Morpholin und 269,0 g (0,5 mol) 7-Brom-2,4-bis-(4-methoxy-benzyloxy)-chinazolin [in 5 L Toluol gelöst] zugegeben. Anschließend wurden 66,47 g (0,7 mol) Natrium-tert-butylat portionsweise hinzugefügt und 12 h bei 90°C gerührt. Nach üblicher Aufarbeitung erhielt man 249,0 g 2,4-Bis-(4-methoxy-benzyloxy)-7-morpholin-4-yl-chinazolin; HPLC/MS (M+H)⁺ = 488 als Öl [Rohprodukt; wurde direkt weiter umgesetzt].

Bei Raumtemperatur wurden 232,0 g (0,48 mol) 2,4-Bis-(4-methoxy-benzyloxy)-7-morpholin-4-yl-chinazolin in 300 mL Hydrogenchlorid/Dioxan (4 N) gelöst und 3 h gerührt. Nach üblicher Aufarbeitung erhielt man 112,5 g 7-Morpholin-4-yl-1H-chinazolin-2,4-dion; HPLC/MS (M+H)⁺ = 248 als Feststoff.

7,7 g (26,4 mmol) 7-Morpholin-4-yl-1H-chinazolin-2,4-dion wurden in 43,0 mL (46,7 mmol) Phosphorylchlorid bei Raumtemperatur suspendiert, anschließend 2,65 mL (15,57 mmol) N-Ethyldiisopropylamin hinzugefügt und 2 h unter Rückfluss erhitzt. Nach üblicher Aufarbeitung erhielt man 6,6 g 2,4-Dichlor-7-morpholin-4-yl-chinazolin*; HPLC/MS (M+H)⁺ = 284/286/288 als Feststoff.
¹H NMR (400 MHz, CDCl₃) δ 7.33 (d, J = 2.5, 1 H), 7.31 (d, J = 2.6, 1 H), 7.08 (d, J = 2.5, 1 H), 3.92 - 3.87 (m, 4H), 3.50 - 3.43 (m, 4H).
* Ishikawa et al. (1985) J. Med. Chem. 28: 1387; Verbindung 71.

### BEISPIEL 2.1: Synthese von 4-Chlor-7-morpholin-4-yl-chinazolin

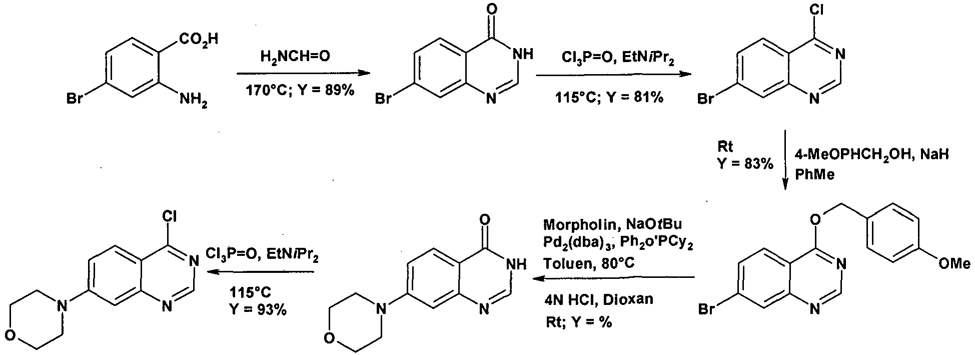

340,0 g (1,56 mol) 2-Amino-4-brom-benzoesäure wurden in 970 mL Formamid suspendiert und anschließend 12 h bei 170°C gerührt. Nach üblicher Aufarbeitung erhielt man 326,0 g 7-Brom-3H-chinazolin-4-on; HPLC/MS (M+H)⁺ = 226 als Feststoff.

Eine Suspension aus 55,0 g (0,24 mol) 7-Brom-3H-chinazolin-4-on in 300 mL Phosphoroxytrichlorid wure langsam mit 20,4 mL (0,12 mol) N-Ethyldiisopropylamin versetzt. Die Reaktionsmischung wurde 3 h bei 115°C gerührt und anschließend auf Raumtemperatur kommen gelassen. Nach üblicher Aufarbeitung erhielt man 48,0 g 7-Brom-4-chlor-chinazolin; HPLC/MS (M+H)⁺ = 244 als Feststoff.

Zu einer Suspension von 80,0 g (2,0 mol) Natriumhydrid [60 %-ig in Parafinöl] in 3,0 L Toluol wurden zwischen 15°C und 20°C 226,0 g (01,64 mol) 4-Methoxybenzylalkohol in 0,5 L Toluol getropft. Anschließend wurde 1 h bei Raumtemperatur nachgerührt. Danach wurden 165,9 g (1,64 mol) 7-Brom-4-chlor-chinazolin portionsweise hinzugegeben und das Reaktionsgemisch 48 h gerührt. Nach üblicher Aufarbeitung erhielt man 194,8 g 7-Brom-4-(4-methoxy-benzyloxy)-chinazolin als Feststoff.
¹H NMR (500 MHz, DMSO) ö 8.85 (s, 1H), 8.14 (s, 1H), 8.03 (d, J = 8.7, 1H), 7.79 (d, J = 10.7, 1H), 7.50 (d, J = 8.7, 2H), 6.97 (d, J 0 8.7, 2H), 5.56 (s, 2H), 3.77 (s, 3H).

Unter Stickstoffatmosphäre wurden 2,82 g (12,5 mmol) Tris(dibenzylidenaceton)-dipalladium(0) und 5,41 g (15,1 mmol) 2-(Dicyclohexylphosphino)biphenyl in 1,5 L Toluol gelöst und nacheinander 269,0 mL (3,1 mol) Morpholin und 213,0 g (0,62 mol) 7-Brom-4-(4-methoxy-benzyloxy)-chinazolin [in 2 L Toluol gelöst] zugegeben. Anschließend wurden 73,0 g (0,8 mol) Natrium-tert-butylat portionsweise hinzugefügt und gerührt. Nach üblicher Aufarbeitung erhielt man 178 g 4-(4-Methoxy-benzyloxy)-7-morpholin-4-yl-chinazolin; HPLC/MS (M+H)⁺ = 350 als Öl [Rohprodukt; wurde direkt weiter umgesetzt].

Bei Raumtemperatur wurden 178 g (0,15 mol) 4-(4-Methoxy-benzyloxy)-7-morpholin-4-yl-chinazolin in 250 mL Hydrogenchlorid/Dioxan (4 N) gelöst und 3 h gerührt. Nach üblicher Aufarbeitung erhielt man 45,0 g 7-Morpholin-4-yl-3H-chinazolin-4-on als Feststoff.
¹H NMR (500 MHz, DMSO) ö 11.84 (s, 1H), 7.97 (s, 1H), 7.93 (d, J = 9.0, 1H), 7.19 (dd, J = 9.0, 2.5, 1 H), 6.95 (d, J = 2.4, 1 H), 3.80 - 3.73 (m, 4H), 3.37 - 3.30 (m, 4H).

Zu einer Suspension von 3,5 g (15,11mmol) 7-Morpholin-4-yl-3H-chinazolin-4-on in 20 mL Phosphorylchlorid wurden langsam 1,3 mL (7,57mmol) N-Ethyldiisopropylamin zugetropft. Anschließend wurde die Reaktionsmischung 2 h bei 115°C gerührt. Nach üblicher Aufarbeitung erhielt man 3,75 g 4-Chlor-7-morpholin-4-yl-chinazolin; HPLC/MS (M+H)⁺ = 250 als Feststoff.
¹H NMR (500 MHz, DMSO) ö 8.84 - 8.79 (m, 1H), 8.03 (d, J = 9.4, 1H), 7.66 (dd, J = 9.4, 2.5, 1H), 7.19 (d, J = 2.5, 1 H), 3.82 - 3.74 (m, 4H), 3.53 - 3.45 (m, 4H).

### BEISPIEL 2.2: Alternative Synthese der Zwischenverbindung 7-Morpholin-4-yl-chinazolin-4-on

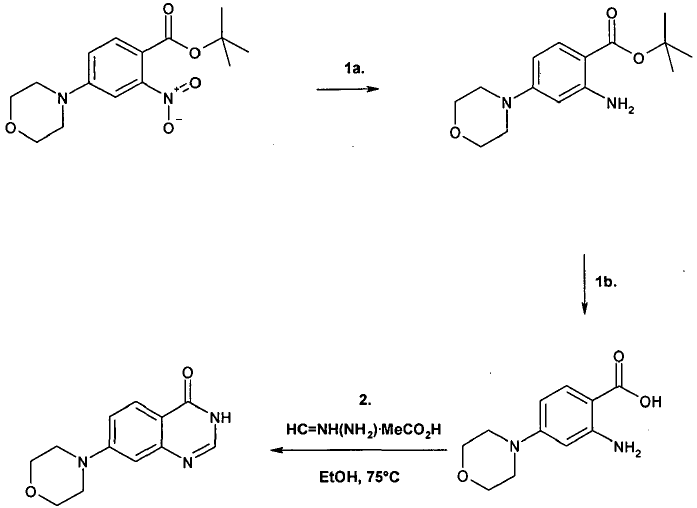

127 g (0,412 mol) 4-Morpholin-4-yl-2-nitro-benzoesäure-tert-butylester (WO 2007/06837 A1) wurden in 1000 mL Tetrahydrofuran gelöst, mit 12,7 g Palladium-Kohle (5 %, wasserfeucht) versetzt und 5 h bei Raumtemperatur über Wasserstoff gerührt. Nach üblicher Aufarbeitung erhielt man 4,5 g (Y = 100 %) 2-Amino-4-morpholin-4-yl-benzoesäure-tert-butylester.
¹H NMR (400 MHz, DMSO) δ 7.50 (d, *J* = 9.0, 1 H), 6.45 (s, 2H), 6.23 - 6.16 (m, 1 H), 6.13 (dd, J = 8.0, 2.5, 1 H), 3.76 - 3.67 (m, 4H), 3.17 - 3.08 (m, 4H).

2-Amino-4-morpholin-4-yl-benzoesäure-tert-butylester wurde nach der Methode aus US 2002/0165218 A1 zu 2-Amino-4-morpholin-4-yl-benzoesäure verseift.

4,22 g (0,019 mol) 2-Amino-4-morpholin-4-yl-benzoesäure und 4,0 g (0,038 mol) Formamidinacetat wurden in 40 mL Ethanol suspendiert. Anschließend wurde das Reaktionsgemisch 16 h unter Rückfluss gerührt. Nach üblicher Aufarbeitung erhielt man 2,2 g (Y = 50 %) 7-Morpholin-4-yl-3H-chinazolin-4-on.
¹H NMR (400 MHz, DMSO+TFA) δ 9.33 (s, 1H), 8.05 (d, *J =* 9.2, 1 H), 7.34 (dd, *J* = 9.2, 2.4, 1 H), 7.03 (d, *J* = 2.4, 1 H), 3.88 - 3.73 (m, 4H), 3.48 - 3.40 (m, 4H). BEISPIEL 2.3: Alternative Synthese von 4-Chlor-7-morpholin-4-yl-chinazolin 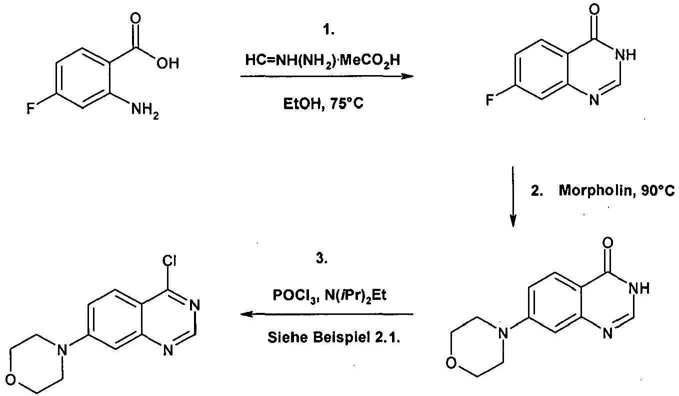 25,0 g (0,156 mol) 2-Amino-4-fluor-benzoesäure und 33,0 g (0,317 mol) Formamidinacetat wurden in 250 mL Ethanol suspendiert. Anschließend wurde das Reaktionsgemisch 16 h unter Rückfluss gerührt. Nach üblicher Aufarbeitung erhielt man 21,1 g (Y = 82 %) 7-Fluor-3H-chinazolin-4-on. ¹H NMR (400 MHz, DMSO) ö 12.31 (s, 1H), 8.19 (dd, *J* = 8.8, 6.4, 1H), 8.14 (s, 1H), 7.45 (dd, *J* = 10.1, 2.5, 1 H), 7.39 (td, *J* = 8.7, 2.6, 1 H). 20,0 g (0,122 mol) 7-Fluor-3H-chinazolin-4-on wurden in 80 mL Morpholin suspendiert und 16 h bei 90°C Innentemperatur gerührt. Nach üblicher Aufarbeitung erhielt man 14,1 g (Y = 50 %) 7-Morpholin-4-yl-3H-chinazolin-4-on (wie in Beispiel 2.2, Reaktionsschritt 1a beschrieben).

Die Synthese von 4-Chlor-7-morpholin-4-yl-chinazolin erfolgte wie in Beispiel 2.1 beschrieben.

### BEISPIEL 2.4: Synthese von 4-Chlor-6-fluor-7-morpholin-4-yl-chinazolin

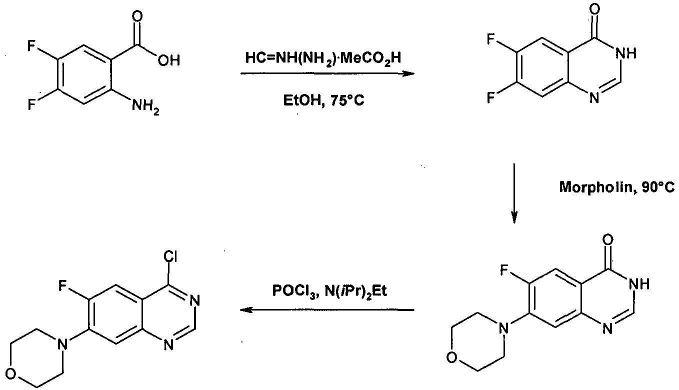

38,0 g (0,213 mol) 2-Amino-4,5-difluoro-benzoesäure und 44,0 g (0,423 mol) Formamidinacetat wurden in 300 mL Ethanol suspendiert. Anschließend wurde das Reaktionsgemisch 16 h unter Rückfluss gerührt. Nach üblicher Aufarbeitung erhielt man 33,37 g (Y = 84 %) 6,7-Difluoro-3H-chinazolin-4-on.
¹H NMR (400 MHz, DMSO) ö 12.54 (s, 1 H), 8.14 (d, *J =* 6.5, 1 H), 8.07 - 7.97 (m, 1 H), 7.73 (dt, *J =* 18.2, 9.1, 1 H).

37,2 g (0,20 mol) 6,7-Difluoro-3H-chinazolin-4-on wurden in 70 mL (0,80 mol) Morpholin suspendiert und 16 h bei 80°C Innentemperatur gerührt. Nach üblicher Aufarbeitung erhielt man 37,3 g (Y = 74 %) 6-Fluor-7-morpholin-4-yl-3H-chinazolin-4-on.
¹H NMR (400 MHz, DMSO) δ 8.03 (s, 1 H), 7.69 (d, *J =* 13.2, 1 H), 7.13 (d, *J* = 8.1, 1 H), 3.82 - 3.74 (m, 4H), 3.22 - 3.16 (m, 4H).

5,0 g (0,02 mol) 6-Fluor-7-morpholin-4-yl-3H-chinazolin-4-on wurden in 37,0 mL (0,04 mol) Phosphorylchlorid bei Raumtemperatur suspendiert, anschließend 3,4 mL (0,02 mol) N-Ethyldiisopropylamin hinzugefügt und 2 h unter Rückfluss erhitzt. Nach üblicher Aufarbeitung erhielt man 6,6 g 4-Chlor-6-fluor-7-morpholin-4-yl-chinazolin.
HPLC/MS (M+H)⁺ = 268/270/272 als Feststoff.

### BEISPIEL 3: Synthese von Piperidin-3-yl-thiazol-2-yl-methanol

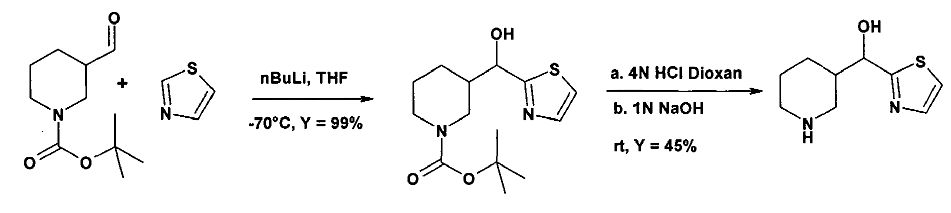

Unter Stickstoffatmosphäre wurden 1,25 mL (17,62mmol) Thiazol in 20 mL Tetrahydrofuran bei -70°C gelöst. Anschließend wurden 11,1 mL (17,62 mmol) [15 % in n-Hexan] zugetropft, so dass die Temperatur -67°C nicht überschritt. Es wurde auf Raumtemperatur erwärmt, danach auf -70°C abgekühlt und 3,76 g (17,62mmol) 3-Formyl-piperidin-1-carbonsäure tert. Butylester, gelöst in 5 mL Tetrahydrofuran, hinzugetropft. Es wurde 1 h bei -70°C gerührt, auf Raumtemperatur erwärmt und 2 h nachgerührt. Nach üblicher Aufarbeitung erhielt man 5,18 g 3-(Hydroxy-thiazol-2-yl-methyl)-piperidin-1-carbonsäure tert. Butylester; HPLC/MS (M+H)⁺ = 299 als Öl.

Bei Raumtemperatur wurden 1,9 g (6,4 mmol) 3-(Hydroxy-thiazol-2-yl-methyl)-piperidin-1-carbonsäure tert. Butylester in 15 mL Hydrogenchlorid/Dioxan (4 N) gelöst und 3 h gerührt. Das Lösungsmittel wurde im Vakuum entfernt und der Rückstand in 20 mL Wasser gelöst. Die wässrige Phase wurde auf pH = 9 mit Natronlauge (1 N) eingestellt und mit 2x25 mL Essigester extrahiert. Nach üblicher Aufarbeitung erhielt man 570 mg Piperidin-3-yl-thiatol-2-yl-methanol; HPLC/MS (M+H)⁺ = 199 als Öl.

### BEISPIEL 4: Synthese von 2-Piperidin-3-ylmethyl-benzothiazol

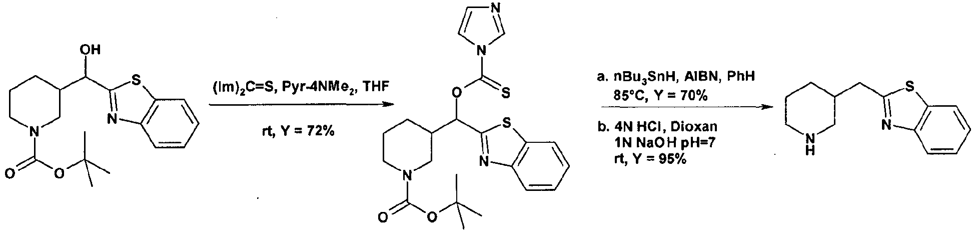

Bei Raumtemperatur wurden 1,0 g (2,87 mmol) 3-(Benzothiazol-2-yl-hydroxy-methyl)-piperidin-1-carbonsäure tert. Butylester in 30 mL Tetrahydrofuran gelöst. Anschließend wurden 0,35 g (2,87 mmol) Dimethyl-pyridin-4-ylamin und 1,53 g (8,61 mmol) 1,1'-Thiocarbonyldiimidazol zugegeben. Danach wurde das Reaktionsgemisch 18 h bei Raumtemperatur gerührt. Nach üblicher Aufarbeitung erhielt man 1,08 g 3-[Benzothiazol-2-yl-(imidazol-1-carbothioyloxy)-methyl]-piperidin-1-carbonsäure tert. Butylester; HPLC/MS (M+H)⁺ = 459 als Öl.

Bei Raumtemperatur wurden nacheinander 1,15 g (4,36 mmol) Tributylzinnhydrid, 53,71 mg (0,33 mmol) α, α'-Azoisobutyronitril und 1,0 g (2,18 mmol) 3-[Benzothiazol-2-yl-(imidazol-1-carbothioyloxy)-methyl]-piperidin-1-carbonsäure tert. Butylester in 10 mL Benzol gelöst. Nach 2 min unter Rühren wurde Vakuum angelegt und 2 min entgast. Dieser Vorgang wurde wiederholt. Im Anschluss wurde die Reaktionsmischung 45 min unter Rückfluss gerührt. Nach üblicher Aufarbeitung erhielt man 0,58 g 3-Benzothiazol-2-ylmethyl-piperidin-1-carbonsäure tert. Butylester; HPLC/MS (M+H)⁺ = 333 als Öl.

0,58 g (1,75 mmol) 3-Benzothiazol-2-ylmethyl-piperidin-1-carbonsäure tert. Butylester wurden in 5,0 mL Hydrogenchlorid/Dioxan (4 N) gelöst und bei Raumtemperatur 1 h gerührt. Das Lösungsmittel wurde im Vakuum entfernt und der Rückstand in 50 mL Wasser gelöst. Die wässrige Phase wurde auf pH = 9 mit Natronlauge (1 N) eingestellt und mit 2x50 mL Essigester extrahiert. Nach üblicher Aufarbeitung erhielt man 0,41 g 2-Piperidin-3-ylmethyl-benzothiazol; HPLC/MS (M+H)⁺ = 233 als Öl.

### BEISPIEL 5: Synthese von 3-Thiophen-3-ylmethyl-piperidin

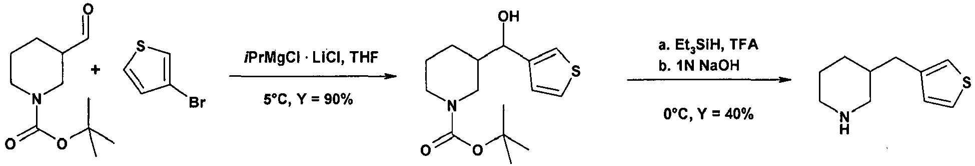

Unter Stickstoffatmosphäre wurden zu 24,77 mL (32,2 mmol) Isopropyl-magnesiumchlorid-Lithiumchlorid-Komplex (1,3 M in Tetrahydrofuran) bei 5°C 5,0 g (30,67 mmol) 3-Bromthiophen in 5 mL Tetrahydrofuran langsam zugetropft. Nach 0,5 h Rühren bei 5°C wurden 6,54 g (30,67 mmol) 3-Formyl-piperidin-1-carbonsäure tert. Butylester langsam zugetropft. Nach dem Entfernen des Kältebades wurde das Reaktionsgemisch 1 h nachgerührt. Nach üblicher Aufarbeitung erhielt man 10,3 g 3-(Hydroxy-thiophen-3-yl-methyl)-piperidin-1-carbonsäure tert. Butylester; HPLC/MS (M+H- tBuO)⁺ = 244 als Öl (Diasteromere).

1,0 g (3,36 mmol) 3-(Hydroxy-thiophen-3-yl-methyl)-piperidin-1-carbonsäure tert. Butylester als Diastereomerengemisch wurden bei 0°C mit 15 mL Trifluoressigsäure gerührt. Nach 15 min wurden 0,80 mL (5,04 mmol) Triethylsilan zugetropft, das Kältebad entfernt und 18 h bei Raumtemperatur gerührt. Nach üblicher Aufarbeitung erhielt man 0,41 g 3-Thiophen-3-ylmethyl-piperidin; HPLC/MS (M+H)⁺ = 182 als Öl.

### BEISPIEL 6: Synthese von 2-Chlor-7-morpholin-4-yl-4-(3-thizol-2-ylmethyl-piperidin-1-yl)-chinazolin

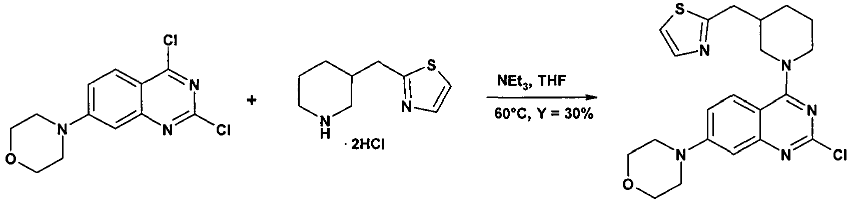

213 mg (0,83 mmol) 3-Thizol-2-ylmethyl-piperidin Dihydrochlorid wurden in 10 mL Tetrahydrofuran suspendiert, 0,3 mL (2,1 mmol) Triethylamin hinzugefügt und 5 min gerührt. Danach wurden 280 mg (0,7 mmol) 2,4-Dichlor-7-morpholin-4-yl-chinazolin hinzugegeben und 2,5 h auf 60°C erhitzt. Nach üblicher Aufarbeitung erhielt man 87 mg 2-Chlor-7-morpholin-4-yl-4-(3-thizol-2-ylmethyl-piperidin-1-yl)-chinazolin (Nr. 36).

### BEISPIEL 7: Synthese von 7-Morpholin-4-yl-4-((S)-3-phenyl-piperidin-1-yl)-chinazolin

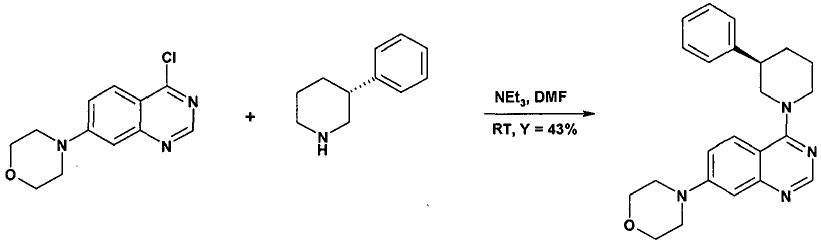

155 mg (0,96 mmol) (R)-3-Phenylpiperidin wurden in 2 mL N,N-Dimethyllformamid gelöst und 3 d bei Raumtemperatur gerührt. Nach üblicher Aufarbeitung erhielt man 130 mg 7-Morpholin-4-yl-4-((S)-3-phenyl-piperidin-1-yl)-chinazolin (Nr. 5).

### BEISPIEL 8: Synthese von 2-Chlor-7-morpholin-4-yl-4-piperidin-1-yl-chinazolin, 7-Morpholin-4-yl-4-piperidin-1-yl-chinazolin-2-ylamin und 7-Morpholin-4-yl-4-piperidin-1-yl-chinazolin-2-carbonitril

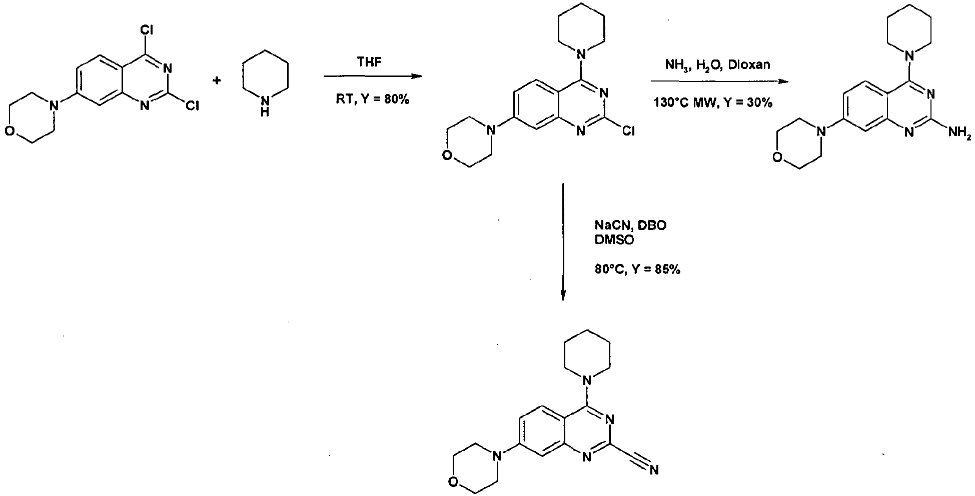

1,59 g (5,0 mmol) 2,4-Dichlor-7-morpholin-4-yl-chinazolin wurden in 50 mL Tetrahydrofuran suspendiert, bei Raumtemperatur mit einer Lösung von 1,24 mL (12,50 mmol) Piperidin in 20 mL Tetrahydrofuran versetzt und 3 h gerührt. Nach üblicher Aufarbeitung erhielt man 1,33g 2-Chlor-7-morpholin-4-yl-4-piperidin-1-yl-chinazolin (Nr. 2).

In einem cem-Mikrowellengerät wurden 0,166 g (0,50 mmol) 2,4-Dichlor-7-morpholin-4-yl-chinazolin und 1,0 mL Ammoniaklösung (32 %) in 2,0 mL Dioxan 150 min bei 130°C (80 Watt) erhitzt. Nach üblicher Aufarbeitung erhielt man 0,04 g 7-Morpholin-4-yl-4-piperidin-1-yl-chinazolin-2-yl-amin (Nr. 57).

0,20 g (0,60 mmol) 2-Chlor-7-morpholin-4-yl-4-piperidin-1-ylchinazolin und 0,067 g (0,60 mmol) 1,4-Diazabicyclo[2.2.2]octan wurden in 3,0 mL Dimethylsulfoxid gelöst. Nach 1,0 h wurden 0,03 g (0,60 mmol) Natriumcyanid hinzugegeben und die Reaktionsmischung anschließend 24 h bei 80°C gerührt. Nach üblicher Aufarbeitung erhielt man 0,17 g 7-Morpholin-4-yl-4-piperidin-1-yl-chinazolin-2-carbonitril (Nr. 58).

Weitere analog hergestellte Verbindungen finden sich in der nachfolgenden Tabelle 2.

**Tab. 2: Verbindungen der Formeln (IA), (IB) und (IC)**

| **Nr**. | **Strukturformel** | **Name** | **Analytik** ¹H NMR (400 MHz, DMSO) δ HPLC-MS (M+H) | **Bindung DNA-PK Ic₅₀ [µM]** |
|---|---|---|---|---|
| 1 | 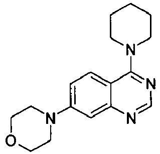 | 7-Morpholin-4-yl-4-piperidin-1-yl-chinazolin | 8.44 (s, 1 H), 7.76 (d, *J* = 9.3, 1 H), 7.29 (d, *J* = 9.3, 1 H), 6.99 (s, 1 H), 3.81 - 3.72 (m, 4H), 3.60 (s, 4H), 3.34 - 3.28 (m, 4H), 1.68 (s, 6H). (M+H) 299. | < 0.1 |
| 2 | 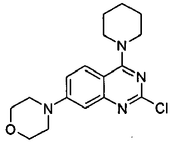 | 2-Chloro-7-morpholin-4-yl-4-piperidin-1-yl-chinazolin | 7.76 (s, 1 H), 7.25 (s, 1 H), 6.91 (d, *J* = 2.6, 1 H), 3.79 - 3.73 (m, 4H), 3.69 (s, 4H), 3.38 - 3.32 (m, 4H), 1.68 (s, 6H). (M+H) 333. | < 0.1 |
| 3 | 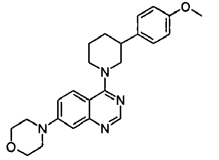 | 4-[3-(4-Methoxyphenyl)-piperidin-1-yl]-7-morpholin-4-yl-chinazolin | 8.46 (s, 1 H), 7.81 (d, *J* = 9.3, 1 H), 7.30 (dd, *J* 9.3, 2.6, 1H), 7.25 (d, *J* = 8.6, 2H), 7.00 (d, *J* = 2.6, 1 H), 6.88 (t, *J* = 13.2, 2H), 4.30 (d, *J* = 12.9, 1 H), 4.23 (d, *J* = 12.7, 1 H), 3.81 - 3.76 (m, 4H), 3.74 (s, 3H), 3.35 - 3.30 (m, 4H), 3.18 - 3.09 (m, 2H), 2.92 (dd, *J* = 11.2, 7.6, 1 H), 2.00 (s, 1 H), 1.90 - 1.71 (m, 3H). (M+H) 405. | 0.1-0.5 |
| 4 | 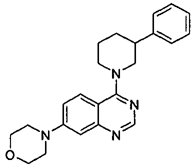 | 7-Morpholin-4-yl-4-(3-phenyl-piperidin-1-yl)-chinazolin | 8.47 (s, 1 H), 7.82 (d, *J* = 9.3, 1 H), 7.37 - 7.29 (m, 5H), 7.25 (dt, *J =* 12.3, 4.2, 1H), 7.01 (d, *J* = 2.6, 1H), 4.28 (dd, *J* = 23.4, 12.9, 2H), 3.81 - 3.73 (m, 4H), 3.35 - 3.32 (m, 4H), 3.22 - 3.13 (m, 2H), 2.98 (dd, *J =* 13.0, 9.3, 1 H), 2.02 (t, *J* = 9.7, 1 H), 1.91 - 1.73 (m, 3H). (M+H) 375. | < 0.1 |
| 5 | 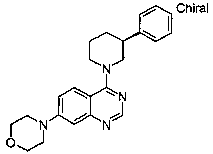 | 7-Morpholin-4-yl-4-((S)-3-phenyl-piperidin-1-yl)-chinazolin | 8.44 (s, 1 H), 7.81 (d, *J* = 9.3, 1 H), 7.33 (d, *J* = 4.3, 4H), 7.31 - 7.22 (m, 2H), 6.99 (d, *J* = 2.5, 1 H), 4.28 (dd, *J* = 22.8, 12.9, 2H), 3.82 - 3.72 (m, 4H), 3.35 - 3.31 (m, 4H), 3.18 (dd, *J* = 22.7, 10.3, 2H), 2.96 (dd, *J* = 12.9, 9.2, 1 H), 2.00 (d, *J* = 11.5, 1 H), 1.93 -1.73 (m, 3H). (M+H) 375. | 0.1-0.5 |
| 6 | 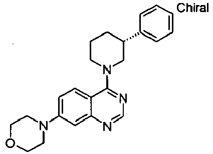 | 7-Morpholin-4-yl-4-((R)-3-phenyl-piperidin-1-yl)-chinazolin | 8.46 (d, *J* = 5.0, 1 H), 7.82 (d, *J* = 9.3, 1 H), 7.35 - 7.30 (m, 5H), 7.25 (dq, *J* = 8.7, 4.3, 1 H), 7.00 (d, *J =* 2.6, 1 H), 4.28 (dd, *J* = 23.1, 12.8, 2H), 3.81 - 3.72 (m, 4H), 3.36 - 3.32 (s, 4H), 3.19 (p, *J* 10.2, 2H), 3.03 - 2.93 (m, 1 H), 2.03 (d, *J* = 7.2, 1H), 1.92 - 1.75 (m, 3H). (M+H) 375. | < 0.1 |
| 7 | 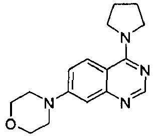 | 7-Morpholin-4-yl-4-pyrrolidin-1-yl-chinazolin | 8.29 (s, 1 H), 8.29 (s, 1 H), 8.10 (d, *J* = 9.4, 1 H), 7.18 (dd, *J* = 9.4, 2.7, 1 H), 6.93 (d, *J* = 2.7, 1 H), 3.81 (t, *J =* 6.5, 4H), 3.78 - 3.74 (m, 4H), 3.35 - 3.25 (m, 4H), 1.95 (dd, *J* = 8.0, 5.1, 4H). (M+H) 285. | 0.1-0.5 |
| 8 | 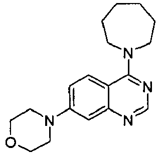 | 4-Azepan-1-yl-7-morpholin-4-yl-chinazolin | 8.31 (s, 1 H), 7.93 (d, *J* = 9.5, 1 H), 7.19 (dd, *J* = 9.5, 2.7, 1 H), 6.94 (d, *J* = 2.7, 1 H), 3.90 - 3.81 (m, 4H), 3.79 - 3.73 (m, 4H), 3.35 - 3.24 (m, 4H), 1.87 (s, 4H), 1.56 (d, *J* = 3.2, 4H). (M+H) 313. | 0.1-0.5 |
| 9 | 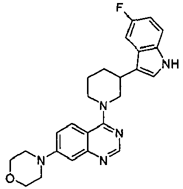 | 4-[3-(5-Fluor-1H-indol-3-yl)-piperidin-1-yl]-7-morpholin-4-yl-chinazolin | 8.49 - 8.44 (m, 1 H), 7.85 (d, *J* = 9.3, 1 H), 7.42 - 7.27 (m, 3H), 7.24 (d, *J* = 2.3, 1 H), 7.00 (d, *J* = 2.5, 1 H), 6.92 (td, *J* = 9.2, 2.5, 1 H), 4.35 (d, *J =* 13.0, 2H), 3.80 - 3.75 (m, 4H), 3.36 - 3.31 (m, 4H), 3.30 - 3.26 (m, 3H), 3.11 (ddd, *J* = 11.8, 7.7, 3.5, 1 H), 2.09 (d, *J* = 10.8, 2H), 1.85 (qd, *J* = 12.6, 3.5, 2H). (M+H) 432. | 0.5-1.0 |
| 10 | 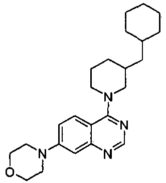 | 4-(3-Cyclohexylmethyl-piperidin-1-yl)-7-morpholin-4-yl-chinazolin | 8.47 (d, *J* = 15.1, 1H), 7.80 (d, *J* = 9.3, 1 H), 7.33 (dd, *J* = 9.3, 2.6, 1 H), 7.03 (t, *J* = 9.8, 1 H), 4.17 (d, *J* = 12.9, 2H), 3.86 - 3.80 (m, 4H), 3.40 - 3.34 (m, 4H), 3.17 - 3.07 (m, 1H), 2.80 (dd, *J* = 12.8, 10.5, 1 H), 1.94 (t, *J* = 14.5, 1 H), 1.88 - 1.63 (m, 7H), 1.44 - 1.33 (m, 1 H), 1.32 - 1.12 (m, 7H), 0.98 - 0.85 (m, 2H). (M+H) 395. | 0.5-1.0 |
| 11 | 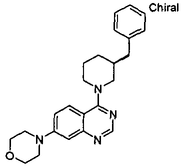 | 4-((S)-3-Benzyl-piperidin-1-yl)-7-morpholin-4-yl-chinazolin | 8.41 (s, 1H), 7.55 (d, *J* = 9.3, 1 H), 7.34 - 7.27 (m, 2H), 7.25 - 7.19 (m, 3H), 7.11 (dd, *J* = 9.3, 2.6, 1H), 6.96 (d, *J* = 2.5, 1H), 4.13 (d, *J* = 13.1, 1H), 4.03 (d, *J* = 12.7, 1H), 3.80-3.72 (m, 4H), 3.37 - 3.28 (s, 4H), 3.10 - 3.00 (m, 1H), 2.81 (dd, *J* = 12.9, 10.5, 1 H), 2.68 - 2.53 (m, 2H), 2.02 (ddd, *J* = 10.6, 7.2, 3.4, 1 H), 1.90 - 1.76 (m, 2H), 1.70 - 1.59 (m, 1 H), 1.35 - 1.24 (m, 1H). (M+H) 389. | 0.1-0.5 |
| 12 | 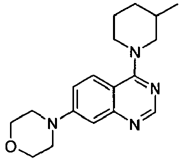 | 4-(3-Methylpiperidin-1-yl)-7-morpholin-4-yl-chinazolin | 8.44 (s, 1 H), 7.77 (d, *J* = 9.3, 1 H), 7.30 (dd, *J* = 9.3, 2.6, 1 H), 6.99 (s, 1H), 4.12 (t, *J* = 14.4, 2H), 3.83-3.69 (m, 4H), 3.38 - 3.24 (m, 4H), 3.06 (t, *J* = 12.3, 1 H), 2.75 (dt, *J =* 24.4, 12.2, 1 H), 1.83 (ddd, *J* = 31.5, 13.8, 3.7, 2H), 1.70 - 1.58 (m, 1 H), 1.21 (dt, *J =* 12.0. 8.2, 1H), 0.92 (d, *J* = 6.6, 3H). (M+H) 313. | 0.1-0.5 |
| 13 | 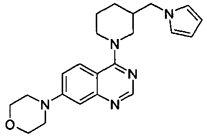 | 7-Morpholin-4-yl-4-(3-pyrrol-1-ylmethyl-piperidin-1-yl)-chinazolin | 8.43 (s, 1 H), 7.59 (d, *J* = 9.3, 1 H), 7.19 (d, *J* = 11.9, 1 H), 6.98 (s, 1H), 6.76 (s, 2H), 6.01 (s, 2H), 4.09 (d, *J* = 12.9, 1 H), 3.94 - 3.83 (m, 3H), 3.80 - 3.75 (m, 4H), 3.33 - 3.29 (m, 4H), 3.04 (t, *J* = 12.2, 1H), 2.89 - 2.81 (m, 1 H), 2.18 (dd, *J* = 22.9, 5.4, 1 H), 1.83 (dd, *J* = 14.6, 5.0, 1 H), 1.79 - 1.73 (m, 1 H), 1.66 (dd, *J =* 20.6, 11.5, 1 H), 1.33 - 1.22 (m, 1 H). (M+H) 378. | < 0.1 |
| 14 | 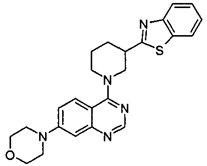 | 4-(3-Benzothiazol-2-yl-piperidin-1-yl)-7-morpholin-4-yl-chinazolin | 8.51 (s, 1 H), 8.08 (d, *J* = 7.9, 1 H), 7.97 (s, 1 H), 7.87 (d, *J* = 9.3, 1 H), 7.50 (t, *J* = 7.6, 1 H), 7.42 (t, *J* = 7.1, 1 H), 7.33 (d, *J* = 9.4, 1H), 7.02 (s, 1H), 4.51 *(d, J* = 12.8, 1 H), 4.12 (d, *J* = 13.0, 1 H), 3.79 - 3.74 (m, 4H), 3.62 (dd, *J* = 15.1, 8.6, 1 H), 3.56 - 3.48 (m, 1H), 3.35 - 3.32 (m, 4H), 2.37 - 2.32 (m, 1 H), 2.03 - 1.78 (m, 4H). (M+H) 432. | < 0.1 |
| 15 | 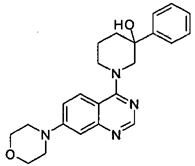 | 1-(7-Morpholin-4-yl-quinazolin-4-yl)-3-phenyl-piperidin-3-ol | 10.54 (sbr, 1 H), 8.43 (s, 1 H), 7.95 (d, *J* = 9.4, 1 H), 7.61 - 7.52 (m, 2H), 7.35 (t, *J* = 7.6, 2H), 7.30 - 7.20 (m, 2H), 6.97 (d, *J* = 2.5, 1 H), 4.37 (d, *J* = 12.9, 1 H), 4.03 (d, *J* = 13.2, 1 H), 3.80 - 3.71 (m, 4H), 3.55 (d, *J =* 13.2, 1 H), 3.32 (m, 4H), 3.25 - 3.14 (m, 1 H), 3.24 - 3.17 (m, 1 H), 2.30 - 2.07 (m, 1 H), 1.83 (d, *J* = 12.5, 1H), 1.65 (d, *J* = 12.4, 1 H). (M+H) 391. | 0.5-1.0 |
| 16 | 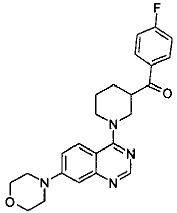 | (4-Fluor-phenyl)-[1-(7-morpholin-4-yl-chinazolin-4-yl)-piperidin-3-yl]-methanon | 8.55 (s, 1 H), 8.22 (dd, *J* = 8.8, 5.6, 2H), 7.85 (d, *J* = 9.3, 1 H), 7.41 (t, *J* = 8.8, 2H), 7.30 (dd, *J* = 9.4, 2.6, 1 H), 7.02 (d, *J* = 2.5, 1 H), 4.38 (d, *J* = 13.0, 1 H), 4.21 *(d, J* = 12.9, 1 H), 3.90 (dd, *J* = 12.2, 8.6, 1 H), 3.80 - 3.74 (m, 4H), 3.37 - 3.34 (m, 4H), 3.24 (dd, *J* = 13.0, 10.6, 2H), 2.04 (s, 1 H), 1.74 (ddd, *J* = 14.8, 14.3, 9.7, 3H). (M+H) 421. | 0.1-0.5 |
| 17 | 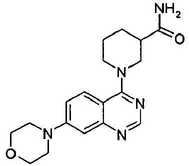 | 1-(7-Morpholin-4-yl-chinazolin-4-yl)-piperidin-3-carbonsäureamid | 8.46 (s, 1 H), 7.81 (d, *J* = 9.3, 1 H), 7.37 (s, 1 H), 7.30 (d, *J* = 9.3, 1 H), 7.01 (s, 1 H), 6.86 (s, 1 H), 4.18 (dd, *J* = 32.3, 13.7, 2H), 3.81 - 3.75 (m, 4H), 3.35 (s, 5H), 3.07 (d, *J* = 11.4, 1 H), 2.02 - 1.92 (m, 1 H), 1.84 - 1.74 (m, 1H), 1.71 - 1.60 (m, 2H). (M+H) 342. | 0.5-1.0 |
| 18 | 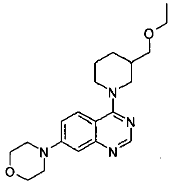 | 4-(3-Ethoxymethyl-piperidin-1-yl)-7-morpholin-4-yl-chinazolin | 8.44 (s, 1H), 7.78 (d, *J* = 9.3, 1H), 7.27 (d, *J* = 9.3, 1 H), 6.99 (s, 1 H), 4.19 - 3.95 (m, 2H), 3.81 - 3.72 (m, 4H), 3.48 - 3.38 (m, 2H), 3.46 - 3.35 (m, 2H), 3.35-3.30 (d, *J* = 4.9, 4H), 3.15 (t, *J* = 10.7, 1 H), 2.97 (dd, *J* = 12.9, 9.9, 1 H), 1.97 (s, 1 H), 1.80 (t, *J* = 15.7, 2H), 1.71 - 1.60 (m, 1 H), 1.31 (dd, *J* = 27.0, 7.4, 1 H), 1.09 (t, *J* = 14.0, 3H). (M+H) 357. | 0.1-0.5 |
| 19 | 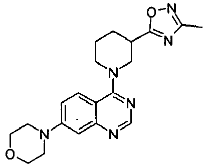 | 4-[3-(3-Methyl-[1,2,4]oxadiazol-5-yl)-piperidin-1-yl]-7-morpholin-4-yl-chinazolin | 8.50 (s, 1 H), 7.83 (d, *J* = 9.3, 1 H), 7.33 (d, *J* = 9.4, 1 H), 7.03 (s, 1 H), 4.37 (q, *J* = 9.1, 1 H), 4.04 (d, *J =* 13.2, 1H), 3.83 - 3.72 (m, 4H), 3.50 (dd, *J* = 14.2, 5.6, 2H), 3.37 - 3.32 (m, 5H), 2.33 (s, 3H), 2.25 (d, *J =* 15.0, 1 H), 1.96 - 1.84 (m, 2H), 1.82 - 1.73 (m, 1H). (M+H) 381. | 0.5-1.0 |
| 20 | 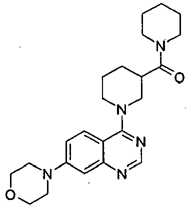 | [1-(7-Morpholin-4-yl-quinazolin-4-yl)-piperidin-3-yl]-piperidin-1-yl-methanon | 8.44 (d, *J* = 5.0, 1 H), 7.79 (d, *J* = 9.3, 1 H), 7.28 (dd, *J* = 9.3, 2.6, 1 H), 6.99 (d, *J* = 2.5, 1 H), 4.23 - 4.16 (m, 2H), 3.79 - 3.73 (m, 4H), 3.57 (dd, *J* = 30.5, 13.4, 2H), 3.49 - 3.39 (m, 2H), 3.35 - 3.30 (m, 4H), 3.16 (ddd, *J = 37.9,* 22.8, 12.8, 2H), 3.03 (dd, *J* = 14.2, 6.9, 1 H), 1.86 (d, *J* = 11.8, 1 H), 1.76 - 1.37 (m, 9H). (M+H) 410. | 0.1-0.5 |
| 21 | 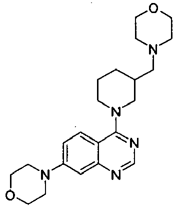 | 7-Morpholin-4-yl-4-(3-morpholin-4-ylmethyl-piperidin-1-yl)-chinazolin | 8.43 (s, 1 H), 7.81 (d, *J* = 9.3, 1H), 7.26 (dd, *J* = 9.3, 2.5, 1 H), 6.98 (d, *J* = 2.5, 1 H), 4.16 (d, *J* = 11.4, 1 H), 4.00 (d, *J* = 13.0, 1H), 3.81 - 3.72 (m, 4H), 3.54 (s, 4H), 3.38 - 3.31 (m, 8H), 3.18 (dd, *J* = 17.0, 6.6, 1 H), 2.93 (dd, *J* = 13.0, 9.5, 1 H), 2.41 (s, 1 H), 2.24 (dd, *J* = 13.4, 8.3, 1 H), 2.13 (dd, *J* = 12.2, 5.4, 1 H), 2.03 - 1.94 (m, 1 H), 1.86 - 1.74 (m, 2H), 1.64 (dd, *J* = 23.8, 10.8, 1 H). (M+H) 398. | 0.5-1.0 |
| 22 | 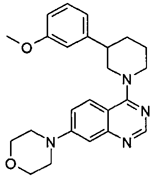 | 4-[3-(3-Methoxyphenyl)-piperidin-1-yl]-7-morpholin-4-yl-chinazolin | 8.45 (d, *J* = 5.4, 1 H), 7.80 (d, *J* = 9.3, 1H), 7.27 (ddd, *J* = 25.3, 12.4, 6.9, 2H), 6.99 (d, *J* = 2.6, 1 H), 6.90 (dd, *J* = 12.7, 5.6, 2H), 6.83 - 6.77 (m, 1 H), 4.26 (dd, *J* = 26.2, 12.7, 2H), 3.78 - 3.71 (m, 7H), 3.31 (dd, *J* = 10.5, 5.7, 4H), 3.23 - 3.12 (m, 2H), 2.94 (dd, *J* = 12.8, 9.1, 1 H), 2.01 - 1.95 (m, 1 H), 1.82 (ddd, *J* = 20.6, 11.6, 4.3, 3H). (M+H) 405. | < 0.1 |
| 23 | 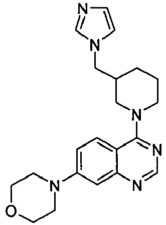 | 4-(3-Imidazol-1-ylmethyl-piperidin-1-yl)-7-morpholin-4-yl-chinazolin | 8.43 (s, 1 H), 7.66 - 7.57 (m, 2H), 7.20 (dd, *J* = 8.7, 3.1, 2H), 6.97 (s, 1 H), 6.92 (s, 1 H), 4.07 (d, *J* = 13.0, 1 H), 3.97 - 3.89 (m, 2H), 3.80 - 3.72 (m, 4H), 3.33 - 3.29 (m, 5H), 3.07 (dd, *J* = 14.7, 7.0, 1 H), 2.87 (dd, *J* = 12.9, 10.3, 1 H), 2.19 (t, *J* = 13.8, 1 H), 1.87 - 1.59 (m, 3H), 1.28 (dd, *J* = 17.6, 6.4, 1 H). (M+H) 379. | < 0.1 |
| 24 | 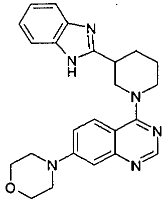 | 4-[3-(1 H-Benzoimidazol-2-yl)-piperidin-1-yl]-7-morpholin-4-yl-chinazolin | 8.49 (s, 1H), 7.85 (d, *J* = 9.3, 1H), 7.55 (d, *J* = 7.4, 1 H), 7.43 (d, *J =* 7.5, 1 H), 7.33 (dd, *J* = 9.4, 2.6, 1 H), 7.19 - 7.09 (m, 3H), 7.02 (d, *J* = 2.6, 1H), 4.30 (d, *J* = 13.2, 1 H), 3.81 - 3.73 (m, 4H), 3.29 - 3.21 (m, 4H), 3.12 - 2.91 (m, 2H), 2.71 - 2.63 (m, 1 H), 2.19 - 2.12 (m, 1 H), 2.04 (ddd, *J* = 41.0, 25.3, 9.6, 2H), 1.78 - 1.68 (m, 1H). (M+H) 415. | 0.5-1.0 |
| 25 | 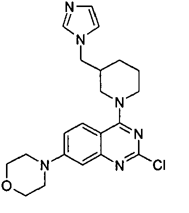 | 2-Chlor-4-(3-imidazol-1-ylmethyl-piperidin-1-yl)-7-morpholin-4-yl-chinazolin | 7.66 - 7.55 (m, 2H), 7.21 (s, 1 H), 7.15 (d, *J* = 12.0, 1 H), 6.96 - 6.87 (m, 2H), 4.16 (d, *J* = 13.1, 1 H), 3.97 (dd, *J* = 12.4, 9.0, 3H); 3.80 - 3.70 (m, 4H), 3.37 - 3.32 (m, 4H), 3.14 (dd, *J* = 17.7, 6.8, 1H), 3.04 - 2.94 (m, 1 H), 2.20 (dd, *J* = 12.0, 5.1, 1 H), 1.87 - 1.79 (m, 1 H), 1.74 (dd, *J* = 10.3, 5.9, 1 H), 1.68 - 1.56 (m, 1 H), 1.33 - 1.23 (m, 1 H). (M+H) 413. | < 0.1 |
| 26 | 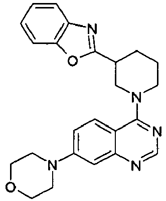 | 4-(3-Benzooxazol-2-yl-piperidin-1-yl)-7-morpholin-4-yl-chinazolin | 8.49 (s, 1 H), 7.86 (d, *J* = 9.3, 1 H), 7.70 (t, *J =* 7.9, 2H), 7.41 -7.27 (m, 3H), 7.02 (d, *J* = 2.4, 1 H), 4.52 (d, *J* = 11.9, 1H), 4.10 (d, *J* = 13.2, 1H), 3.80 - 3.71 (m, 4H), 3.52 (ddd, *J =* 16.2, 15.0, 8.3, 2H), 3.36 - 3.32 (m, 4H), 2.40 - 2.30 (m, 1 H), 2.05 - 1.74 (m, 4H). (M+H) 416. | 0.1-0.5 |
| 27 | 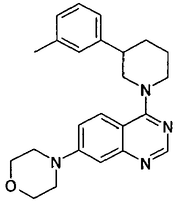 | 7-Morpholin-4-yl-4-(3-m-tolyl-piperidin-1-yl)-chinazolin | 8.46 (s, 1H), 7.80 (d, *J* = 9.3, 1 H), 7.29 (dd, *J* = 9.4, 2.6, 1 H), 7.21 (t, *J* = 7.5, 1 H), 7.15 - 7.09 (m, 2H), 7.04 (d, *J* = 7.4, 1 H), 6.99 (d, *J* = 2.5, 1 H), 4.29 (dd, *J* = 28.8, 12.8, 2H), 3.76 (dd, *J* = 11.1, 6.4, 4H), 3.32 - 3.28 (m, 4H), 3.23 - 3.13 (m, 2H), 2.92 (t, *J* = 11.0, 1 H), 2.30 (s, 3H), 1.99 (s, 1 H), 1.88-1.71 (m, 3H). (M+H) 389. | < 0.1 |
| 28 | 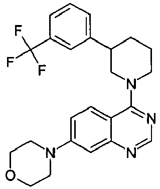 | 7-Morpholin-4-yl-4-[3-(3-trifluoromethyl-phenyl)-piperidin-1-yl]-chinazolin | 8.46 (s, 1 H), 7.82 (d, *J* = 9.3, 1 H), 7.72 - 7.65 (m, 2H), 7.59 (dt, *J =* 15.1, 7.6, 2H), 7.29 (dd, *J* = 9.3, 2.4, 1 H), 7.00 (d, *J* = 2.4, 1 H), 4.27 (t, *J* = 13.1, 3H), 3.81 - 3.74 (m, 4H), 3.34 - 3.30 (m, 4H), 3.22 (t, *J* = 12.0, 1H), 3.16 - 3.07 (m, 1 H), 2.02 (s, 1 H), 1.84 (dt, *J* = 18.3, 13.1, 3H). (M+H) 443. | 0.1-0.5 |
| 29 | 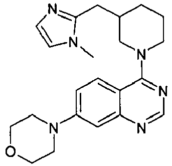 | 4-[3-(1-Methyl-1H-imidazol-2-ylmethyl)-piperidin-1-yl]-7-morpholin-4-yl-chinazolin | 8.41 (s, 1 H), 7.80 (d, *J* = 9.3, 1 H), 7.22 (dd, *J* = 9.3, 2.5, 1 H), 7.00 (d, *J* = 9.6, 1H), 6.96 (d, *J* = 2.5, 1H), 6.82 (s, 1 H), 4.16 (t, *J* = 12.4, 2H), 3.80-3.71 (m, 4H), 3.54 (s, 3H), 3.32-3.25 (m, 4H), 3.06 - 2.95 (m, 1 H), 2.83 (dd, *J* = 12.7, 10.6, 1 H), 2.65 - 2.57 (m, 1 H), 2.27 (s, 1 H), 1.94-1.89 (m, 1 H), 1.82 (d, *J* = 13.4, 1H), 1.74 - 1.61 (m, 2H), 1.40 - 1.28 (m, 1 H). (M+H) 393. | < 0.1 |
| 30 | 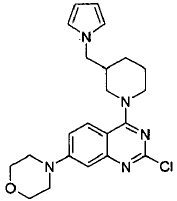 | 2-Chloro-7-morpholin-4-yl-4-(3-pyrrol-1-ylmethyl-piperidin-1-yl)-chinazolin | 7.53 (d, *J* = 9.4, 1 H), 7.12 (dd, *J* = 9.4, 2.6, 1H), 6.90 (d, *J* = 2.5, 1 H), 6.77 (t, *J* = 2.0, 2H), 6.02 (t, *J* = 2.0, 2H), 4.20 (d, *J* = 13.2, 1 H), 3.98 (d, *J* = 12.3, 1H), 3.91 - 3.81 (m, 2H), 3.78 - 3.73 (m, 4H), 3.38 - 3.33 (m, 4H), 3.16 - 3.07 (m, 1 H), 2.96 (dd, *J* = 13.1, 10.4, 1 H), 2.22 - 2.11 (m, 1 H), 1.87 -1.71 (m, 2H), 1.63 (dd, *J* = 24.6, 11.8, 1 H), 1.29 (qd, *J* = 12.4, 4.0, 1H). (M+H) 412. | < 0.1 |
| 31 | 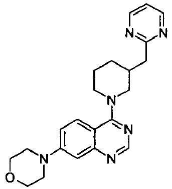 | 7-Morpholin-4-yl-4-(3-pyrimidin-2-ylmethyl-piperidin-1-yl)-chinazolin | 8.77 (d, *J* = 4.9, 2H), 8.41 (s, 1 H), 7.69 (d, *J* = 9.3, 1 H), 7.36 (t, *J* = 4.9, 1 H), 7.21 (dd, *J* = 9.3, 2.6, 1 H), 6.96 (d, *J* = 2.6, 1H), 4.17 - 4.06 (m, 3H), 3.82 - 3.73 (m, 4H), 3.41 - 3.34 (m, 4H), 3.07 - 3.01 (m, 1 H), 2.86 (dt, *J* = 14.3, 7.4, 3H), 1.84 (dd, *J* = 28.1, 13.1, 2H), 1.69 (t, *J* = 12.5, 1H), 1.37 (dd, *J* = 20.1, 11.4, 1 H). (M+H) 391. | < 0.1 |
| 32 | 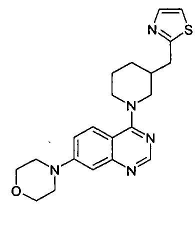 | 7-Morpholin-4-yl-4-(3-thiazol-2-ylmethyl-piperidin-1-yl)-chinazolin | 8.42 (s, 1 H), 7.75 (d, *J* = 3.3, 1 H), 7.69 (d, *J* = 9.3, 1 H), 7.59 (d, *J =* 3.3, 1 H), 7.21 (d, *J* = 6.7, 1 H), 6.97 (s, 1 H), 4.11 (t, *J* = 10.9, 2H), 3.81 - 3.73 (m, 4H), 3.33 - 3.24 (m, 4H), 3.04 (dd, *J* = 26.6, 9.2, 3H), 2.91 - 2.83 (m, 1 H), 2.25 (ddd, *J* = 14.0, 8.7, 5.2, 1 H), 1.91 (d, *J* = 12.7, 1 H), 1.85 - 1.75 (m, 1 H), 1.75 - 1.64 (m, 1H), 1.36 (dt, *J* = 11.3, 6.1, 1 H). (M+H) 396. | < 0.1 |
| 33 | 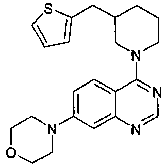 | 7-Morpholin-4-yl-4-(3-thiophen-2-ylmethyl-piperidin-1-yl)-chinazolin | 8.44 (s, 1H), 7.64 (d, *J* = 9.3, 1 H), 7.36 (dd, *J* = 5.1, 1.2, 1 H), 7.18 (dd, *J* = 9.4, 2.6, 1 H), 6.96 (dd, *J* = 5.1, 3.3, 2H), 6.87 (d, *J* = 2.4, 1 H), 4.14 (t, *J* = 9.6, 2H), 3.81 - 3.73 (m, 4H), 3.09 (dd, *J* = 13.8, 8.5, 1 H), 2.93 - 2.76 (m, 4H), 2.06 - 1.87 (m, 3H), 1.81 (d, *J* = 13.3, 1H), 1.65 (dd, *J* = 24.4, 11.8, 1 H), 1.38 - 1.20 (m, 3H). (M+H) 395. | < 0.1 |
| 34 | 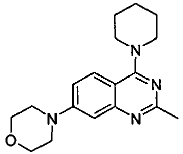 | 2-Methyl-7-morpholin-4-yl-4-piperidin-1-yl-chinazolin | 7.71 (d, *J* = 9.3, 1 H), 7.21 (dd, *J* = 9.3, 2.6, 1 H), 6.92 (d, *J* = 2.6, 1 H), 3.81 - 3.71 (m, 4H), 3.57 (s, 4H), 3.32 - 3.27 (m, 4H), 2.43 (s, 3H), 1.67 (s, 6H). (M+H) 313. | > 1.0 |
| 35 | 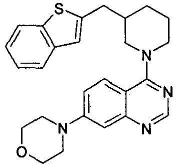 | 4-(3-Benzo[b]thiophen-2-ylmethyl-piperidin-1-yl)-7-morpholin-4-yl-chinazolin | 8.40 (s, 1 H), 7.93 (d, *J* = 7.7, 1 H), 7.73 (d, *J* = 7.2, 1H), 7.51 (d, *J* = 9.3, 1 H), 7.37 - 7.25 (m, 2H), 7.17 (s, 1 H), 6.91 (d, *J* = 2.5, 1 H), 6.81 (dd, *J* = 9.3, 2.6, 1 H), 4.12 (dd, *J =* 19.5, 13.9, 2H), 3.73 (t, *J* = 4.8, 4H), 3.25 - 3.14 (m, 4H), 3.09 - 3.01 (m, 1 H), 2.99 - 2.78 (m, 3H), 2.13 (s, 1 H), 1.98 (d, *J* = 6.7, 1 H), 1.84 (d, *J* = 13.4, 1H), 1.68 (d, *J* = 12.4, 1H), 1.37 (dd, *J* = 21.7, 9.9, 1 H). (M+H) 445. | 0.1-0.5 |
| 36 | 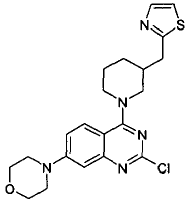 | 2-Chlor-7-morpholin-4-yl-4-(3-thiazol-2-ylmethyl-piperidin-1-yl)-chinazolin | 7.78 (d, *J* = 3.3, 1 H), 7.70 (d, *J =* 9.4, 1 H), 7.62 (d, *J* 3.3, 1 H), 7.17 (dd, *J* = 9.4, 2.6, 1 H), 6.90 (d, *J =* 2.6, 1 H), 4.21 (d, *J* = 12.9, 2H), 3.81 - 3.72 (m, 4H), 3.37 - 3.34 (m, 4H), 3.19 - 3.08 (m, 1 H), 3.06 - 2.93 (m, 3H), 2.26 (ddd, *J* = 10.5, 7.1, 3.5, 1 H), 1.93 (dd, *J* = 12.8, 3.1, 1 H), 1.87 - 1.78 (m, 1 H), 1.72 - 1.60 (m, 1 H), 1.47 - 1.33 (m, 1H). (M+H) 430. | < 0.1 |
| 37 | 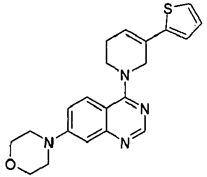 | 7-Morpholin-4-yl-4-(5-thiophen-2-yl-3,6-dihydro-2H-pyridin-1-yl)-chinazolin | 8.48 (d, *J* = 14.5, 1H), 7.91 (d, *J* = 9.3, 1 H), 7.43 (dd, *J* = 5.1, 0.8, 1 H), 7.34 (dd, *J* = 9.4, 2.6, 1 H), 7.14 (d, *J* = 3.0, 1 H), 7.08 - 7.00 (m, 2H), 6.34 (t, *J* = 4.0, 1 H), 4.55 (d, *J* = 1.6, 2H), 3.83 (t, *J* = 5.6, 2H), 3.80 - 3.76 (m, 4H), 3.37 - 3.34 (m, 4H), 2.55 (d, *J* = 3.4, 2H). (M+H) 379. | 0.1-0.5 |
| 38 | 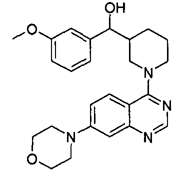 | (3-Methoxy-phenyl)-[1-(7-morpholin-4-yl-chinazolin-4-yl)-piperidin-3-yl]-methanol | Diastereomerengemisch | < 0.1 |
| | | | | |
| | | | (M+H) 435. | |
| 39 | 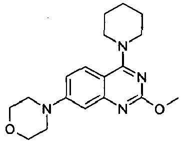 | 2-Methoxy-7-morpholin-4-yl-4-piperidin-1-yl-chinazolin | 7.69 (d, *J* = 9.3, 1 H), 7.08 (dd, *J* = 9.3, 2.6, 1H), 6.82 (d, *J* = 2.5, 1 H), 3.86 (s, 3H), 3.78 - 3.70 (m, 4H), 3.59 (s, 4H), 3.32 - 3.28 (m, 4H), 1.67 (s, 6H). (M+H) 329. | > 1.0 |
| 40 | 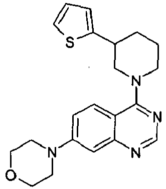 | 7-Morpholin-4-yl-4-(3-thiophen-2-yl-piperidin-1-yl)-chinazolin | 8.48 (s, 1 H), 7.85 (d, *J* = 9.3, 1 H), 7.44 - 7.28 (m, 2H), 7.16 - 6.97 (m, 3H), 4.54 (s, 1H), 4.25 (d, *J* = 12.8, 2H), 3.86 - 3.70 (m, 4H), 3.37 - 3.26 (m, 5H), 3.26 - 3.10 (m, 2H), 2.18 (d, *J* = 8.8, 1H), 1.94 - 1.69 (m, 2H). (M+H) 381. | < 0.1 |
| 41 | 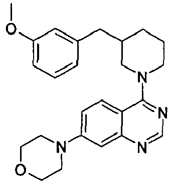 | 4-[3-(3-Methoxybenzyl)-piperidin-1-yl]-7-morpholin-4-yl-chinazolin | 8.40 (s, 1H), 7.56 (d, *J* = 9.3, 1 H), 7.21 (t, *J* = 7.7, 1 H), 7.10 (dd, *J* = 9.3, 2.3, 1 H), 6.95 (d, *J* = 2.3, 1 H), 6.78 (d, *J* = 7.2, 3H), 4.08 (dd, *J =* 9.2, 13.1, 2H), 3.80 - 3.75 (m, 4H), 3.72 (s, 3H), 3.37 - 3.29 (m, 4H), 3.04 (t, *J* = 11.1, 1 H), 2.80 (dd, *J* = 12.7, 10.7, 1 H), 2.62-2.51 (m, 1 H), 2.08 - 1.95 (m, 2H), 1.82 (dd, *J* = 22.1, 13.3, 2H), 1.70 - 1.59 (m, 2H). (M+H) 419. | < 0.1 |
| 42 | 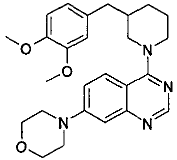 | 4-[3-(3,4-Dimethoxybenzyl)-piperidin-1-yl]-7-morpholin-4-yl-chinazolin | 8.40 (d, *J* = 4.9, 1 H), 8.13 (s, 1H), 7.48 (d, *J* = 9.4, 1 H), 7.03 (dd, *J =* 9.3, 2.6, 1H), 6.93 (d, *J* = 2.5, 1 H), 6.84 (dd, *J* = 13.1, 5.0, 1 H), 6.69 (dd, *J* = 8.1, 1.9, 1H), 4.19 (d, *J =* 13.4, 1H), 4.02 (d, *J* = 12.8, 1H), 3.80 - 3.75 (m, 4H), 3.72 (s, 6H), 3.30.3.25 (m, 4H), 3.09 - 2.99 (m, 1 H), 2,85 - 2.74 (m, 1 H), 2.56 (dd, *J* = 13.4, 6.4, 1 H), 2.43 (dd, *J* = 13.2, 8.3, 1 H), 2.00 (s, 1 H), 1.93 - 1.78 (m, 2H), 1.64 (d, *J* = 12.1, 1 H), 1.36 - 1.24 (m, 1H). (M+H) 449. | 0.1-0.5 |
| 43 | 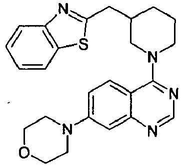 | 4-(3-Benzothiazol-2-ylmethyl-piperidin-1-yl)-7-morpholin-4-yl-chinazolin | 8.41 (s, 1 H), 8.08 (d, *J* = 7.4, 1 H), 7.99 (d, *J* = 7.8, 1 H), 7.59 (d, *J* = 9.0, 1 H), 7.51 (dd, *J* = 8.7, 7.8, 1 H), 7.43 (t, *J* = 7.0, 1 H), 6.98 - 6.87 (m, 2H), 4.14 (d, *J* = 12.8, 2H), 3.74 (t, *J* = 4.8, 4H), 3.20 (t, *J* = 9.2, 4H), 3.18 - 3.00 (m, 3H), 2.92 (dd, *J* = 12.9, 10.5, 1H), 2.39 (dd, *J* = 7.6, 6.6, 1H), 1.99 (dd, *J* = 12.5, 2.5, 1 H), 1.88-1.78 (m, 1H). 1.76 - 1.62 (m, 1 H), 1.50 - 1.35 (m, 1 H). (M+H) 446. | 0.1-0.5 |
| 44 | 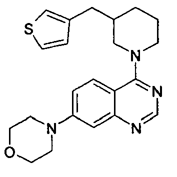 | 7-Morpholin-4-yl-4-(3-thiophen-3-ylmethyl-piperidin-1-yl)-chinazolin | 8.40 (s, 1 H), 7.57 (d, *J* = 9.3, 1 H), 7.52 - 7.42 (m, 1 H), 7.20 - 7.11 (m, 2H), 7.02 (d, *J* = 4.8, 1 H), 6.95 (d, *J* = 2.5, 1 H), 4.13 (d, *J* = 12.9, 1 H), 4.05 *(d, J* = 12.7, 1 H), 3.80 - 3.71 (m, 4H), 3.32 - 3.28 (m, 4H), 3.08 - 2.98 (m, 1 H), 2.83 - 2.73 (m, 1 H), 2.64 - 2.53 (m, 2H), 2.08 -1.98 (m, 1H), 1.87 (dd, *J* = 13.9, 1.7, 1 H), 1.81 (dd, *J* = 9.9, 6.5, 1 H), 1.71 - 1.54 (m, 1 H), 1.34 - 1.21 (m, 1 H). (M+H) 395. | < 0.1 |
| 45 | 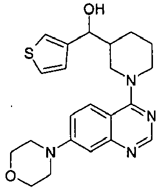 | [1-(7-Morpholin-4-yl-chinazolin-4-yl)-piperidin-3-yl]-thiophen-3-yl-methanol | Diastereomerengemisch | < 0.1 |
| | | | (M+H) 411. | |
| 46 | 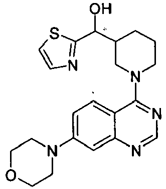 | [1-(7-Morpholin-4-yl-chinazolin-4-yl)-piperidin-3-yl]-thiazol-2-yl-methanol | Diastereomerengemisch | < 0.1 |
| | | | (M+H) 412. | |
| 47 | 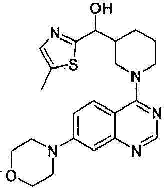 | (5-Methyl-thiazol-2-yl)-[1-(7-morpholin-4-yl-chinazolin-4-yl)-piperidin-3-yl]-methanol | Diastereomerengemisch | < 0.1 |
| | | | (M+H) 426. | |
| 48 | 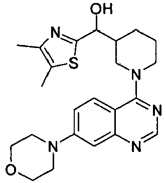 | (4,5-Dimethylthiazol-2-yl)-[1-(7-morpholin-4-yl-chinazolin-4-yl)-piperidin-3-yl]-methanol | Diastereomerengemisch | < 0.1 |
| | | | (M+H) 440. | |
| 49 | 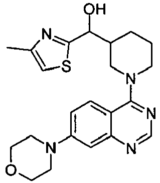 | (4-Methyl-thiazol-2-yl)-[1-(7-morpholin-4-yl-chinazolin-4-yl)-piperidin-3-yl]-methanol | Diastereomerengemisch | < 0.1 |
| | | | (M+H) 426. | |
| 50 | 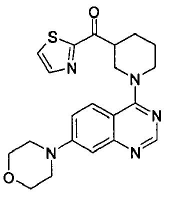 | [1-(7-Morpholin-4-yl-chinazolin-4-yl)-piperidin-3-yl]-thiazol-2-yl-methanon | 8.44 (s, 1 H), 8.24 (t, *J* = 4.6, 2H), 7.94 (d, *J* = 9.3, 1 H), 7.32 (d, *J =* 6.7, 1 H), 7.00 (s, 1 H), 4.42 (d, *J =* 9.8, 1 H), 4.14 (d, *J* = 12.9, 1 H), 3.98 (t, *J* = 8.2, 1 H), 3.82 - 3.71 (m, 4H), 3.41 - 3.33 (m, 4H), 3.24 - 3.09 (m, 1 H), 2.20 (s, 1 H), 1.90 (d, *J* = 7.7, 2H), 1.80 (t, *J* = 9.8, 2H). (M+H) 410. | < 0.1 |
| 51 | 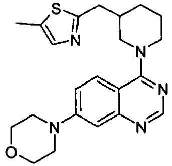 | 4-[3-(5-Methyl-thiazol-2-ylmethyl)-piperidin-1-yl]-7-morpholin-4-yl-chinazolin | 8.42 (s, 1 H), 7.69 (d, *J* = 9.3, 1 H), 7.38 (d, *J* = 0.9, 1 H), 7.20 (dd, *J =* 9.3, 2.5, 1 H), 6.97 (d, *J* = 2.5, 1 H), 4.11 (t, *J* = 12.3, 2H), 3.80 - 3.72 (m, 4H), 3.31 (m, 4H), 3.09 - 3.02 (m, 1 H), 2.94 - 2.84 (m, 3H), 2.40 (s, 3H), 2.19 (ddd, *J* = 10.3, 6.9, 3.5, 1 H), 1.91 (dd, *J* = 9.1, 3.0, 1 H), 1.80 (dd, *J* = 10.0, 3.3, 1H), 1.67 (dd, *J =* 24.6, 11.9, 1 H), 1.34 (ddd, *J* = 15.3, 12.3, 4.0, 1H). (M+H) 410. | < 0.1 |
| 52 | 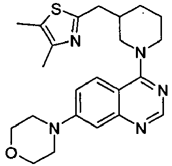 | 4-[3-(4,5-Dimethyl-thiazol-2-ylmethyl)-piperidin-1-yl]-7-morpholin-4-yl-chinazolin | 8.45 - 8.39 (m, 1 H), 7.68 (d, *J* = 9.3, 1 H), 7.20 (dd, *J* = 9.3, 2.6, 1 H), 6.98 (d, *J* = 2.6, 1 H), 4.18 - 4.04 (m, 2H), 3.81 - 3.74 (m, 4H), 3.33 - 3.30 (m, 4H), 3.09 - 3.02 (m, 1 H), 2.92 - 2.77 (m, 3H), 2.28 (s, 3H), 2.20 (s, 3H), 2.15 (dtd, *J* = 13.9, 7.0, 3.4, 1 H), 1.91 (dt, *J* = 9.3, 6.2, 1 H), 1.84 - 1.74 (m, 1 H), 1.66 (ddd, *J* = 15.7, 9.8, 4.0, 1H), 1.37 - 1.27 (m, 1 H). (M+H) 424. | < 0.1 |
| 53 | 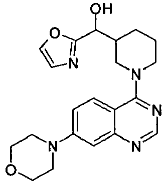 | [1-(7-Morpholin-4-yl-chinazolin-4-yl)-piperidin-3-yl]-oxazol-2-yl-methanol | Diastereomerengemisch | < 0.1 |
| | | | (M+H) 396. | |
| 54 | 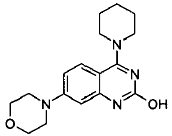 | 7-Morpholin-4-yl-4-piperidin-1-yl-chinazolin-2-ol | 10.52 (s, 1 H), 7.51 (d, *J* = 9.2, 1 H), 6.78 (d, *J* = 6.8, 1 H), 6.50 (s, 1 H), 3.78 - 3.71 (m, 4H), 3.58 (s, 4H), 3.27 - 3.20 (m, 4H), 1.65 (s, 6H). (M+H) 315. | > 1.0 |
| 55 | 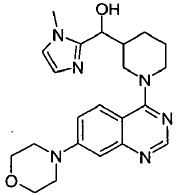 | (1-Methyl-1H-imidazol-2-yl)-[1-(7-morpholin-4-yl-chinazolin-4-yl)-piperidin-3-yl]-methanol | Diastereomerengemisch | 0.1-0.5 |
| | | | (M+H) 409. | |
| 56 | 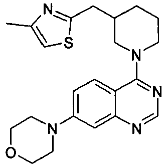 | 4-[3-(4-Methyl-thiazol-2-ylmethyl)-piperidin-1-yl]-7-morpholin-4-yl-chinazolin | 8.42 (s, 1 H), 7.67 (d, *J* = 9.3, 1 H), 7.20 (dd, *J* = 9.3, 2.6, 1 H), 7.09 (d, *J* = 1.0, 1H), 6.97 (d, *J* = 2.5, 1H), 4.12 (dd, *J* = 22.1, 12.3, 2H), 3.80 - 3.72 (m, 4H), 3.33 - 3.29 (m, 4H), 3.06 (t, *J* = 10.9, 1 H), 2.96 - 2.84 (m, 3H), 2.32 (s, 3H), 2.19 (dd, *J* = 10.5, 7.0, 1 H), 1.91 (d, *J* = 12.8, 1H), 1.81 (d, *J* = 13.2, 1 H), 1.69 (t, *J* = 12.5, 1H), 1.41 - 1.29 (m, 1H). (M+H) 410. | < 0.1 |
| 57 | 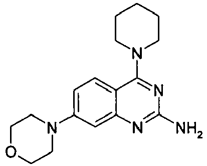 | 7-Morpholin-4-yl-4-piperidin-1-yl-chinazolin-2-ylamin | 7.64 (d, *J* = 9.3, 1 H), 7.19 (s, 2H), 6.96 (d, *J* = 11.1, 1 H), 6.65 (s, 1 H), 3.75 (m, 4H), 3.31 (m, 8H), 1.69 (s, 6H). (M+H) 314. | 0.5-1.0 |
| 58 | 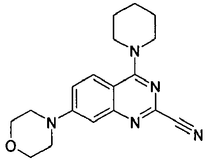 | 7-Morpholin-4-yl-4-piperidin-1-yl-chinazolin-2-carbonitril | 7.82 (d, *J* = 9.4, 1 H), 7.40 (dd, *J =* 8.0, 5.4, 1H), 7.04 (d, *J* = 2.6, 1 H), 3.75 (dd, *J* = 10.7, 5.5, 8H), 3.43 - 3.29 (m, 4H), 1.70 (s, 6H). (M+H) 324. | 0.1-0.5 |
| 59 | 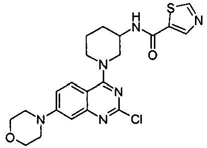 | Thiazol-5-carbonsäure-[1-(2-chloro-7-morpholin-4-yl-chinazolin-4-yl)-piperidin-3-yl]-amid | 9.23 (s, 1 H), 8.70 (d, *J* = 7.2, 1 H), 8.50 (s, 1 H), 7.92 (d, *J* = 9.4, 1 H), 7.27 (dd, *J* = 9.4, 2.5, 1 H), 6.92 (d, *J* = 2.5, 1H), 4.31 (d, *J* = 12.4, 1H), 4.19 (d, *J* = 13.4, 1 H), 3.81 - 3.72 (m, 4H), 3.37 - 3.33 (m, 4H), 3.24 - 3.11 (m, 1 H), 2.07 (s, 2H), 1.91 (s, 2H), 1.83 - 1.65 (m, 2H). (M+H) 459. | < 0.1 |
| 60 | 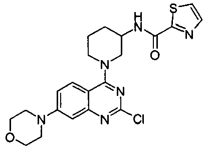 | Thiazol-2-carbonsäure-[1-(2-chlor-7-morpholin-4-yl-chinazolin-4-yl)-piperidin-3-yl]-amid | 8.89 (d, *J* = 7.9, 1 H), 8.03 (dd, *J* = 9.8, 3.1, 2H), 7.90 (d, *J* = 9.4, 1 H), 7.27 (dd, *J* = 9.4, 2.6, 1 H), 6.92 (d, *J* = 2.6, 1 H), 4.21 (dd, *J* = 25.4, 12.4, 2H), 3.78 - 3.73 (m, 4H), 3.37 - 3.33 (m, 4H), 3.23 - 3.13 (m, 1 H), 2.06 - 2.01 (m, 2H), 1.87 (t, *J* = 10.3, 2H), 1.79 -1.71 (m, 2H). (M+H) 459. | < 0.1 |
| 61 | 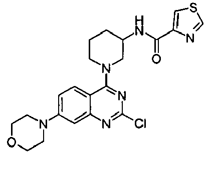 | Thiazol-4-carbonsäure-[1-(2-chlor-7-morpholin-4-yl-chinazolin-4-yl)-piperidin-3-yl]-amid | 9.18 (d, *J* = 2.0, 1 H), 8.46 (d, *J* = 8.0, 1 H), 8.34 (d, *J* = 2.0, 1 H), 7.92 (d, *J* = 9.4, 1H), 7.26 (dd, *J* 9.4, 2.6, 1 H), 6.92 (d, *J* = 2.6, 1 H), 4.29 - 4.08 (m, 2H), 3.78 - 3.71 (m, 4H), 3.39 - 3.34 (m, 4H), 3.27 - 3.12 (m, 3H), 1.99 (s, 1 H), 1.91 - 1.70 (m, 3H). (M+H) 459. | 0.1-0.5 |
| 62 | 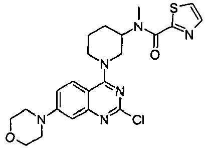 | Thiazol-2-carbonsäure-[1-(2-chlor-7-morpholin-4-yl-chinazolin-4-yl)-piperidin-3-yl]-methyl-amid | (M+H) 474. | 0.5-1.0 |
| 63 | 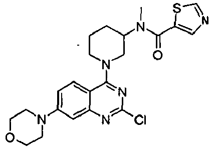 | Thiazol-5-carbonsäure-[1-(2-chlor-7-morpholin-4-yl-chinazolin-4-yl)-piperidin-3-yl]-methyl-amid | (M+H) 474. | 0.1-0.5 |
| 64 | 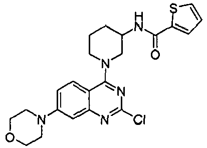 | Thiophen-2-carbonsäure-[1-(2-chlor-7-morpholin-4-yl-chinazolin-4-yl)-piperidin-3-yl]-amid | 8.44 (d, *J* = 7.3, 1 H), 7.93 (d, *J =* 9.4, 1 H), 7.83 - 7.79 (m, 1 H), 7.76 (d, *J* = 5.0, 1 H), 7.26 (dd, *J* = 9.4, 2.6, 1 H), 7.15 (dd, *J* = 4.9, 3.8, 1 H), 6.91 (d, *J* = 2.5, 1 H), 4.31 (d, *J =* 9.5, 1H), 4.20 (d, *J* = 12.8, 1H), 4.03 (dd, *J =* 14.1, 7.0, 1 H), 3.78 - 3.72 (m, 4H), 3.38 - 3.31 (m, 4H), 3.19-3.07 (m, 2H), 2.09 - 2.02 (m, 1 H), 1.95 - 1.85 (m, 1 H), 1.73 (dd, *J* = 17.1, 8.4, 2H). (M+H) 458. | 0.1-0.5 |
| 65 | 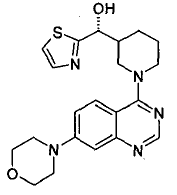 | (R)-[1-(7-Morpholin-4-yl-chinazolin-4-yl)-piperidin-3-yl]-thiazol-2-yl-methanol | 8.42 (s, 1H), 7.75 (d, *J* = 3.2, 1H), 7.70 - 7.61 (m, 2H), 7.21 (dd, *J =* 9.3, 2.4, 1H), 6.97 (d, *J* = 2.4, 1 H), 6.28 (d, *J* = 5.3, 1 H), 4.78 (t, *J* = 5.4, 1H), 4.17 (t, *J* = 15.1, 2H), 3.81 - 3.70 (m, 4H), 3.34 - 3.30 (m, 4H), 3.01 - 2.85 (m, 2H), 2.21 (d, *J* = 5.1, 1 H), 1.79 (d, *J* = 10.4, 2H), 1.68-1.46 (m, 2H). (M+H) 412. | < 0.1 |
| 66 | 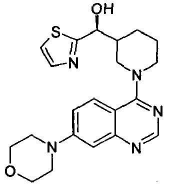 | (S)-[1-(7-Morpholin-4-yl-chinazolin-4-yl)-piperidin-3-yl]-thiazol-2-yl-methanol | 8.43 (d, *J* = 6.9, 1H), 7.85 *(d, J* = 3.2, 1 H), 7.67 (dd, *J* = 8.9, 6.3, 2H), 7.22 (dd, *J* = 9.3, 2.5, 1 H), 6.97 (d, *J* = 2.5, 1 H), 6.35 (d, *J* = 5.0, 1 H), 4.79 (t, *J* = 5.3, 1 H), 4.21 - 4.08 (m, 2H), 3.81 - 3.74 (m, 4H), 3.37 - 3.30 (m, 4H), 3.01 (ddd, *J* = 24.5, 17.4, 11.1, 2H), 2.24 *(d, J* = 5.8, 1H), 1.89 - 1.80 (m, 1 H), 1.76 (d, *J* = 10.0, 1 H), 1.72 - 1.59 (m, 1 H), 1.50 (qd, *J* = 12.2, 4.0, 1H). (M+H) 412. | < 0.1 |
| 67 | 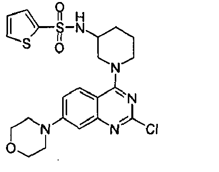 | Thiophen-2-sulfonsäure-[1-(2-chlor-7-morpholin-4-yl-chinazolin-4-yl)-piperidin-3-yl]-amid | 8.20 (d, *J* = 7.0, 1 H), 7.91 (dd, *J* = 5.0, 1.1, 1H), 7.72 (d, *J* = 9.4, 1H), 7.67 (dd, *J* = 3.6, 1.1, 1 H), 7.22 (dd, *J* = 9.4, 2.5, 1 H), 7.16 (dd, *J* = 4.8, 3.9, 1 H), 6.92 (d, *J* = 2.5, 1 H), 4.03 (dd, *J* = 14.2, 7.1, 2H), 3.92 *(d, J* = 13.2, 1H), 3.81-3.72 (m, 4H), 3.40 - 3.35 (m, 4H), 3.22 (t, *J* = 10.8, 1 H), 3.13 (dd, *J* = 12.9, 9.3, 1 H), 1.81 (d, *J* = 9.6, 2H), 1.65 -1.41 (m, 2H). (M+H) 495. | < 0.1 |
| 68 | 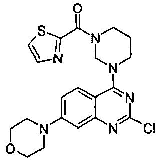 | [3-(2-Chlor-7-morpholin-4-yl-chinazolin-4-yl)-tetrahydro-pyrimidin-1-yl]-thiazol-2-yl-methanon | (M+H) 445 | |
| 69 | 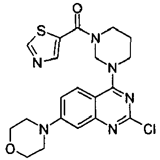 | [3-(2-Chlor-7-morpholin-4-yl-chinazolin-4-yl)-tetrahydro-pyrimidin-1-yl]-thiazol-4-yl-methanon | (M+H) 445 | |
| 70 | 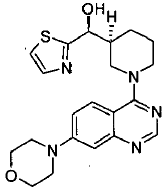 | | ¹H NMR (500 MHz, DMSO) δ 8.42 (s, 1 H), 7.85 (d, *J* = 3.2, 1 H), 7.67 (dd, *J* = 10.5, 6.3, 2H), 7.21 (dd, *J* = 9.3, 2.6, 1 H), 6.97 (d, *J* = 2.5, 1 H), 6.34 (dd, *J* = 26.7, 5.4, 1 H), 4.79 (t, *J* = 5.6, 1 H), 4.13 (t, *J* = 14.5, 2H), 3.81 - 3.75 (m, 4H), 3.34 - 3.28 (m, 4H), 3.11 - 2.87 (m, 2H), 2.30 - 2.16 (m, 1 H), 1.83 (dd, *J* = 9.9, 3.2, 1H), 1.80 - 1.72 (m, 1 H), 1.73 - 1.58 (m, 1 H), 1.50 (qd, *J* = 12.2, 3.9, 1H) | |
| 71 | 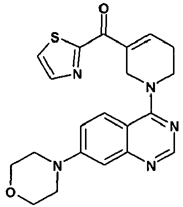 | | M+H) 408 | |
| 72 | 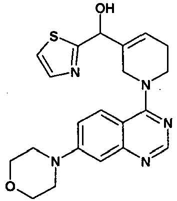 | | (M+H) 410 | |
| 73 | 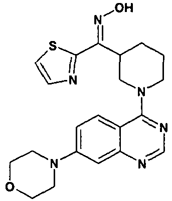 | | (M+H) 426 | |
| 74 | 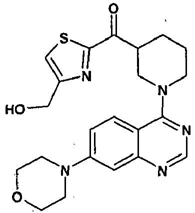 | | (M+H) 441 | |
| 75 | 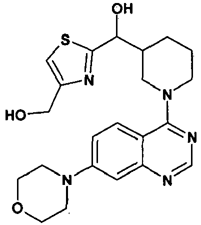 | | (M+H) 443 | |
| 76 | 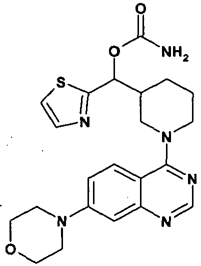 | | (M+H) 456 | |
| 77 | 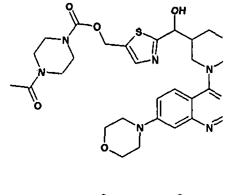 | | (M+H) 597 | |
| 78 | 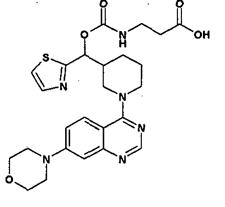 | | (M+H) 528 | |

Des Weiteren können die nachfolgenden Verbindungen analog hergestellt werden.

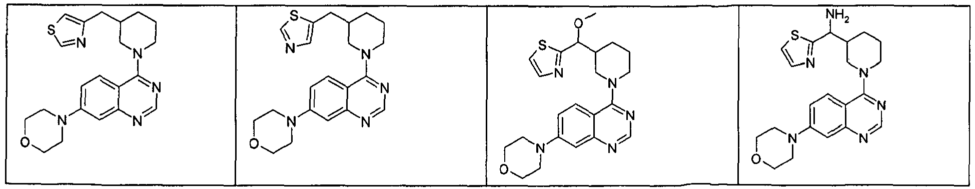

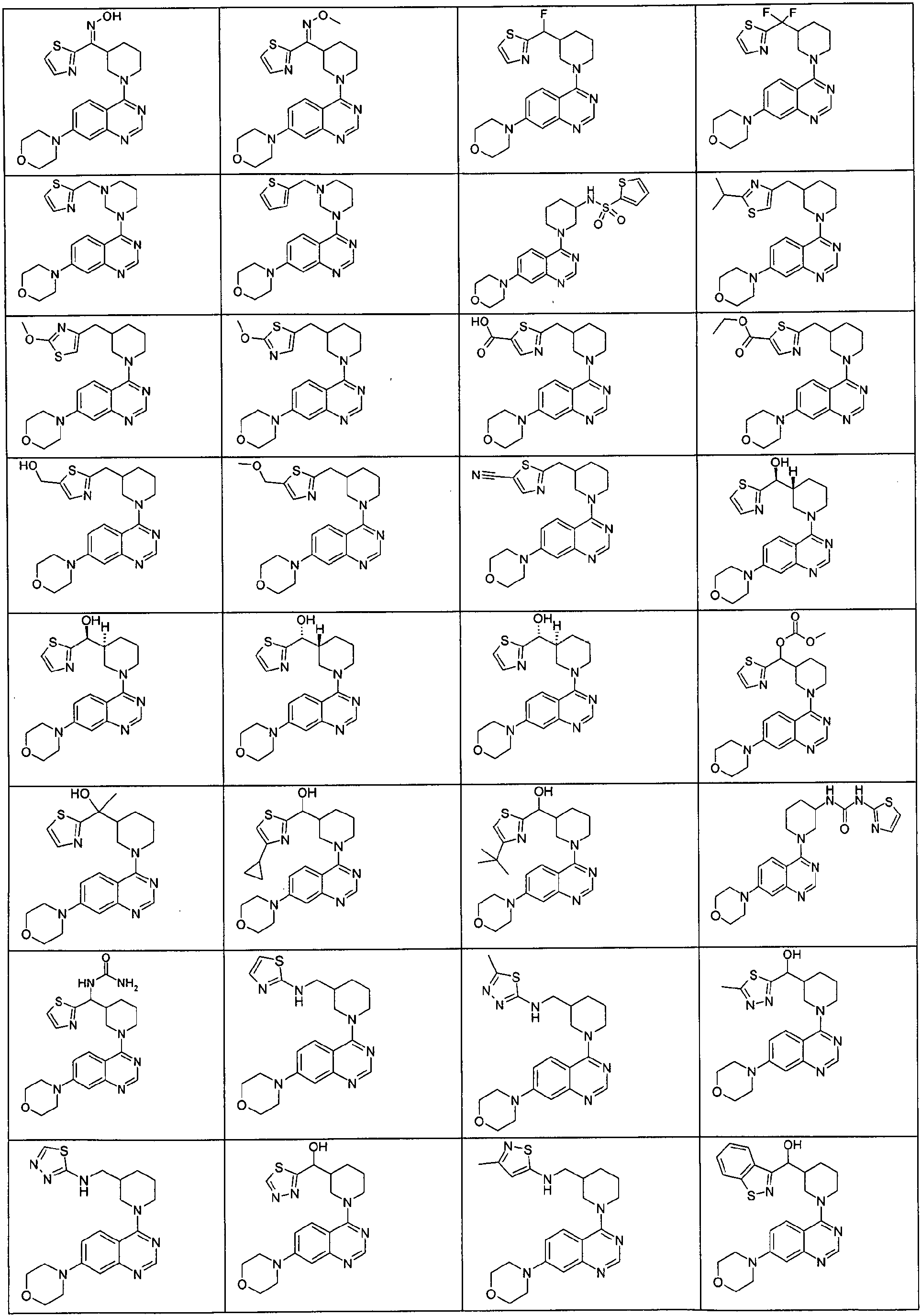

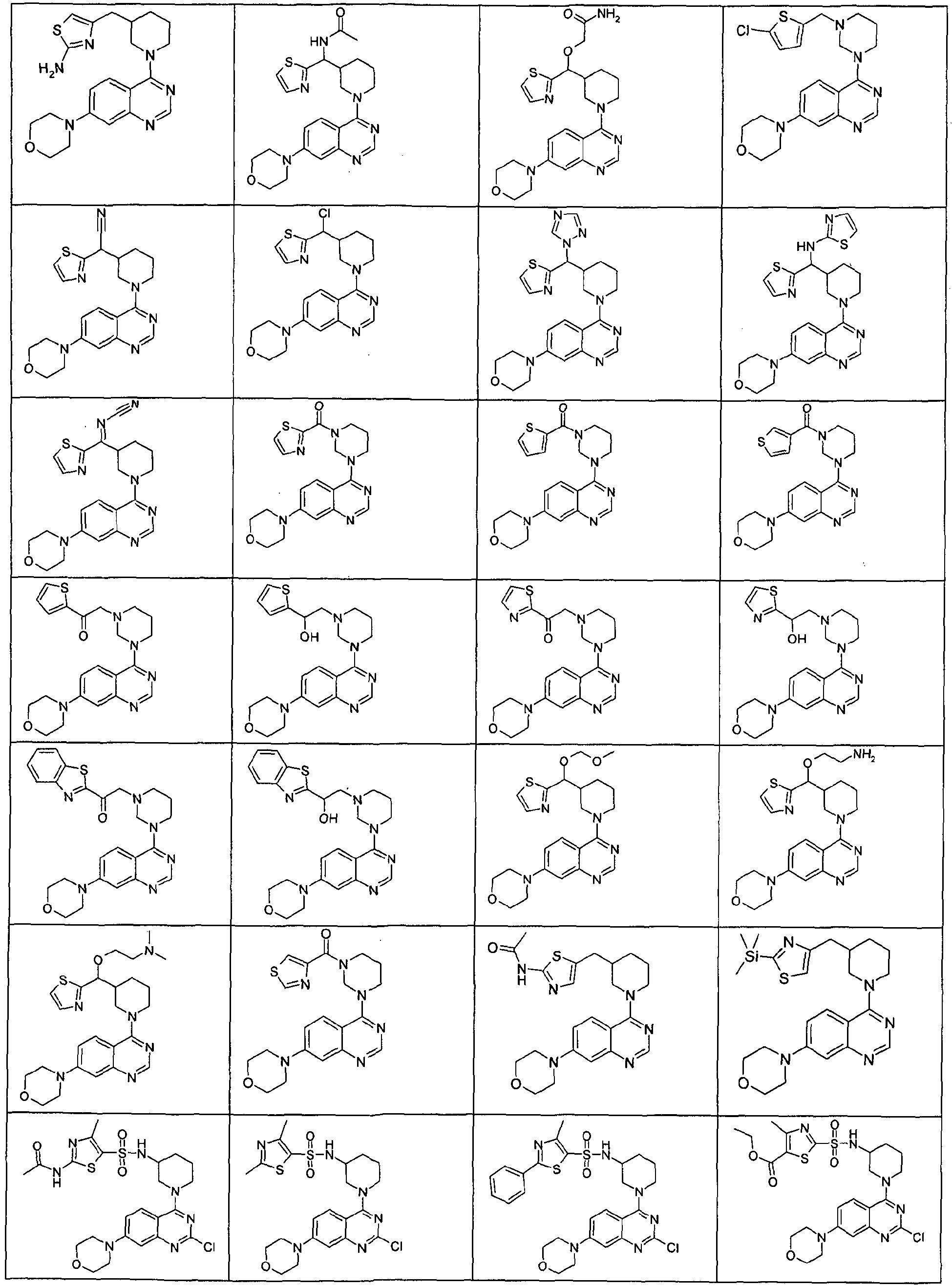

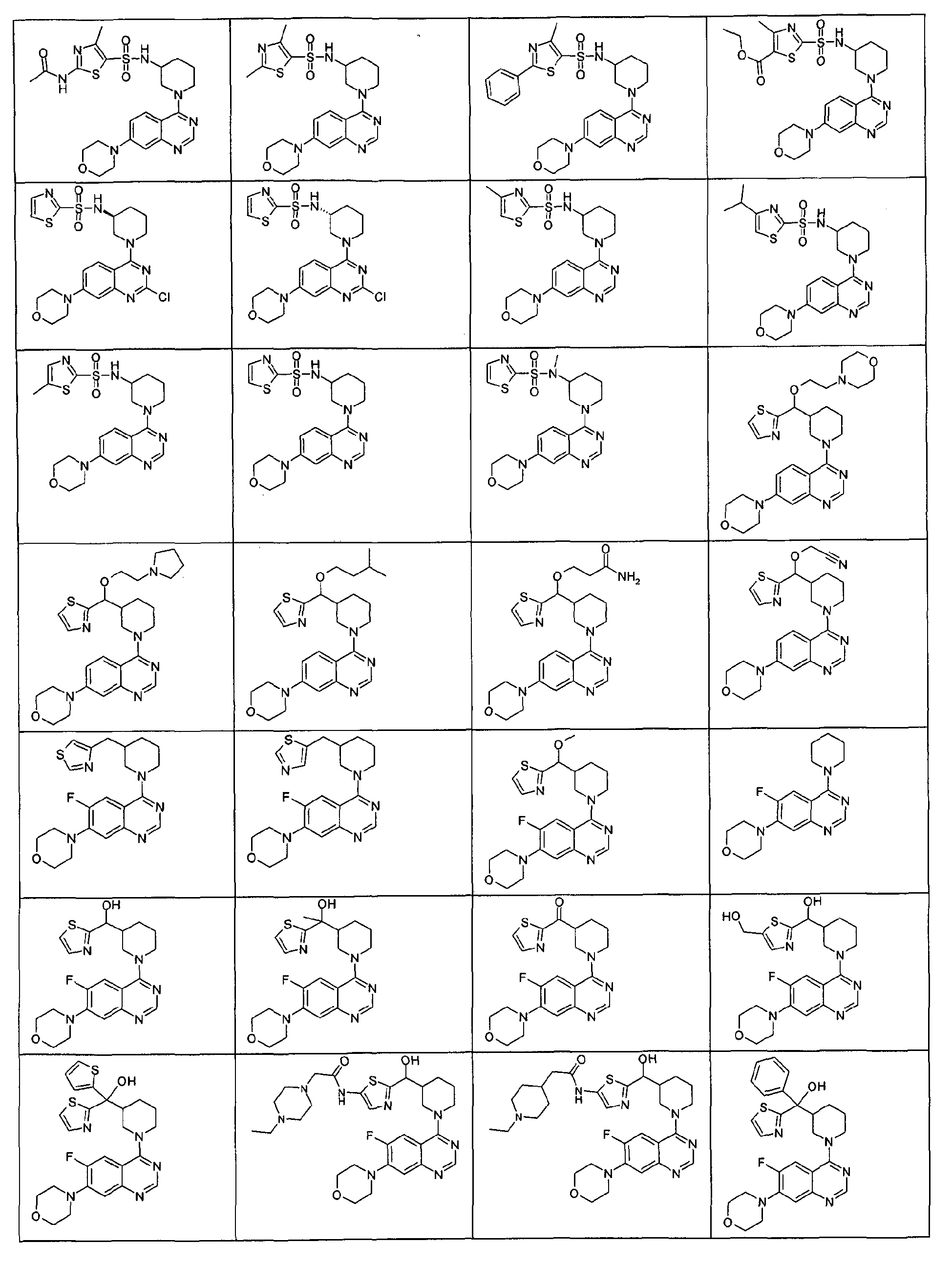

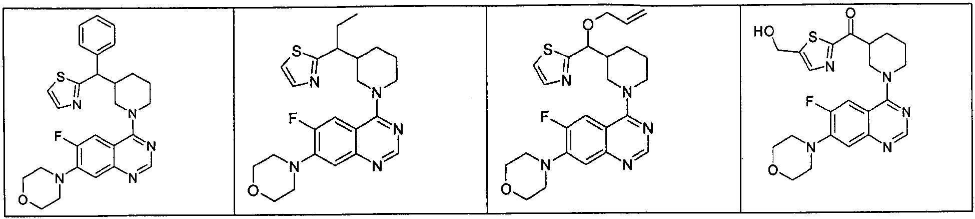

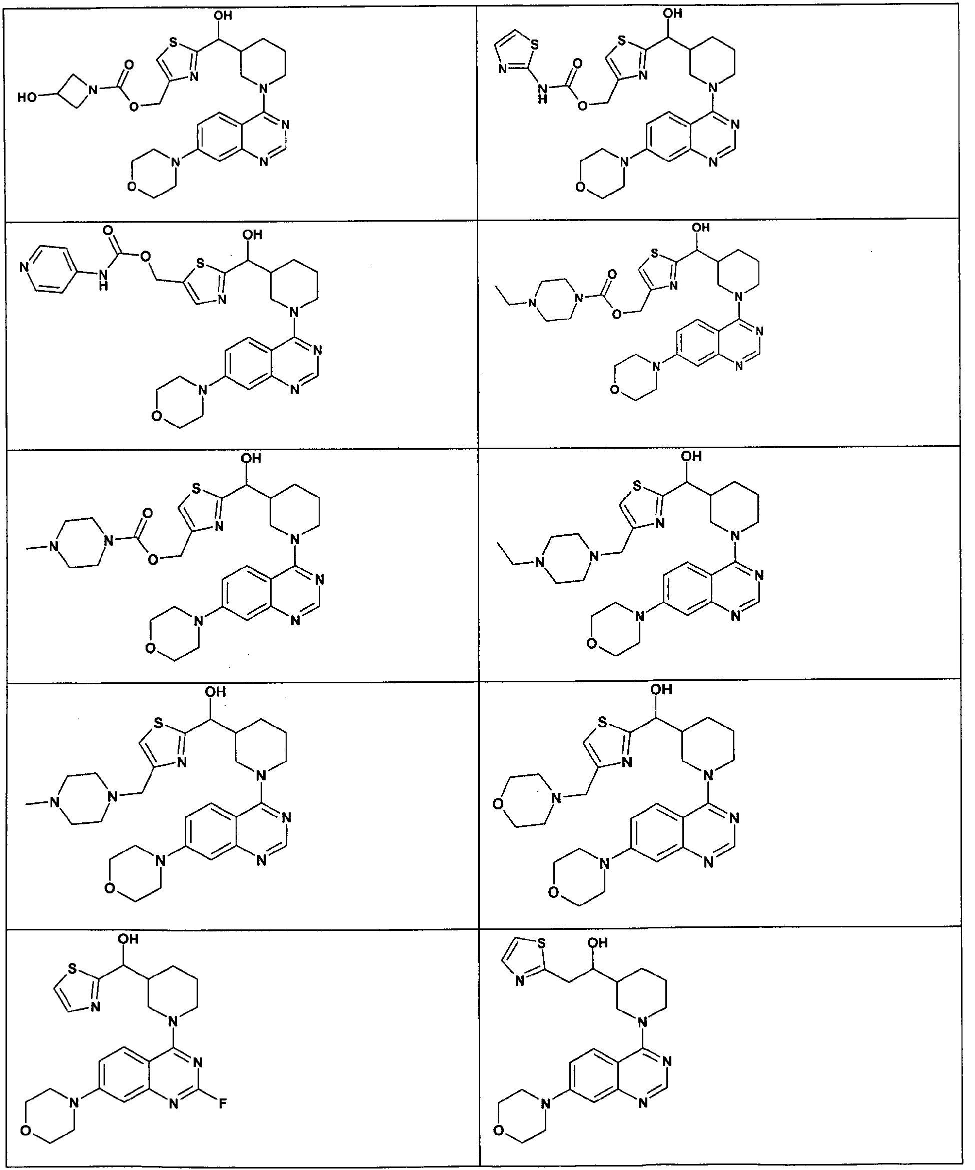

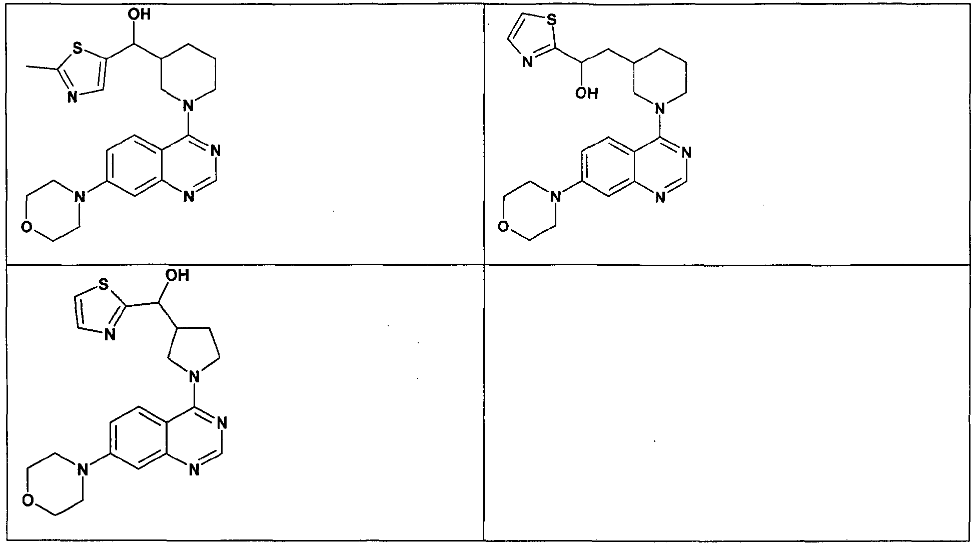

### BEISPIEL 9: DNA-PK / Biochemischer Assay

Der Kinase-Assay erfolgte in mit Streptavidin beschichteten 348-Well-Mikrotiter-FlashPlates. Dazu wurden 1,5 µg DNA-PK-Proteinkomplex und 100 ng biotinyliertes Substrat, wie z.B. PESQEAFADLWKK-Biotin-NH2 ("Biotin-DNA-PK-Peptid"), in einem Gesamtvolumen von 36,5 µ| (34,25 mM HEPES/KOH; 7,85 mM Tris-HCl; 68,5 mM KCl; 5 µM ATP; 6,85 mM MgCl₂; 0,5 mM EDTA; 0,14 mM EGTA; 0,69 mM DTT; pH 7,4) mit 500 ng DNA aus Kälberthymus, 0,1 µCi 33P-ATP und 1,8 % DMSO pro Well mit bzw. ohne die Testverbindung 90 min bei Raumtemperatur inkubiert. Die Reaktion wurde mit 50 µl/Well 200 mM EDTA gestoppt. Nach Inkubieren für weitere 30 min bei Raumtemperatur wurde die Flüssigkeit entfernt. Jedes Well wurde dreimal mit 100 µl 0,9 %-iger Kochsalzlösung gewaschen. Eine unspezifische Reaktion (Leerwert) wurde mit 10 µM eines eigenen Kinase-Inhibitors ermittelt. Die Radioaktivitätsmessung erfolgte mit einem TopCount. IC₅₀-Werte wurden in RS1 berechnet.
Literatur: Kashishian et al. (2003) Molecular Cancer Therapeutics 1257.

### BEISPIEL 10: Pharmazeutische Zubereitungen

### Beispiel A: Iniektionsgläser

Eine Lösung von 100 g erfindungsgemäßem Wirkstoff und 5 g Dinatriumhydrogenphosphat wurde in 3 I zweifach destilliertem Wasser mit 2 N Salzsäure auf pH 6,8 eingestellt, steril filtriert, in Injektionsgläser abgefüllt, unter sterilen Bedingungen lyophilisiert und steril verschlossen. Jedes Injektionsglas enthielt 5 mg erfindungsgemäßen Wirkstoff.

### Beispiel B: Suppositorien

Man schmolz ein Gemisch von 20 g erfindungsgemäßem Wirkstoff mit 100 g Sojalecithin und 1400 g Kakaobutter, goss es in Formen und lies es erkalten. Jedes Suppositorium enthielt 20 mg erfindungsgemäßen Wirkstoff.

### Beispiel C: Lösung

Man bereitete eine Lösung aus 1 g erfindungsgemäßem Wirkstoff, 9,38 g NaH₂PO₄ *2 H₂O, 28,48 g Na₂HPO₄ *12 H₂O und 0,1 g Benzalkoniumchlorid in 940 ml zweifach destilliertem Wasser. Man stellte auf pH 6,8 ein, füllte auf 1 I auf und sterilisierte durch Bestrahlung. Diese Lösung konnte in Form von Augentropfen verwendet werden.

### Beispiel D: Salbe

Man mischte 500 mg erfindungsgemäßen Wirkstoff mit 99,5 g Vaseline unter aseptischen Bedingungen.

### Beispiel E: Tabletten

Ein Gemisch von 1 kg erfindungsgemäßem Wirkstoff, 4 kg Laktose, 1,2 kg Kartoffelstärke, 0,2 kg Talk und 0,1 kg Magnesiumstearat wurde in üblicher Weise zu Tabletten verpresst, derart, dass jede Tablette 10 mg erfindungsgemäßen Wirkstoff enthielt.

### Beispiel F: Dragees

Analog Beispiel E wurden Tabletten gepresst, die danach in üblicher Weise mit einem Überzug aus Saccharose, Kartoffelstärke, Talk, Tragant und Farbstoff überzogen wurden.

### Beispiel G: Kapseln

2 kg erfindungsgemäßer Wirkstoff wurden in üblicher Weise in Hartgelatinekapseln gefüllt, so dass jede Kapsel 20 mg erfindungsgemäßen Wirkstoff enthielt.

### Beispiel H: Ampullen

Eine Lösung von 1 kg erfindungsgemäßem Wirkstoff in 60 I zweifach destilliertem Wasser wurde steril filtriert, in Ampullen abgefüllt, unter sterilen Bedingungen lyophilisiert und steril verschlossen. Jede Ampulle enthielt 10 mg erfindungsgemäßen Wirkstoff.

### Beispiel I: Inhalations-Spray

Man löste 14 g erfindungsgemäßen Wirkstoff in 10 I isotonischer NaCI-Lösung und füllte die Lösung in handelsübliche Sprühgefäße mit Pump-Mechanismus. Die Lösung konnte in Mund oder Nase gesprüht werden. Ein Sprühstoß (etwa 0,1 ml) entsprach einer Dosis von etwa 0,14 mg.

## Patentansprüche

1. Verbindungen der Formel (IA) 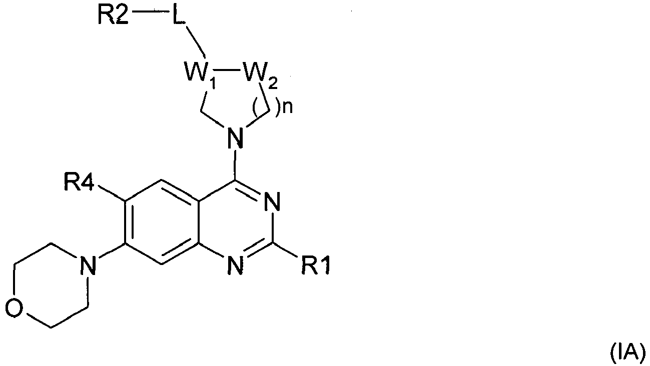 worin
R1 H, Hal, CN, A, OY, NYY oder -NH-C(NYY)=NY,
R2 H, Cyc, Ar, Het¹ oder Het²,
R3 Y, OH oder OA,
R4 H oder Hal,
Y H, A oder Alk-OA,
W₁ CR3 oder N,
W₂ CH₂,
L Einfachbindung, -CYR3-, -CO-, -CO-NY-, -NY-CO-, -NY-CO-NY-, -NY-SO₂-, -C(=NR3)-, -C(=N-CN)-, -Alk-, -Alk-NY-, Alk-CO-, -Alk-CO-NY-, -AlkO-, -Alk-OAlk-, -Alk-C(Y)(OY)-, -C(Y)(CN)-, -C(Y)(Het¹)-, -C(R3)(Het¹)-, -C(Y)(Het¹)-NY-, -C(Y)(Het²)-, -C(Y)(OY)-, -C(Y)(OCOOY)-, -C(Y)(NYY)-, -C(Y)(NY-COY)-, -C(Y)(NY-CO-NYY)-, -C(Y)(OAlk-CN)-, -C(Y)(OAlk-Het²)-, -C(Y)(OAlk-NYY)-, -C(Y)(OAlk-CO-NYY)-, -C(Y)(OY)-Alk-, -C(Y)(OCO-NYY)-, -C(Y)(OCO-NY-Alk-COOY)- oder -C(Y)(OY)-Het¹-Alk-OCO-NY-,
A unverzweigtes oder verzweigtes Alkyl mit 1-10 C-Atomen, wobei unabhängig voneinander 1-7 H-Atome durch Hal ersetzt sein können,
Alk Alkylen mit 1-6 C-Atomen, wobei unabhängig voneinander 1-4 H-Atome durch Hal und/oder CN ersetzt sein können,
Cyc zyklisches Alkyl mit 3-7 C-Atomen, wobei unabhängig voneinander 1-4 H-Atome durch A, Hal und/oder OY ersetzt sein können,
Ar unsubstituiertes oder ein-, zwei- oder dreifach durch Y, OY, Hal, CN, COOY, -Alk-OY, NYY, -NY-COY, SiY₃, Cyc und/oder Phenyl substituiertes Phenyl, Naphthyl oder Biphenyl,
Het¹ ein- oder zweikerniges Heteroaryl mit 2-9 C-Atomen und 1-4 N-, O- und/oder S-Atomen, das unsubstituiert oder ein-, zwei- oder dreifach durch Y, OY, Hal, CN, COOY, -Alk-OY, NYY, -NY-COY, -Alk-Het², -Alk-OCO-Het², -Alk-OCO-NY-Het², -NY-CO-Het², -NY-CO-Alk-Het², SiY₃, Cyc und/oder Phenyl substituiert sein kann, wobei Pyridylmethoxy ausgeschlossen ist, wenn R1 NYY ist,
Het² einen einkernigen gesättigten Heterozyklus mit 2-7 C-Atomen und 1-4 N-, O- und/oder S-Atomen, der unsubstituiert oder einfach durch R3 und/oder COY substituiert sein kann,
Hal F, Cl, Br oder I, und
n 1, 2, 3 oder 4
bedeuten,
und/oder ihre physiologisch unbedenklichen Salze, Tautomere und/oder Stereoisomere, einschließlich deren Mischungen in allen Verhältnissen.

2. Verbindungen nach Anspruch 1, worin
L Einfachbindung, -CYR3-, -CO-, -CO-NY-, -NY-CO-, -NY-SO₂-, -Alk- oder -Alk-OAlk- bedeutet.

3. Verbindungen nach Anspruch 1 mit der Teilformel (IB) 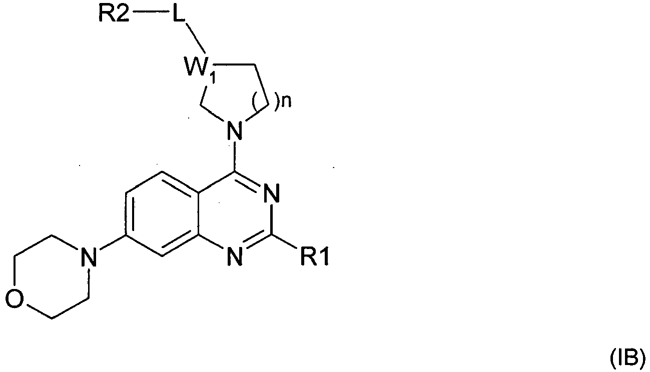 worin
W₁ CH oder N,
R1 H, Hal, CN, A, OA oder NH₂,
R2 H, Ar, Het¹ oder Het²,
R3 A, Alk-OH, OH oder OA,
Y H, A oder Alk-OA,
L Einfachbindung, -CYR3-, -CO-, -CO-NY-, -NY-CO-, -NY-SO₂-, -Alk- oder -Alk-OAlk-,
A unverzweigtes oder verzweigtes Alkyl mit 1-4 C-Atomen, wobei unabhängig voneinander 1-5 H-Atome durch Hal ersetzt sein können,
Alk Alkylen mit 1-3 C-Atomen, wobei 1-2 H-Atome durch Hal ersetzt sein können,
Ar unsubstituiertes oder ein- oder zweifach durch R3 oder Hal substituiertes Phenyl,
Het¹ unsubstituiertes oder ein- oder zweifach durch Y oder Hal substituiertes Heteroaryl aus der Gruppe: 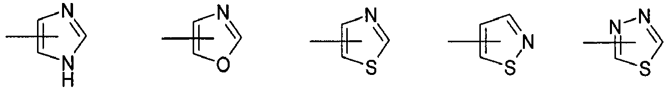 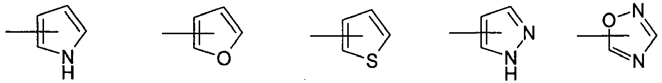 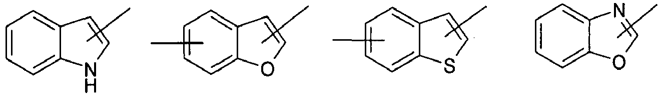 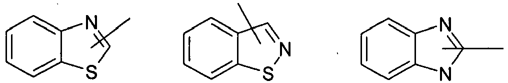 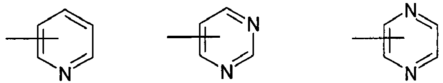
Het² einen unsubstituierten oder einfach durch A substituierten Heterozyklus aus der Gruppe: 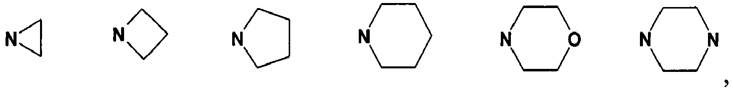
Hal F, Cl oder Br, und
n 1, 2 oder 3
bedeuten,
und/oder ihre physiologisch unbedenklichen Salze, Tautomere und/oder Stereoisomere, einschließlich deren Mischungen in allen Verhältnissen.

4. Verbindungen nach einem der Ansprüche 1 bis 3, ausgewählt aus der Gruppe:
| | |
|---|---|
| 1 | 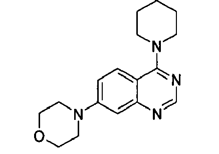 |
| 2 | 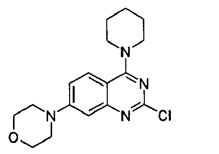 |
| 4 | 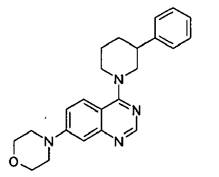 |
| 6 | 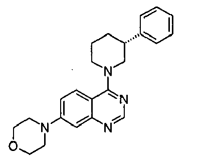 |
| 13 | 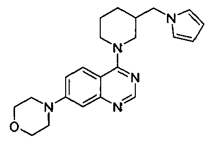 |
| 14 | 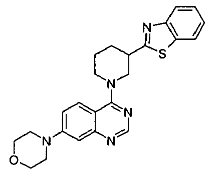 |
| 22 | 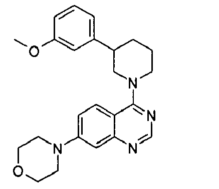 |
| 23 | 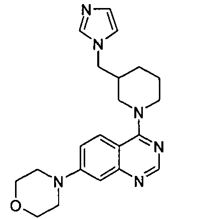 |
| 25 | 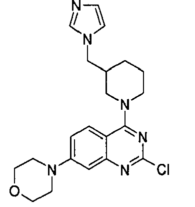 |
| 27 | 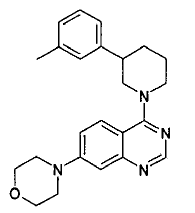 |
| 29 | 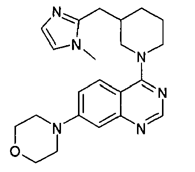 |
| 30 | 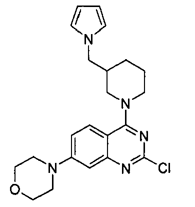 |
| 31 | 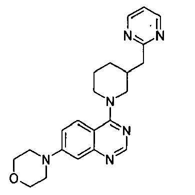 |
| 32 | 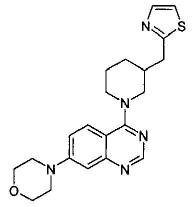 |
| 33 | 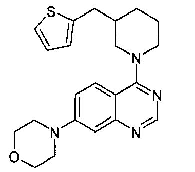 |
| 36 | 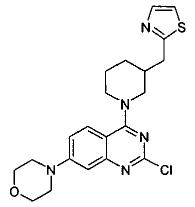 |
| 38 | 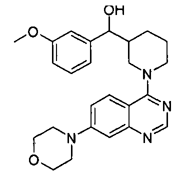 |
| 40 | 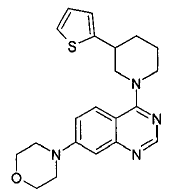 |
| 41 | 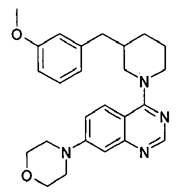 |
| 44 | 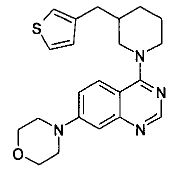 |
| 45 | 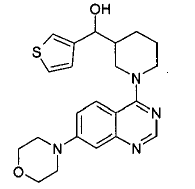 |
| 46 | 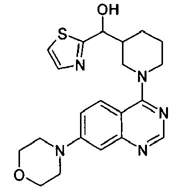 |
| 47 | 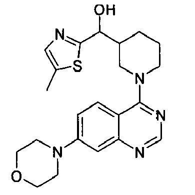 |
| 48 | 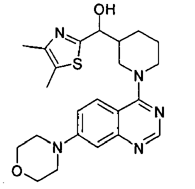 |
| 49 | 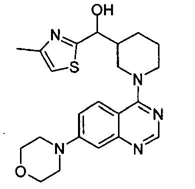 |
| 50 | 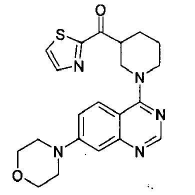 |
| 51 | 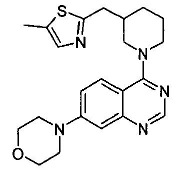 |
| 52 | 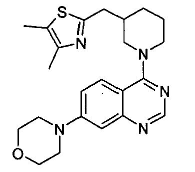 |
| 53 | 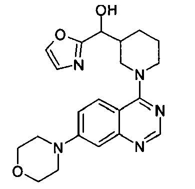 |
| 56 | 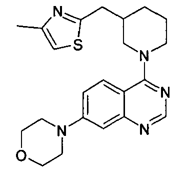 |
| 59 | 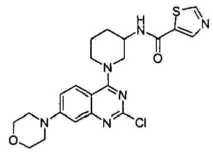 |
| 60 | 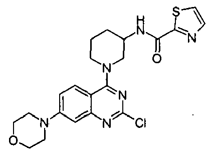 |
| 65 | 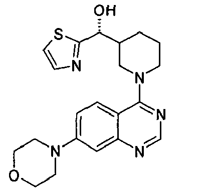 |
| 66 | 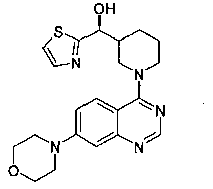 |
| 67 | 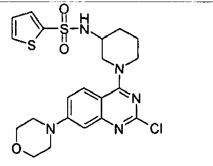 |
| 70 | 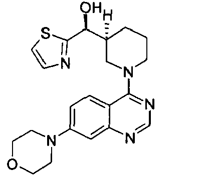 |
und/oder ihre physiologisch unbedenklichen Salze, Tautomere und/oder Stereoisomere, einschließlich deren Mischungen in allen Verhältnissen.

5. Verfahren zum Herstellen von Verbindungen der Formel (IA) nach Anspruch 1 und/oder ihrer physiologisch unbedenklichen Salze, Tautomere und/oder Stereoisomere mit den folgenden Schritten:
(a) Umsetzen einer Verbindung der Formel (II) 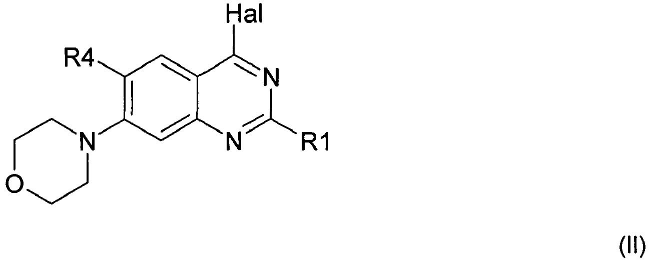 worin R1, R4, Y, A, Alk und Hal die in Anspruch 1 angegebene Bedeutung aufweisen,
mit einer Verbindung der Formel (III) 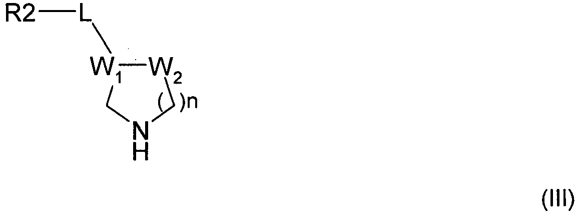 worin R2, R3, Y, W₁, W₂, L, A, Alk, Cyc, Ar, Het¹, Het², Hal und n die in Anspruch 1 angegebene Bedeutung aufweisen,
unter Erhalt der Verbindungen der Formel (IA) 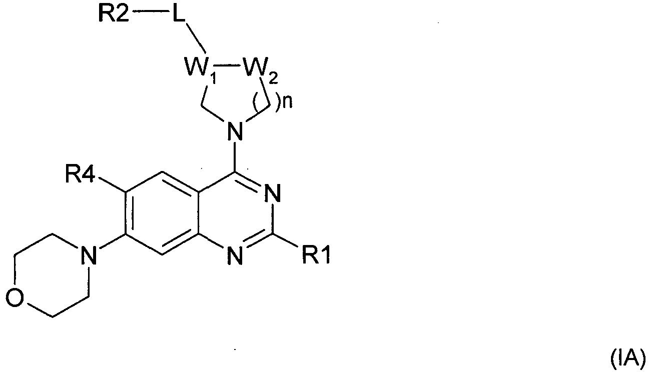 worin R1, R2, R3, R4, Y, W₁, W₂, L, A, Alk, Cyc, Ar, Het¹, Het², Hal und n die in Anspruch 1 angegebene Bedeutung aufweisen,
und gegebenenfalls
(b) Umwandeln einer Base oder Säure der Verbindungen der Formel (IA) in eines ihrer Salze.

6. Zwischenverbindungen der Formel (II), 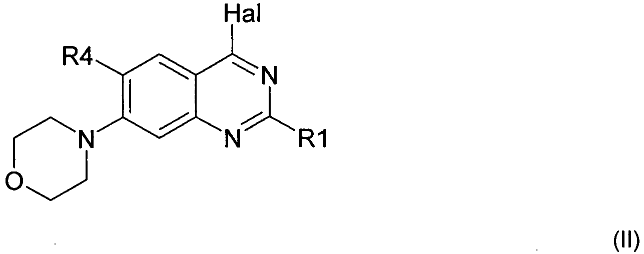 worin
R1 H, CN, A, OY, NYY oder -NH-C(NYY)=NY,
R4 H oder Hal,
Y H, A oder Alk-OA,
A unverzweigtes oder verzweigtes Alkyl mit 1-10 C-Atomen, wobei unabhängig voneinander 1-7 H-Atome durch Hal ersetzt sein können,
Alk Alkylen mit 1-6 C-Atomen, wobei unabhängig voneinander 1-4 H-Atome durch Hal und/oder CN ersetzt sein können, und
Hal F, Cl, Br oder I
bedeuten,
und/oder ihre physiologisch unbedenklichen Salze, Tautomere und Stereoisomere, einschließlich deren Mischungen in allen Verhältnissen.

7. Verfahren zum Herstellen von Zwischenverbindungen der Formel (II) nach Anspruch 6 und/oder ihrer physiologisch unbedenklichen Salze, Tautomere und/oder Stereoisomere mit den folgenden Schritten:
(a) Umsetzen einer Verbindung der Formel (IIC) 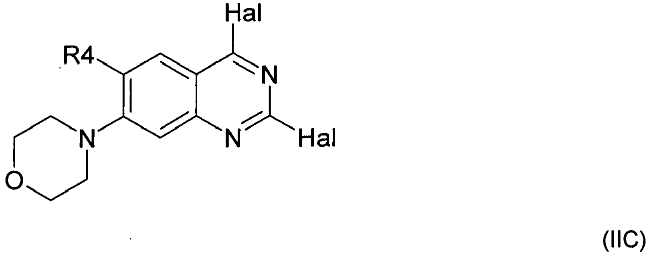 worin R4 und Hal die in Anspruch 6 angegebene Bedeutung aufweisen,
mit einer Verbindung X-R1, worin
X ein Element der 1. Hauptgruppe bedeutet, und
R1, Y, A, Alk und Hal die in Anspruch 6 angegebene Bedeutung aufweisen, wobei H₂ für X-R1 ausgeschlossen ist,
unter Erhalt der Zwischenverbindungen der Formel (II) 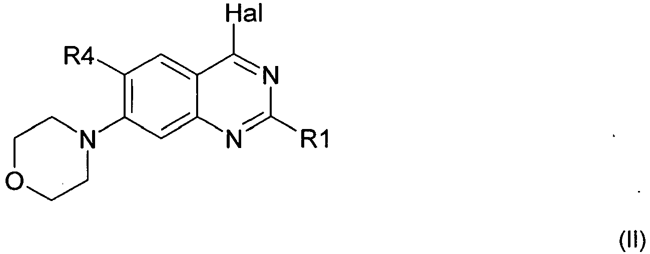 worin R1, R4, Y, A, Alk und Hal die in Anspruch 6 angegebene Bedeutung aufweisen,
und gegebenenfalls
(b) Umwandeln einer Base oder Säure der Verbindungen der Formel (II) in eines ihrer Salze.

8. Verfahren zum Herstellen von Zwischenverbindungen der Formel (II) nach Anspruch 6 und/oder ihrer physiologisch unbedenklichen Salze, Tautomere und/oder Stereoisomere mit den folgenden Schritten:
(a) Umsetzen einer Verbindung der Formel (IIE) 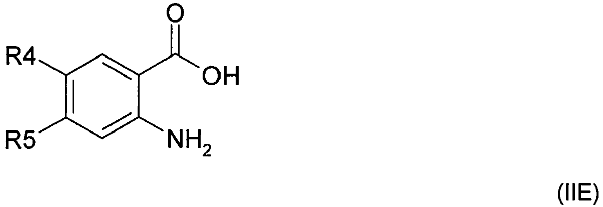 worin
R4 H oder Hal,
R5 Morpholinyl oder Hal, und
Hal F, Cl, Br oder I
bedeuten,
mit Formamidinacetat in einem Alkohol unter Erhalt der Zwischenverbindungen der Formel (IID-1) 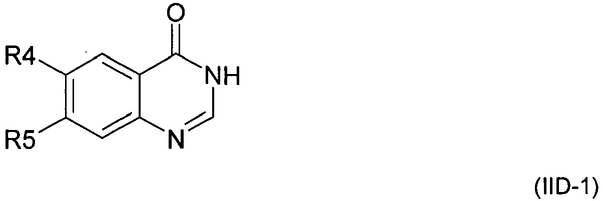 worin
R4 H oder Hal,
R5 Morpholinyl oder Hal, und
Hal F, Cl, Br oder I
bedeuten,
und wenn R5 Hal ist
(b) Umsetzen der Zwischenverbindungen der Formel (IID-1) mit Morpholin unter Erhalt der Zwischenverbindungen der Formel (IID) 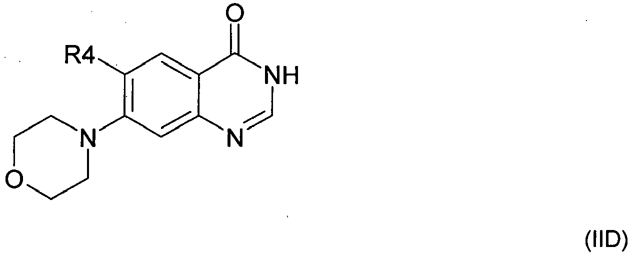 worin
R4 H oder Hal, und
Hal F, Cl, Br oder I
bedeuten,
(b') Umsetzen der Zwischenverbindungen der Formel (IID) mit einem Halogenierungsmittel in einem organischen Amin unter Erhalt von Zwischenverbindungen der Teilformel (II) 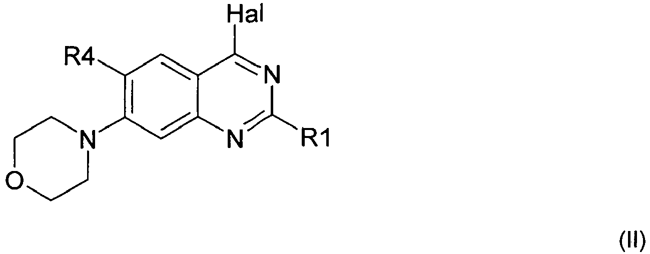 worin
R1 H,
R4 H oder Hal, und
Hal F, Cl, Br oder I
bedeuten, und gegebenenfalls
(b") Umwandeln einer Base oder Säure der Zwischenverbindungen der Formel (II) in eines ihrer Salze.

9. Verwendung von Verbindungen nach einem der Ansprüche 1 bis 4 und/oder ihrer physiologisch unbedenklichen Salze, Tautomere und/oder Stereoisomere, einschließlich deren Mischungen in allen Verhältnissen, zur Hemmung von Serin-Threonin-Proteinkinasen in-vitro.

10. Verwendung von mindestens einer Verbindung nach einem der Ansprüche 1 bis 4 und/oder ihrer physiologisch unbedenklichen Salze, Tautomere und/oder Stereoisomere, einschließlich deren Mischungen in allen Verhältnissen, zur Sensibilisierung von Krebszellen gegenüber Antikrebsmittel und/oder ionisierender Strahlung unter der Maßgabe, dass die Sensibilisierung in-vivo nicht am menschlichen und/oder tierischen Körper erfolgt.

11. Arzneimittel umfassend mindestens eine Verbindung nach einem der Ansprüche 1 bis 4 und/oder ihre physiologisch unbedenklichen Salze, Tautomere und/oder Stereoisomere, einschließlich deren Mischungen in allen Verhältnissen.

12. Pharmazeutisches Zusammensetzung umfassend als Wirkstoff eine wirksame Menge von mindestens einer Verbindung nach einem der Ansprüche 1 bis 4 und/oder ihrer physiologisch unbedenklichen Salze, Tautomere und/oder Stereoisomere, einschließlich deren Mischungen in allen Verhältnissen, zusammen mit pharmazeutisch verträglichen Hilfsstoffen, in Kombination mit mindestens einem Antikrebsmittel.

13. Verbindungen nach einem der Ansprüche 1 bis 4 und/oder ihre physiologisch unbedenklichen Salze, Tautomere und/oder Stereoisomere, einschließlich deren Mischungen in allen Verhältnissen, zur Verwendung in der Prophylaxe, Therapie und/oder Verlaufskontrolle von Krebs, Tumoren, Metastasen und/oder Störungen der Angiogenese, in Kombination mit Radiotherapie und/oder mit mindestens einem Antikrebsmittel.

## Claims

1. Compounds of the formula (IA) 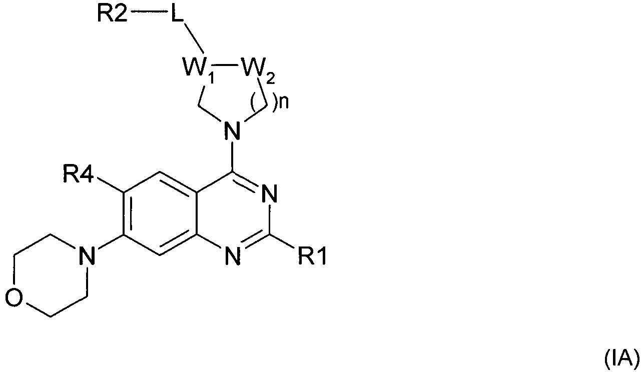 in which
R1 denotes H, Hal, CN, A, OY, NYY or -NH-C(NYY)=NY,
R2 denotes H, Cyc, Ar, Het¹ or Het²,
R3 denotes Y, OH or OA,
R4 denotes H or Hal,
Y denotes H, A or Alk-OA,
W₁ denotes CR3 or N,
W₂ denotes CH₂,
L denotes a single bond, -CYR3-, -CO-, -CO-NY-, -NY-CO-, -NY-CO-NY-, -NY-SO₂-, -C(=NR3)-, -C(=N-CN)-, -Alk-, -Alk-NY-, Alk-CO-, -Alk-CO-NY-, -AlkO-, -Alk-OAlk-, -Alk-C(Y)(OY)-, -C(Y)(CN)-, -C(Y)(Het¹)-, -C(R3)(Het¹)-, -C(Y)(Het¹)-NY-, -C(Y)(Het²)-, -C(Y)(OY)-, -C(Y)(OCOOY)-, -C(Y)(NYY)-, -C(Y)(NY-COY)-, -C(Y)(NY-CO-NYY)-, -C(Y)(OAlk-CN)-, -C(Y)(OAlk-Het²)-, -C(Y)(OAlk-NYY)-, -C(Y)(OAlk-CO-NYY)-, -C(Y)(OY)-Alk-, -C(Y)(OCO-NYY)-, -C(Y)(OCO-NY-Alk-COOY)- or -C(Y)(OY)-Het¹-Alk-OCO-NY-,
A denotes unbranched or branched alkyl having 1-10 C atoms, where 1-7 H atoms may be replaced, independently of one another, by Hal,
Alk denotes alkylene having 1-6 C atoms, where 1-4 H atoms may be replaced, independently of one another, by Hal and/or CN,
Cyc denotes cyclic alkyl having 3-7 C atoms, where 1-4 H atoms may be replaced, independently of one another, by A, Hal and/or OY,
Ar denotes phenyl, naphthyl or biphenyl, each of which is unsubstituted or mono-, di- or trisubstituted by Y, OY, Hal, CN, COOY, -Alk-OY, NYY, -NY-COY, SiY₃, Cyc and/or phenyl,
Het¹ denotes mono- or bicyclic heteroaryl having 2-9 C atoms and 1-4 N, O and/or S atoms, which may be unsubstituted or mono-, di- or trisubstituted by Y, OY, Hal, CN, COOY, -Alk-OY, NYY, -NY-COY, -Alk-Het², -Alk-OCO-Het², -Alk-OCO-NY-Het², -NY-CO-Het², -NY-CO-Alk-Het², SiY₃, Cyc and/or phenyl, where pyridyl-methoxy is excluded if R1 is NYY,
Het² denotes a monocyclic saturated heterocycle having 2-7 C atoms and 1-4 N, O and/or S atoms, which may be unsubstituted or monosubstituted by R3 and/or COY,
Hal denotes F, Cl, Br or I, and
n denotes 1, 2, 3 or 4,
and/or physiologically acceptable salts, tautomers and/or stereoisomers thereof, including mixtures thereof in all ratios.

2. Compounds according to Claim 1 in which
L denotes a single bond, -CYR3-, -CO-, -CO-NY-, -NY-CO-, -NY-SO₂-, -Alk- or -Alk-OAlk-.

3. Compounds according to Claim 1 having the sub-formula (IB) 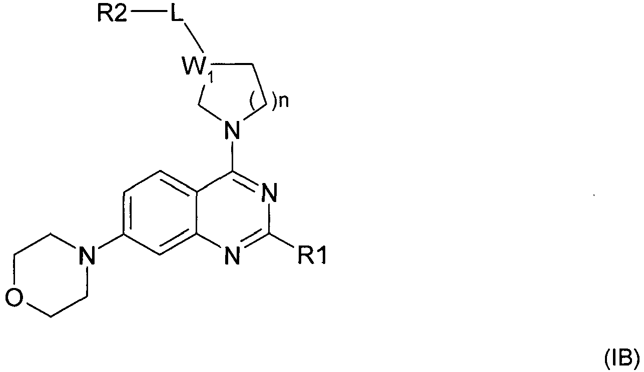 in which
W₁ denotes CH or N,
R1 denotes H, Hal, CN, A, OA or NH₂,
R2 denotes H, Ar, Het¹ or Het²,
R3 denotes A, Alk-OH, OH or OA,
Y denotes H, A or Alk-OA,
L denotes a single bond, -CYR3-, -CO-, -CO-NY-, -NY-CO-, -NY-SO₂-, -Alk- or -Alk-OAlk-,
A denotes unbranched or branched alkyl having 1-4 C atoms, where 1-5 H atoms may be replaced, independently of one another, by Hal,
Alk denotes alkylene having 1-3 C atoms, where 1-2 H atoms may be replaced by Hal,
Ar denotes phenyl which is unsubstituted or mono- or disubstituted by R3 or Hal,
Het¹ denotes heteroaryl which is unsubstituted or mono- or disubstituted by Y or Hal, from the group: 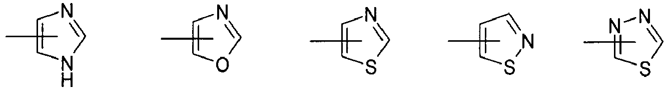 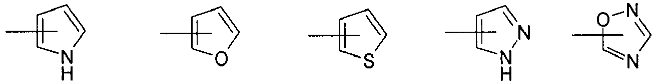 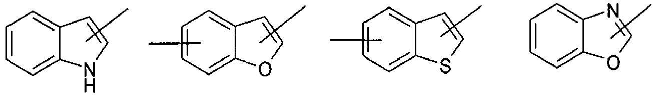 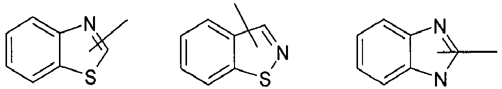 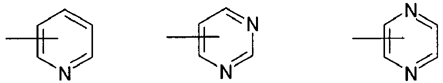
Het² denotes a heterocycle which is unsubstituted or monosubstituted by A, from the group: 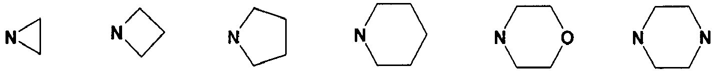
Hal denotes F, Cl or Br, and
n denotes 1, 2 or 3,
and/or physiologically acceptable salts, tautomers and/or stereoisomers thereof, including mixtures thereof in all ratios.

4. Compounds according to one of Claims 1 to 3, selected from the group:
| | |
|---|---|
| 1 | 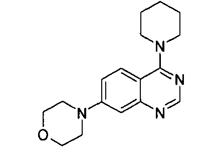 |
| 2 | 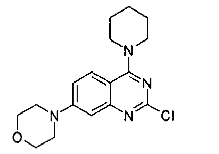 |
| 4 | 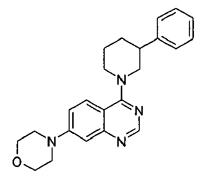 |
| 6 | 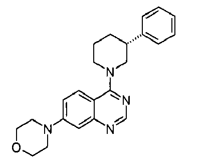 |
| 13 | 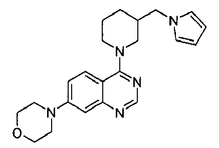 |
| 14 | 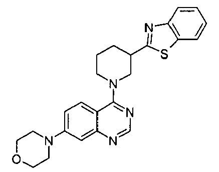 |
| 22 | 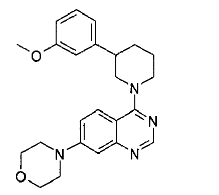 |
| 23 | 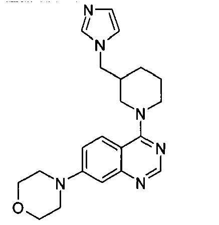 |
| 25 | 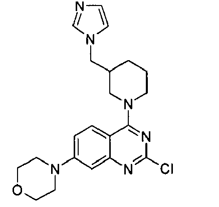 |
| 27 | 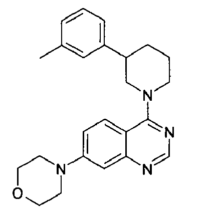 |
| 29 | 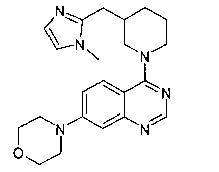 |
| 30 | 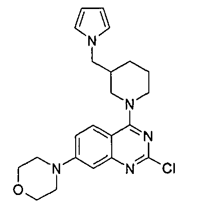 |
| 31 | 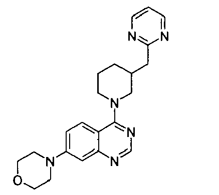 |
| 32 | 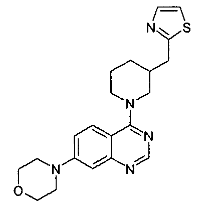 |
| 33 | 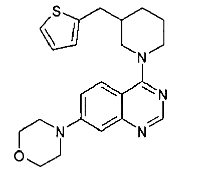 |
| 36 | 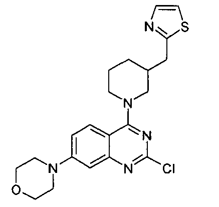 |
| 38 | 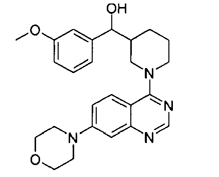 |
| 40 | 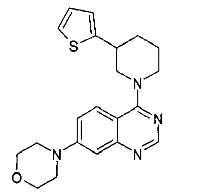 |
| 41 | 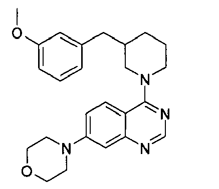 |
| 44 | 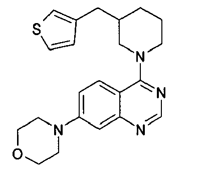 |
| 45 | 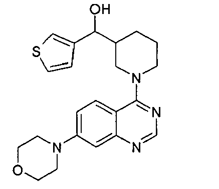 |
| 46 | 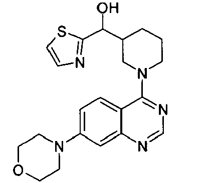 |
| 47 | 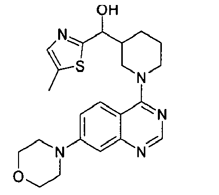 |
| 48 | 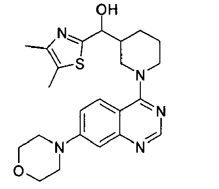 |
| 49 | 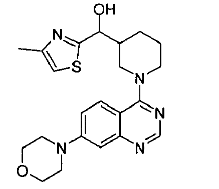 |
| 50 | 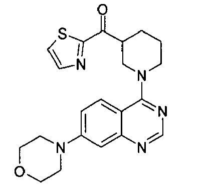 |
| 51 | 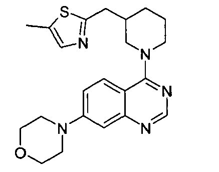 |
| 52 | 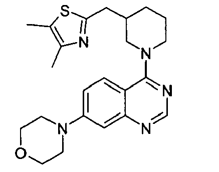 |
| 53 | 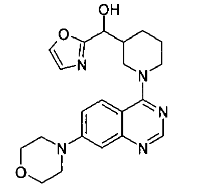 |
| 56 | 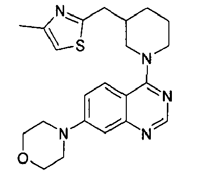 |
| 59 | 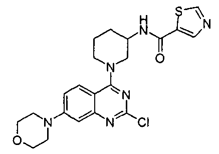 |
| 60 | 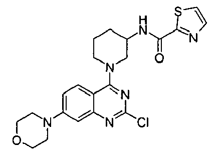 |
| 65 | 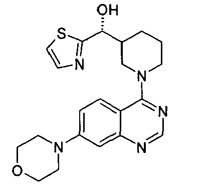 |
| 66 | 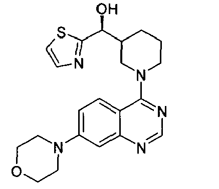 |
| 67 | 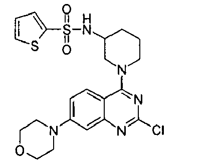 |
| 70 | 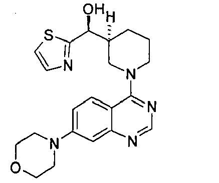 |
and/or physiologically acceptable salts, tautomers and/or stereoisomers thereof, including mixtures thereof in all ratios.

5. Process for the preparation of compounds of the formula (IA) according to Claim 1 and/or physiologically acceptable salts, tautomers and/or stereoisomers thereof having the following steps:
(a) reaction of a compound of the formula (II) 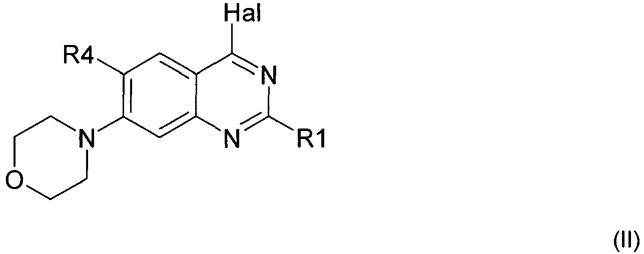 in which R1, R4, Y, A, Alk and Hal have the meaning indicated in Claim 1,
with a compound of the formula (III) 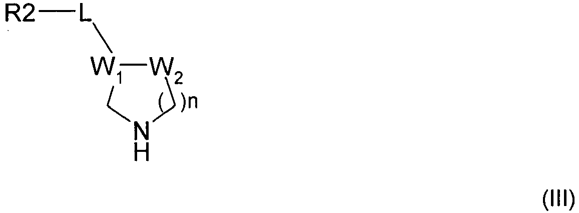 in which R2, R3, Y, W₁, W₂, L, A, Alk, Cyc, Ar, Het¹, Het², Hal and n have the meaning indicated in Claim 1,
to give the compounds of the formula (IA) 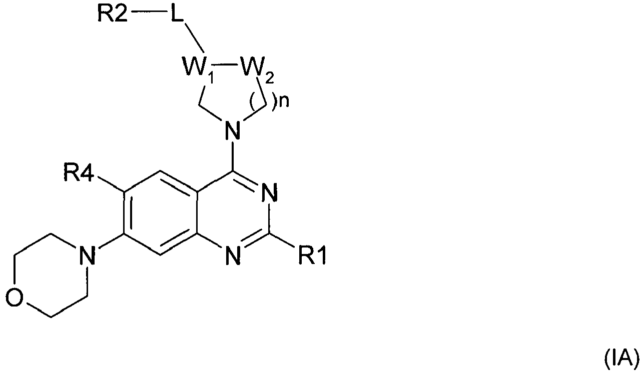 in which R1, R2, R3, R4, Y, W₁, W₂, L, A, Alk, Cyc, Ar, Het¹, Het², Hal and n have the meaning indicated in Claim 1, and optionally
(b) conversion of a base or acid of the compounds of the formula (IA) into one of their salts.

6. Intermediate compounds of the formula (II) 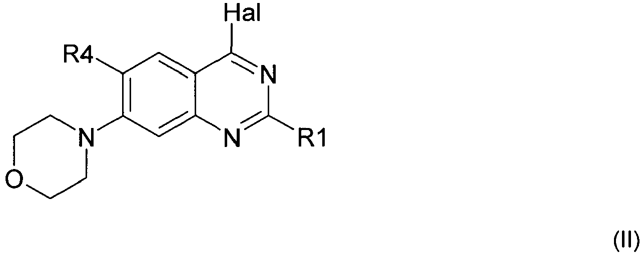 in which
R1 denotes H, CN, A, OY, NYY or -NH-C(NYY)=NY,
R4 denotes H or Hal,
Y denotes H, A or Alk-OA,
A denotes unbranched or branched alkyl having 1-10 C atoms, where 1-7 H atoms may be replaced, independently of one another, by Hal,
Alk denotes alkylene having 1-6 C atoms, where 1-4 H atoms may be replaced, independently of one another, by Hal and/or CN, and
Hal denotes F, Cl, Br or I,
and/or physiologically acceptable salts, tautomers and stereoisomers thereof, including mixtures thereof in all ratios.

7. Process for the preparation of intermediate compounds of the formula (II) according to Claim 6 and/or physiologically acceptable salts, tautomers and/or stereoisomers thereof having the following steps:
(a) reaction of a compound of the formula (IIC) 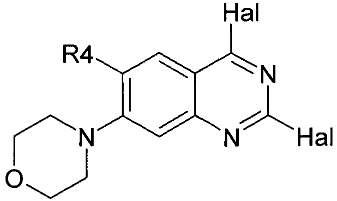 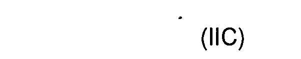 in which R4 and Hal have the meaning indicated in Claim 6,
with a compound X-R1, in which
X denotes an element from the 1st main group, and
R1, Y, A, Alk and Hal have the meaning indicated in Claim 6, where H₂ for X-R1 is excluded,
to give the intermediate compounds of the formula (II) 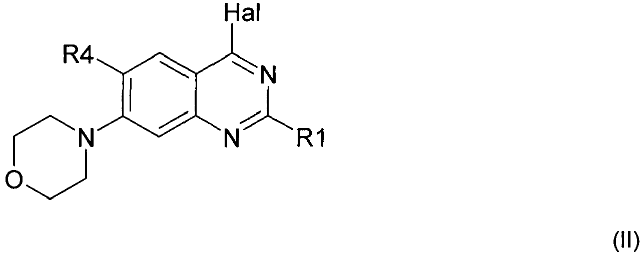 in which R1, R4, Y, A, Alk and Hal have the meaning indicated in Claim 6,
and optionally
(b) conversion of a base or acid of the compounds of the formula (II) into one of their salts.

8. Process for the preparation of intermediate compounds of the formula (II) according to Claim 6 and/or physiologically acceptable salts, tautomers and/or stereoisomers thereof having the following steps:
(a) reaction of a compound of the formula (IIE) 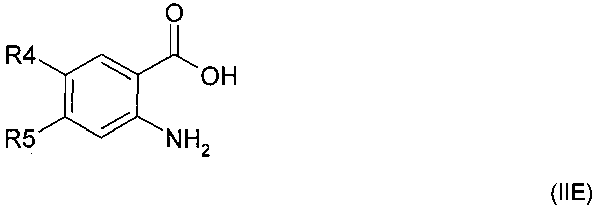 in which
R4 denotes H or Hal,
R5 denotes morpholinyl or Hal, and
Hal denotes F, Cl, Br or I,
with formamidine acetate in an alcohol to give the intermediate compounds of the formula (IID-1) 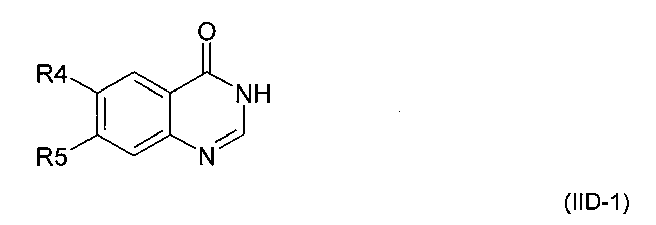 in which
R4 denotes H or Hal,
R5 denotes morpholinyl or Hal, and
Hal denotes F, Cl, Br or I,
and, if R5 is Hal,
(b) reaction of the intermediate compounds of the formula (IID-1) with morpholine to give the intermediate compounds of the formula (IID) 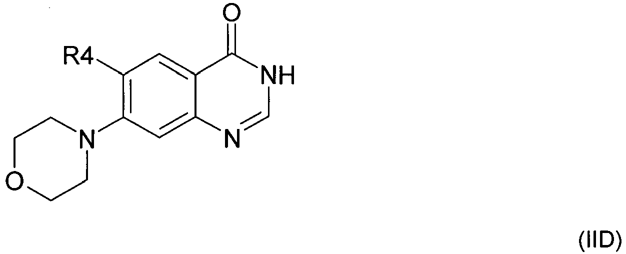 in which
R4 denotes H or Hal, and
Hal denotes F, Cl, Br or I,
(b') reaction of the intermediate compounds of the formula (IID) with a halogenating agent in an organic amine to give intermediate compounds of the sub-formula (II) 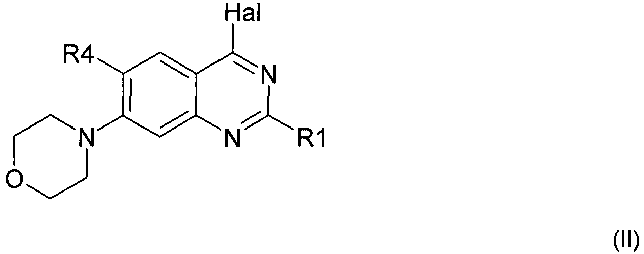 in which
R1 denotes H,
R4 denotes H or Hal, and
Hal denotes F, Cl, Br or I,
and optionally
(b") conversion of a base or acid of the intermediate compounds of the formula (II) into one of their salts.

9. Use of compounds according to one of Claims 1 to 4 and/or physiologically acceptable salts, tautomers and/or stereoisomers thereof, including mixtures thereof in all ratios, for the inhibition of serine/threonine protein kinases in vitro.

10. Use of at least one compound according to one of Claims 1 to 4 and/or physiologically acceptable salts, tautomers and/or stereoisomers thereof, including mixtures thereof in all ratios, for the sensitisation of cancer cells to anticancer agents and/or ionising radiation, with the proviso that the sensitisation does not take place in vivo on the human and/or animal body.

11. Medicament comprising at least one compound according to one of Claims 1 to 4 and/or physiologically acceptable salts, tautomers and/or stereoisomers thereof, including mixtures thereof in all ratios.

12. Pharmaceutical composition comprising, as active compound, an effective amount of at least one compound according to one of Claims 1 to 4 and/or physiologically acceptable salts, tautomers and/or stereoisomers thereof, including mixtures thereof in all ratios, together with pharmaceutically tolerated assistants, in combination with at least one anticancer agent.

13. Compounds according to one of Claims 1 to 4 and/or physiologically acceptable salts, tautomers and/or stereoisomers thereof, including mixtures thereof in all ratios, for use in the prophylaxis, therapy and/or progress control of cancer, tumours, metastases and/or angiogenesis disorders, in combination with radiotherapy and/or with at least one anticancer agent.

## Revendications

1. Composés de la formule (IA): 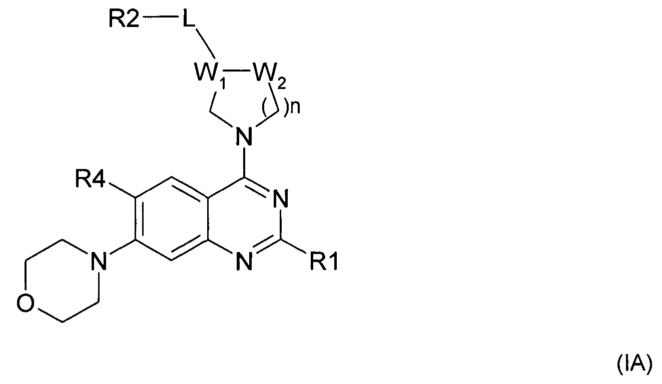 dans laquelle :
R1 représente H, Hal, CN, A, OY, NYY ou -NH-C(NYY)=NY,
R2 représente H, Cyc, Ar, Het¹ ou Het²,
R3 représente Y, OH ou OA,
R4 représente H ou Hal,
Y représente H, A ou Alk-OA,
W₁ représente CR3 ou N,
W₂ représente CH₂,
L représente une liaison simple, -CYR3-, -CO-, -CO-NY-, -NY-CO-, -NY-CO-NY-, -NY-SO₂-, -C(=NR3)-, -C(=N-CN)-, -Alk-, -Alk-NY-, Alk-CO-, -Alk-CO-NY-, -AlkO-, -Alk-OAlk-, -Alk-C(Y)(OY)-, -C(Y)(CN)-, -C(Y)(Het¹)-, -C(R3)(Het¹)-, -C(Y)(Het¹)-NY-, -C(Y)(Het²)-, -C(Y)(OY)-, -C(Y)(OCOOY)-, -C(Y)(NYY)-, -C(Y)(NY-COY)-, -C(Y)(NY-CO-NYY)-, -C(Y)(OAlk-CN)-, -C(Y)(OAIk-Het²)-, -C(Y)(OAlk-NYY)-, -C(Y)(OAlk-CO-NYY)-, -C(Y)(OY)-Alk-, -C(Y)(OCO-NYY)-, -C(Y)(OCO-NY-Alk-COOY)- ou -C(Y)(OY)-Het¹-Alk-OCO-NY-,
A représente alkyle non ramifié ou ramifié comportant 1-10 atome(s) de C, où 1-7 atome(s) de H peut/peuvent être remplacé(s), indépendamment l'un de l'autre ou les uns des autres, par Hal,
Alk représente alkylène comportant 1-6 atome(s) de C, où 1-4 atome(s) de H peut/ peuvent être remplacé(s), indépendamment l'un de l'autre ou les uns des autres, par Hal et/ou CN,
Cyc représente alkyle cyclique comportant 3-7 atomes de C, où 1-4 atome(s) de H peut/peuvent être remplacé(s), indépendamment l'un de l'autre ou les uns des autres, par A, Hal et/ou OY,
Ar représente phényle, naphtyle ou biphényle, dont chacun est non substitué ou monosubstitué, disubstitué ou trisubstitué par Y, OY, Hal, CN, COOY, -Alk-OY, NYY, -NY-COY, SiY₃, Cyc et/ou phényle,
Het¹ représente hétéroaryle monocyclique ou bicyclique comportant 2-9 atomes de C et 1-4 atome(s) de N, de O et/ou de S, lequel peut être non substitué ou monosubstitué, disubstitué ou trisubstitué par Y, OY, Hal, CN, COOY, -Alk-OY, NYY, -NY-COY, -Alk-Het², -Alk-OCO-Het², -Alk-OCO-NY-Het², -NY-CO-Het², -NY-CO-Alk-Het², SiY₃, Cyc et/ou phényle, où pyridylméthoxy est exclu si R1 est NYY,
Het² représente un hétérocycle saturé monocyclique comportant 2-7 atomes de C et 1-4 atome(s) de N, de O et/ou de S, lequel peut être non substitué ou mono-substitué par R3 et/ou COY,
Hal représente F, CI, Br ou I, et
n représente 1, 2, 3 ou 4,
et/ou leurs sels, tautomères et/ou stéréo-isomères physiologiquement acceptables, y compris leurs mélanges selon tous rapports.

2. Composés selon la revendication 1 dans lesquels :
L représente une liaison simple, -CYR3-, -CO-, -CO-NY-, -NY-CO-, -NY-SO₂-, -Alk- ou -Alk-OAlk-.

3. Composés selon la revendication 1 présentant la sous-formule (IB) : 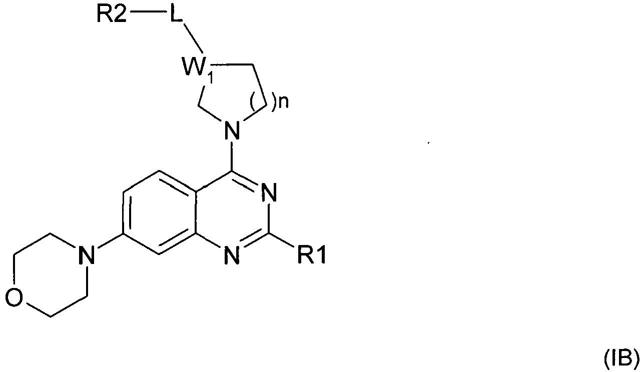 dans laquelle :
W₁ représente CH ou N,
R1 représente H, Hal, CN, A, OA ou NH₂,
R2 représente H, Ar, Het¹ ou Het²,
R3 représente A, Alk-OH, OH ou OA,
Y représente H, A ou Alk-OA,
L représente une liaison simple, -CYR3-, -CO-, -CO-NY-, -NY-CO-, -NY-SO₂-, -Alk- ou -Alk-OAlk-,
A représente alkyle non ramifié ou ramifié comportant 1-4 atome(s) de C, où 1-5 atome(s) de H peut/peuvent être remplacé(s), indépendamment l'un de l'autre ou les uns des autres, par Hal,
Alk représente alkylène comportant 1-3 atome(s) de C, où 1-2 atome(s) de H peut/peuvent être remplacé(s) par Hal,
Ar représente phényle qui est non substitué ou monosubstitué ou disubstitué par R3 ou Hal,
Het¹ représente hétéroaryle qui est non substitué ou monosubstitué ou disubstitué par Y ou Hal, parmi le groupe : 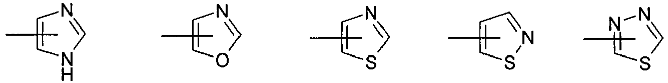 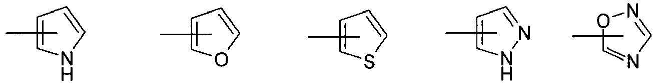 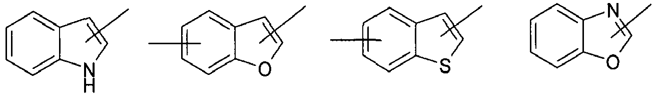 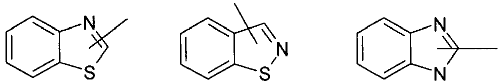 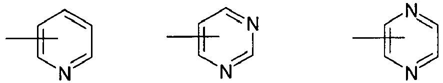
Het² représente un hétérocycle qui est non substitué ou monosubstitué par A, parmi le groupe : 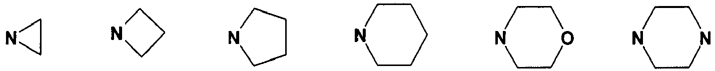
Hal représente F, Cl ou Br, et
n représente 1, 2 ou 3,
et/ou leurs sels, tautomères et/ou stéréo-isomères physiologiquement acceptables, y compris leurs mélanges selon tous rapports.

4. Composés selon une ou plusieurs des revendications 1 à 3, choisis parmi le groupe :
| | |
|---|---|
| 1 | 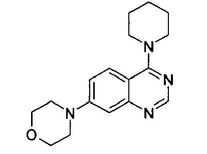 |
| 2 | 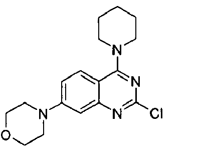 |
| 4 | 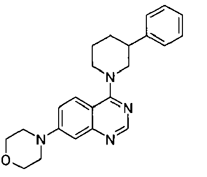 |
| 6 | 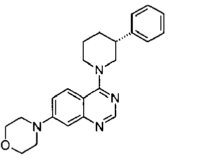 |
| 13 | 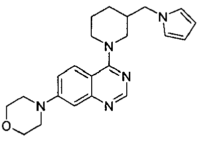 |
| 14 | 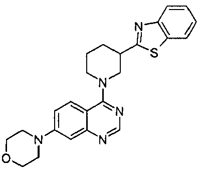 |
| 22 | 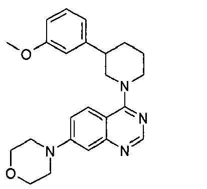 |
| 23 | 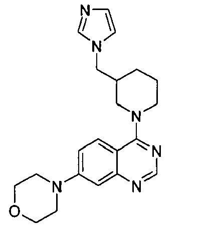 |
| 25 | 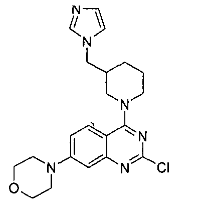 |
| 27 | 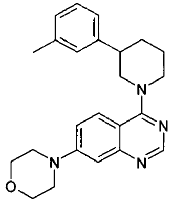 |
| 29 | 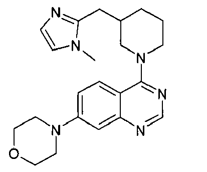 |
| 30 | 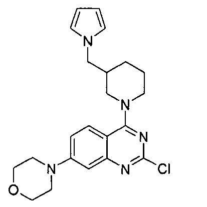 |
| 31 | 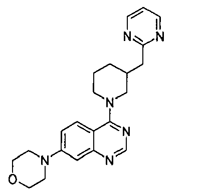 |
| 32 | 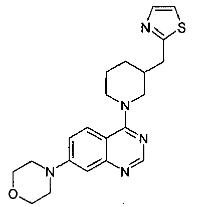 |
| 33 | 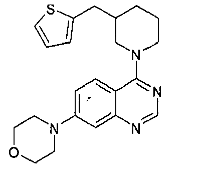 |
| 36 | 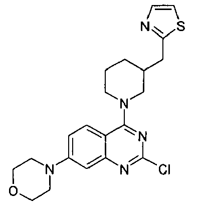 |
| 38 | 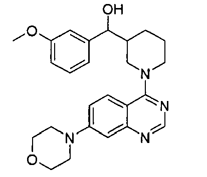 |
| 40 | 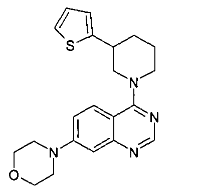 |
| 41 | 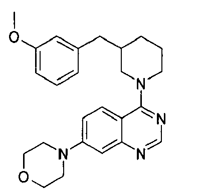 |
| 44 | 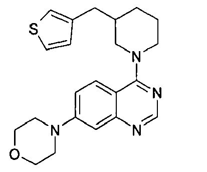 |
| 45 | 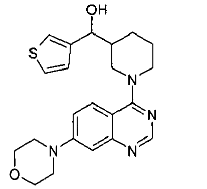 |
| 46 | 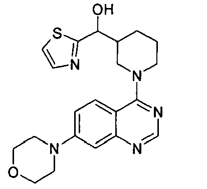 |
| 47 | 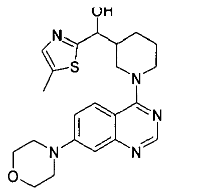 |
| 48 | 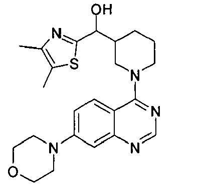 |
| 49 | 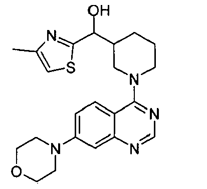 |
| 50 | 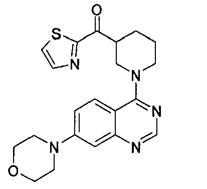 |
| 51 | 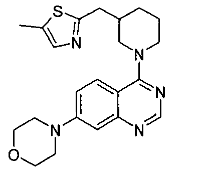 |
| 52 | 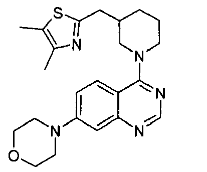 |
| 53 | 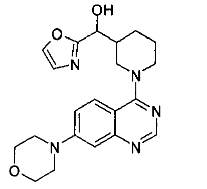 |
| 56 | 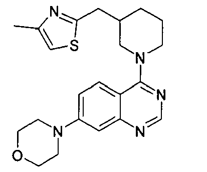 |
| 59 | 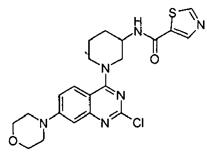 |
| 60 | 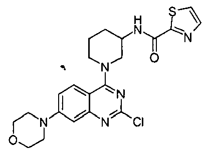 |
| 65 | 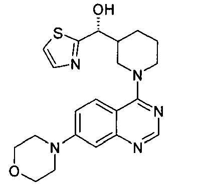 |
| 66 | 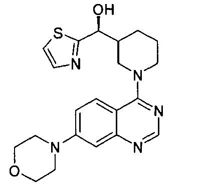 |
| 67 | 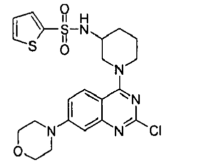 |
| 70 | 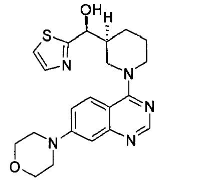 |
et/ou leurs sels, tautomères et/ou stéréo-isomères physiologiquement acceptables, y compris leurs mélanges selon tous rapports.

5. Procédé pour la préparation de composés de la formule (IA) selon la revendication 1 et/ou de leurs sels, tautomères et/ou stéréo-isomères physiologiquement acceptables comportant les étapes qui suivent :
(a) réaction d'un composé de la formule (II) : 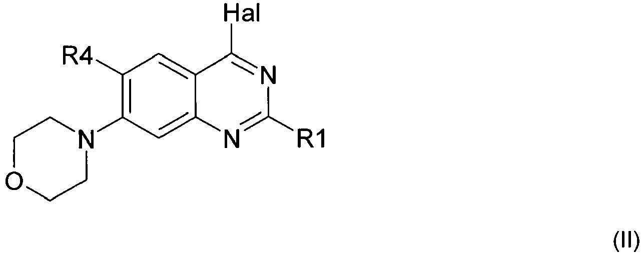 dans laquelle R1, R4, Y, A, Alk et Hal présentent la signification indiquée selon la revendication 1,
avec un composé de la formule (III) : 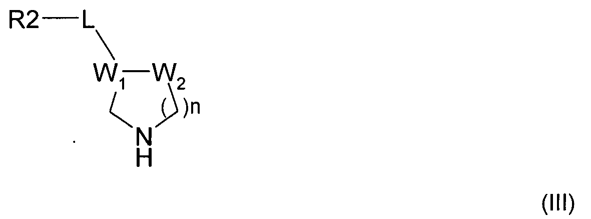 dans laquelle R2, R3, Y, W₁, W₂, L, A, Alk, Cyc, Ar, Het¹, Het², Hal et n présentent la signification indiquée selon la revendication 1,
pour obtenir les composés de la formule (IA) : 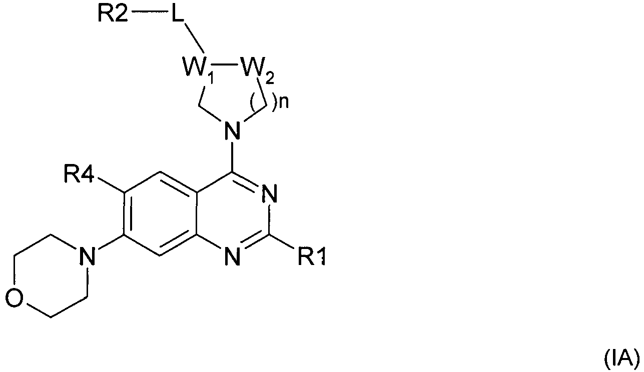 dans laquelle R1, R2, R3, R4, Y, W₁, W₂, L, A, Alk, Cyc, Ar, Het¹, Het², Hal et n présentent la signification indiquée selon la revendication 1,
et en option
(b) conversion d'une base ou d'un acide des composés de la formule (IA) selon l'un de leurs sels.

6. Composés intermédiaires de la formule (II) : 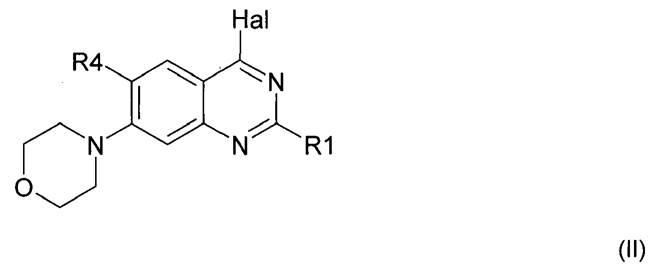 dans laquelle :
R1 représente H, CN, A, OY, NYY ou -NH-C(NYY)=NY,
R4 représente H ou Hal,
Y représente H, A ou Alk-OA,
A représente alkyle non ramifié ou ramifié comportant 1-10 atome(s) de C, où 1-7 atome(s) de H peut/peuvent être remplacé(s), indépendamment l'un de l'autre ou les uns des autres, par Hal,
Alk représente alkylène comportant 1-6 atome(s) de C, où 1-4 atome(s) de H peut/ peuvent être remplacé(s), indépendamment l'un de l'autre ou les uns des autres, par Hal et/ou CN, et
Hal représente F, Cl, Br ou I,
et/ou leurs sels, tautomères et/ou stéréo-isomères physiologiquement acceptables, y compris leurs mélanges selon tous rapports.

7. Procédé pour la préparation de composés intermédiaires de la formule (II) selon la revendication 6 et/ou de leurs sels, tautomères et/ou stéréo-isomères physiologiquement acceptables comportant les étapes qui suivent :
(a) réaction d'un composé de la formule (IIC) : 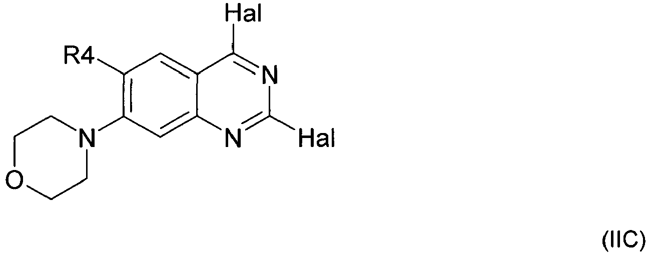 dans laquelle R4 et Hal présentent la signification indiquée selon la revendication 6,
avec un composé X-R1, dans lequel :
X représente un élément pris parmi le 1er groupe principal, et
R1, Y, A, Alk et Hal présentent la signification indiquée selon la revendication 6,
où H₂ pour X-R1 est exclu,
pour obtenir les composés intermédiaires de la formule (II) : 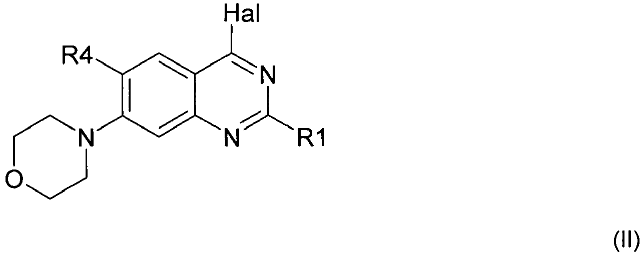 dans laquelle R1, R4, Y, A, Alk et Hal présentent la signification indiquée selon la revendication 6,
et en option
(b) conversion d'une base ou d'un acide des composés de la formule (II) selon l'un de leurs sels.

8. Procédé pour la préparation de composés intermédiaires de la formule (II) selon la revendication 6 et/ou de leurs sels, tautomères et/ou stéréo-isomères physiologiquement acceptables comportant les étapes qui suivent :
(a) réaction d'un composé de la formule (IIE) : 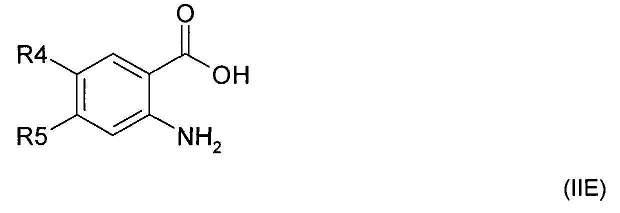 dans laquelle :
R4 représente H ou Hal,
R5 représente morpholinyle ou Hal, et
Hal représente F, CI, Br ou I,
avec de l'acétate de formamidine dans un alcool pour obtenir les composés intermédiaires de la formule (IID-1) : 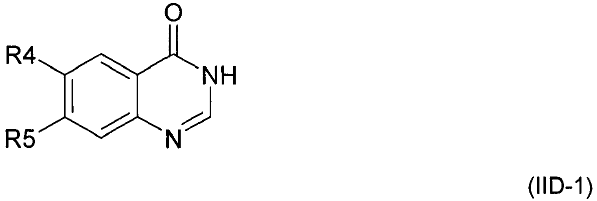 dans laquelle :
R4 représente H ou Hal,
R5 représente morpholinyle ou Hal, et
Hal représente F, Cl, Br ou I,
et, si R5 est Hal,
(b) réaction des composés intermédiaires de la formule (IID-1) avec de la morpholine pour obtenir les composés intermédiaires de la formule (IID) : 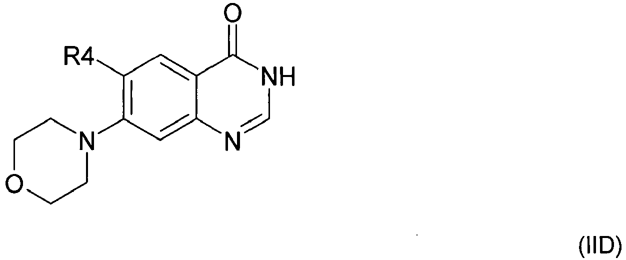 dans laquelle :
R4 représente H ou Hal, et
Hal représente F, Cl, Br ou I,
(b') réaction des composés intermédiaires de la formule (IID) avec un agent d'halogénation dans un amine organique pour obtenir des composés intermédiaires de la sous-formule (II) : 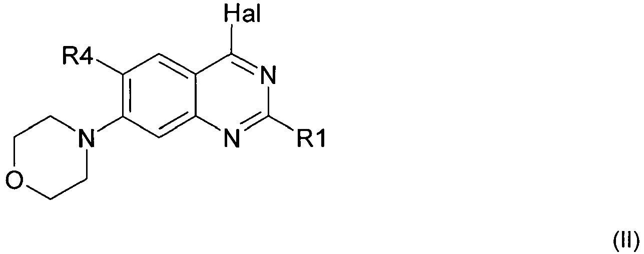 dans laquelle :
R1 représente H,
R4 représente H ou Hal, et
Hal représente F, CI, Br ou I,
et en option
(b") conversion d'une base ou d'un acide des composés intermédiaires de la formule (II) selon l'un de leurs sels.

9. Utilisation de composés selon l'une des revendications 1 à 4 et/ou de leurs sels, tautomères et/ou stéréo-isomères physiologiquement acceptables, y compris leurs mélanges selon tous rapports, pour l'inhibition des protéines kinases sérine/thréonine in vitro.

10. Utilisation d'au moins un composé selon l'une des revendications 1 à 4 et/ou de leurs sels, tautomères et/ou stéréo-isomères physiologiquement acceptables, y compris de leurs mélanges selon tous rapports, pour la sensibilisation de cellules cancéreuses vis-à-vis d'agents anti-cancer et/ou d'un rayonnement ionisant, étant entendu que la sensibilisation n'est pas effectuée in vivo sur le corps d'un être humain et/ou d'un animal.

11. Médicament comprenant au moins un composé selon l'une des revendications 1 à 4 et/ou leurs sels, tautomères et/ou stéréo-isomères physiologiquement acceptables, y compris leurs mélanges selon tous rapports.

12. Composition pharmaceutique comprenant, en tant que composé actif, une quantité efficace d'au moins un composé selon l'une des revendications 1 à 4 et/ou leurs sels, tautomères et/ou stéréo-isomères physiologiquement acceptables, y compris leurs mélanges selon tous rapports, en association avec des contributeurs pharmaceutiquement tolérés, en combinaison avec au moins un agent anti-cancer.

13. Composés selon l'une des revendications 1 à 4 et/ou leurs sels, tautomères et/ou stéréo-isomères physiologiquement acceptables, y compris leurs mélanges selon tous rapports, pour une utilisation au niveau de la prophylaxie, la thérapie et/ou le contrôle de la progression du cancer, de tumeurs, de métastases et/ou de troubles de l'angiogenèse, en combinaison avec une radiothérapie et/ou avec au moins un agent anticancer.
